# EUROPEAN PATENT SPECIFICATION

(11) **EP 3 054 940 B1**
(45) Date of publication and mention of the grant of the patent: **23.09.2020**
(21) Application number: 14852793.0
(22) Date of filing: 06.10.2014
(51) Int. Cl.: A61K 31/232, A61K 9/107, A61K 9/48, A61K 31/202, A61K 31/565, A61P 1/16

(54) **COMPOSITIONS AND METHODS FOR TREATING NON-ALCOHOLIC STEATOHEPATITIS**
ZUSAMMENSETZUNGEN UND VERFAHREN ZUR BEHANDLUNG NICHTALKOHOLISCHER STEATOHEPATITIS
COMPOSITIONS ET MÉTHODES DE TRAITEMENT DE LA STÉATOHÉPATITE NON ALCOOLIQUE

(30) Priority: 07.10.2013 US 201361887824 P
(43) Date of publication of application: 17.08.2016
(73) Proprietor: Mochida Pharmaceutical Co., Ltd., Tokyo 160-8515 (JP)
(72) Inventor: SUZUKI, Ayako, Little Rock, Arkansas 72211 (US); HARADA, Tsuyoshi, Tokyo 160-8515 (JP); MIZUGUCHI, Kiyoshi, Tokyo 160-8515 (JP)
(74) Representative: Grünecker Patent- und Rechtsanwälte PartG mbB
(86) International application number: PCT/JP2014/077120
(87) International publication number: WO 2015/053379

(56) References cited:
- EP-A1- 2 719 382
- WO-A1-2009/028457
- WO-A1-2013/127728
- JP-A- 2009 534 317
- JP-A- 2011 519 846
- US-A1- 2012 264 824
- TANAKA NAOKI ET AL: "Highly purified eicosapentaenoic acid treatment improves nonalcoholic steatohepatitis", JOURNAL OF CLINICAL GASTROENTEROLOGY, RAVEN PRESS LTD., NEW YORK, NY, US, vol. 42, no. 4, 1 April 2008 (2008-04-01), pages 413-418, XP009167353, ISSN: 0192-0790, DOI: 10.1097/MCG.0B013E31815591AA
- GESIRA SOARES DE ASSIS FLORENTINO ET AL: "NONALCOHOLIC FATTY LIVER DISEASE IN MENOPAUSAL WOMEN", ARQUIVOS DE GASTROENTEROLOGIA, vol. 50, no. 3, 1 September 2013 (2013-09-01), pages 180-185, XP55338129, BR ISSN: 0004-2803, DOI: 10.1590/S0004-28032013000200032
- PETRA M. WITT ET AL: "The incorporation of marine n-3 PUFA into platelets and adipose tissue in pre- and postmenopausal women: a randomised, double-blind, placebo-controlled trial", BRITISH JOURNAL OF NUTRITION, vol. 104, no. 03, 25 March 2010 (2010-03-25), pages 318-325, XP055338104, UK ISSN: 0007-1145, DOI: 10.1017/S0007114510000371
- PROCEEDINGS OF THE 61ST MEETING OF JAPAN SOCIETY OF HOME ECONOMICS vol. 61 ST, 2009, page 32, XP008183176
- PROSTAGLANDINS LEUKOT ESSENT FATTY ACIDS vol. 61, no. 6, 1999, pages 381 - 390, XP055343166
- KANZO vol. 46, no. SUPPLE, 2005, page A329
- KANZO vol. 43, no. SUPPLE, 2002, page A397, XP008183155
- KAN TAN SUI vol. 60, no. 5, 2010, pages 759 - 764, XP008183243
- OBSTETRICAL AND GYNECOLOGICAL PRACTICE vol. 56, no. 8, 2007, pages 1161 - 1165, XP008183184
- PROCEEDINGS OF THE 65TH ANNUAL MEETING OF THE JAPAN SOCIETY OF NUTRITION AND FOOD SCIENCE vol. 65 TH, 2011, page 110, XP008183228
- KANZO vol. 53, no. SUPPLE, 2012, page A706, XP008183232

## Description

### BACKGROUND OF THE INVENTION

Heavy alcohol use is known to lead to liver complications, including alcoholic hepatitis which is often characterized by fatty liver and inflammation. Alcoholic hepatitis can ultimately lead to cirrhosis of the liver (scarring) and hardening of the liver tissue. However, individuals that do not consume excessive amounts of alcohol can also be found to have liver disease complications. Non-alcoholic fatty liver disease (NAFLD) is understood to encompass a broad spectrum of liver diseases, including steatosis (simple fatty liver), non-alcoholic steatohepatitis (NASH) and advanced scarring of the liver (cirrhosis). NASH has traditionally been diagnosed by means of a liver biopsy to characterize the liver histology, particularly with respect to the degree and characteristics of inflammation, fibrosis and steatosis (fat accumulation). NASH then generally refers to clinical findings based upon the liver biopsy of a patient with steatohepatitis, combined with the absence of significant alcohol consumption (Neuschwander-Tetri, B. A. and S. H. Caldwell (2003) Hepatology 37(5): 1202-1209).

In NASH, fat accumulation is seen in varying degrees of inflammation (hepatitis) and may lead to more serious conditions involving scarring (fibrosis). Patients having NASH are also often characterized by abnormal levels of liver enzymes, such as aspartate aminotransferase (AST) and alanine aminotransferase (ALT). Currently, there are few therapies to slow down or alter the course of further disease progression in NASH. Therefore, there remains a need for effective NASH treatments.

### SUMMARY OF THE INVENTION

The present invention addresses the above-mentioned needs and provides related advantages as well.

In one aspect, the present invention provides a method for treating a fatty liver disease or disorder in a subject in need thereof. The method can comprise selecting a subject having or suspected of having a fatty liver disease or disorder, wherein the subject can be a woman and further wherein the age of the subject can be less than 50; and administering a therapeutically effective amount of a pharmaceutical composition comprising at least one compound selected from the group consisting of ethyl eicosapentanoate (EPA-E), eicosapentaenoic acid (EPA) and its pharmaceutically acceptable amides, salts, esters and phospholipids. In some cases EPA-E or EPA present may be at least 40% by weight in total of the fatty acids and their derivatives.

In another aspect, the present invention also provides a method for treating a fatty liver disease or disorder in a subject in need thereof, comprising: selecting a subject having or suspected of having a fatty liver disease or disorder, wherein the subject can be a pre-menopausal woman; and administering a therapeutically effective amount of a pharmaceutical composition comprising at least one compound selected from the group consisting of ethyl eicosapentanoate (EPA-E), eicosapentaenoic acid (EPA) and its pharmaceutically acceptable amides, salts, esters and phospholipids. In some cases EPA-E or EPA present may be at least 40% by weight in total of the fatty acids and their derivatives.

In yet another aspect of the present invention, a method is provided for treating a fatty liver disease or disorder in a subject in need thereof, comprising: selecting a subject having or suspected of having a fatty liver disease or disorder, wherein the subject can receive a hormone replacement therapy; and administering a therapeutically effective amount of a pharmaceutical composition comprising at least one compound selected from the group consisting of ethyl eicosapentanoate (EPA-E), eicosapentaenoic acid (EPA) and its pharmaceutically acceptable amides, salts, esters and phospholipids. In some cases EPA-E or EPA present may be at least 40% by weight in total of the fatty acids and their derivatives. In some embodiments, the hormone in the hormone replacement therapy can be estrogen, progesterone, progestin or any combination thereof.

In another aspect, the present invention provides a method for treating a fatty liver disease or disorder in a subject in need thereof, comprising: administering a therapeutically effective amount of a pharmaceutical composition comprising at least one compound selected from the group consisting of ethyl eicosapentanoate (EPA-E), eicosapentaenoic acid (EPA) and its pharmaceutically acceptable amides, salts, esters and phospholipids in combination with an estrogen, progesterone, progestin or any combination thereof.

In yet another aspect, the current invention provides a method for treating a fatty liver disease or disorder in a subject in need thereof, comprising: selecting a subject having or suspected of having a fatty liver disease or disorder, wherein the serum level of total estrogen in the subject can be higher than 10 pg/ml; and administering a therapeutically effective amount of a pharmaceutical composition comprising at least one compound selected from the group consisting of ethyl eicosapentanoate (EPA-E), eicosapentaenoic acid (EPA) and its pharmaceutically acceptable amides, salts, esters and phospholipids. In some cases EPA-E or EPA present may be at least 40% by weight in total of the fatty acids and their derivatives.

In still yet another aspect, the current invention provides a pharmaceutical composition comprising: at least one compound selected from the group consisting of ethyl eicosapentanoate (EPA-E), eicosapentaenoic acid (EPA) and its pharmaceutically acceptable amides, salts, esters and phospholipids; an effective amount of an estrogen, wherein said effective amount can be an amount capable of achieving a physiological concentration of total estrogen at 10 pg/ml or above in serum or plasma; and a pharmaceutically acceptable carrier.

In some embodiments, the estrogen can be Estrone, Estradiol, Estriol or their pharmaceutically acceptable analog. In some embodiments, the estrogen can comprise Estrone, Estradiol, Estriol, their pharmaceutically acceptable analog or any combination thereof.

In some embodiments, the subject is a female. In some embodiments, the age of the subject is less than 40, 45, 50, 55, 60, 65 or 70.

In some embodiments, the subject has a physiological concentration of estrogen at 15 pg/ml or above in serum or plasma. The subject can have a physiological concentration of estrogen at 30 pg/ml or above in serum or plasma. The subject can have a physiological concentration of estrogen at 50 pg/ml or above in serum or plasma. The subject can have a physiological concentration of estrogen at 100 pg/ml or above in serum or plasma. The subject can have a physiological concentration of estrogen at 0, 1, 2, 3, 4, 5, 6, 7, 8, 9, 10, 11, 12, 13, 14,15, 20, 25, 30, 35, 40, 50, 60, 70, 80, 90, 100, 200, 300, 400 pg/ml or above in serum or plasma.

In some embodiments, the subject can have a physiological concentration of endogenous estrogen that is less than 35 pg/mL in serum or plasma. The subject can have a physiological concentration of endogenous estrogen that is less than 15 pg/mL in serum or plasma. The subject can have a physiological concentration of endogenous estrogen that is less than 10 pg/mL in serum or plasma. In some embodiments, the subject has a physiological concentration of endogenous estrogen that is less than 35, 30, 25, 20, 15, 14, 13, 12, 11, 10, 9, 8, 7, 6, 5, 4, 3, 2 or 1 pg/mL in serum or plasma.

In some embodiments, the fatty liver disease or disorder is selected from the group consisting of Non-Alcoholic Fatty Liver Disease (NAFLD) and Non-Alcoholic Steatohepatitis (NASH).

In some embodiments, the fatty liver disease is characterized by the baseline pretreatment level in the subject of at least one criteria selected from the group consisting of ALT in a range of 10 to 300 IU/L, AST in a range of 10 to 250 IU/L, HDL/C in a range of 25 to 55 mg/dl, LDL-C in a range of 100 to 200 mg/dl, triglycerides in a range of 100 to 1000 mg/dl, TC in a range of 170 to 300 mg/dl, High TG and low HDL-C, TG/HDL-C ratio in a range of 3.75 to 10, non-HDL-C in a range of 100 to 250 mg/dl, Free fatty acid in a range of 400 to 1000 *µ* Eq/L, HOMA-IR in a range of 1.5 to 5, HbA1c in a range of 5.7 to 10%, Fasting plasma glucose in a range of 100 to 200 mg/dl, impaired glucose tolerance and metabolic syndrome. The fatty liver disease can be characterized by the baseline pretreatment level in the subject of at least one criteria selected from the group consisting of low level of EPA, docosapentaenoic acid (DPA), docosahexaenoic acid (DHA), EPA/arachidonic acid (AA), DHA/AA. DHA/DPA, AA/Homo-γ-linoleic acid: and high level of AA, monounsaturated fatty acid (MUFA), Palmitoleic acid, Oleic acid, Oleic acid/Stearic acid, Palmitoleic acid/Palmitic acid, *γ*-linoleic acid/Linoleic acid, Adrenic acid/AA compared to each average level in subjects with fatty liver disease.

In some embodiments, the therapeutically effective amount of EPA-E and/or EPA and/or its pharmaceutically acceptable amides, salts, esters or phospholipids administered to the subject can be an amount between about 1800 and about 2700 mg per day. The therapeutically effective amount of EPA-E and/or EPA and/or its pharmaceutically acceptable amides, salts, esters or phospholipids administered to the subject can be an amount between about 1000 and about 4000 mg per day. The therapeutically effective amount of EPA-E and/or EPA and/or its pharmaceutically acceptable amides, salts, esters or phospholipids administered to the subject can be at least 1800 mg per day. The therapeutically effective amount of EPA-E and/or EPA and/or its pharmaceutically acceptable amides, salts, esters or phospholipids administered to the subject can be 2700 mg per day.

In some embodiments, the subject is administered EPA-E and/or EPA and/or its pharmaceutically acceptable amides, salts, esters or phospholipids for at least 1 month. The subject can be administered EPA-E and/or EPA and/or its pharmaceutically acceptable amides, salts, esters or phospholipids for at least 3 months. The subject can be administered EPA-E and/or EPA and/or its pharmaceutically acceptable amides, salts, esters or phospholipids for at least 1 year. In some embodiments, the EPA-E and/or EPA and/or its pharmaceutically acceptable amides, salts, esters or phospholipids is administered orally.

In some embodiments, selecting a subject having or suspected of having a fatty liver disease or disorder comprises selecting a subject having a score selected from the group consisting of: a NAS score greater than or equal to 3, a steatosis score equal to or greater than 1, a lobular inflammation score equal to or greater than 1, a ballooning score equal to or greater than 1 and a fibrosis score equal to or greater than 1.

In some embodiments, the subject is further characterized by having at least one condition selected from the group consisting of high triglycerides and low HDL-C, impaired glucose tolerance and metabolic syndrome. In some embodiments, selecting a subject having or suspected of having a fatty liver disease or disorder comprises selecting a subject who is overweight. In some embodiments, selecting a subject having or suspected of having a fatty liver disease or disorder comprises selecting a subject with a body mass index (BMI) greater than or equal to 25 kg/m². In some embodiments, selecting a subject having or suspected of having a fatty liver disease or disorder comprises selecting a subject with a familial history of fatty liver disease. In some embodiments, the selecting a subject having or suspected of having a fatty liver disease or disorder comprises selecting a subject with a NAS score greater than or equal to 4.

In some embodiments, the subject does not have a conditions selected from the following group consisting of alcoholic liver injury, drug-induced liver injury, chronic active hepatitis, cirrhosis, liver cancer, hepatic steatosis and hepatocyte apoptosis. In some embodiments, the subject does not suffer from injury in the liver. In some embodiments, the subject does not exhibit liver dysfunction.

In some embodiments, after administration of ethyl eicosapentanoate, said subject exhibits the following changes in said at least one marker as compared to the baseline level of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95% reduction for ALT, AST, TG, TG/HDL ratio, Fee fatty acid, AA, MUFA, Palmitoleic acid, Oleic acid, Oleic acid/Stearic acid ratio, Palmitoleic acid/Palmitic acid ratio, Adrenic acid/AA ratio, Ferritin, Thioredoxin, TNF *α*, sTNF-R1, sTNF-R2, Hs-CRP, CRGF, sCD40, Leptin, complement factor D, CK18 fragment, serum HMGB1, Fas, Hyaluronic acid, Type IV collagen (7s domain), procollagen III peptide or PAI-1; at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 200, 250, 300, 350, 400, 450, 500, 600, 700, 800, 900, 1000, 1500, 2000, 3000, 4000, 5000% increase for EPA or EPA/AA ratio; at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 200, 250, 300, 350, 400, 450, 500% increase for DPA, AA/Homo- *γ-*linoleic acid ratio or Serum adiponectin; no worsening of ALP, bilirubin, GGT, Albumin, HDL-C, LDL-C, TC, non-HDL-C, HOMA-IR, HbA1c, Glucose, Fasting plasma glucose, postprandial plasma glucose, OGTT, platelet count, M30, sFas, NASH risk score or BMI.

In some embodiments, the methods as described herein can further comprise improving the NAS score in said subject. The NAS score can be improved by at least 30%. In some embodiments, the methods as described herein can further comprise improving the steatosis score in said subject. The steatosis score can be improved by at least 30%.

In some embodiments, the pharmaceutical composition is administered to the subject 1 to 4 times per day. The pharmaceutical composition can be present in one or more capsules. The pharmaceutical composition as can be formulated for oral administration.

In some embodiments, the pharmaceutical composition can comprise a self-emulsifying composition. In some embodiments, the present disclosure provides for the composition comprising a self-emulsifying composition comprising 50 to 95% by weight of an omega-3 polyunsaturated fatty acids or a pharmaceutically acceptable salt or ester thereof. In some embodiments, the present disclosure provides for the composition comprising a self-emulsifying composition comprising 50 to 95% by weight, of EPA-E or a pharmaceutically acceptable salt or ester thereof.

In some embodiments, the present disclosure provides for the composition comprising a self-emulsifying composition comprising at least 60%, by weight, of EPA-E or a pharmaceutically acceptable salt or ester thereof.

In some embodiments, the present disclosure provides for the composition comprising a self-emulsifying composition comprising at least 70%, by weight, of EPA-E or a pharmaceutically acceptable salt or ester thereof.

In some embodiments, the present disclosure provides for the composition comprising a self-emulsifying composition comprising at least 80%, by weight, of EPA-E or a pharmaceutically acceptable salt or ester thereof.

In some embodiments, the present disclosure provides for the composition comprising a self-emulsifying composition comprising at least 90%, by weight, of EPA-E or a pharmaceutically acceptable salt or ester thereof.

In some embodiments, the present disclosure provides for the composition comprising a self-emulsifying composition comprising at least 95%, by weight, of EPA-E or a pharmaceutically acceptable salt or ester thereof.

In some embodiments, the present disclosure provides for the composition comprising a self-emulsifying composition comprising at least 96%, by weight, of EPA-E or a pharmaceutically acceptable salt or ester thereof.

In some embodiments, the present disclosure provides for the composition comprising, 5 to 50% by weight, an emulsifier having a hydrophilic lipophilic balance of at least 10.

In some embodiments, the present disclosure provides for the composition comprising, 10 to 50% by weight, an emulsifier having a hydrophilic lipophilic balance of at least 10.

In some embodiments, the present disclosure provides for the composition comprising, 20 to 50% by weight, an emulsifier having a hydrophilic lipophilic balance of at least 10.

In some embodiments, the present disclosure provides for the composition comprising, 30 to 50% by weight, an emulsifier having a hydrophilic lipophilic balance of at least 10.

In some embodiments, the present disclosure provides for the composition comprising, 40 to 50% by weight, an emulsifier having a hydrophilic lipophilic balance of at least 10.

In some embodiments, the present disclosure provides for the composition comprising, ethanol content up to 4% by weight in relation to the total content of the compound and the emulsifier.

In some embodiments, the present disclosure provides for the composition not containing ethanol.

In some embodiments, the present disclosure provides for the emulsifier at least one member selected from the group consisting of polyoxyethylene hydrogenated castor oil, polyoxyethylene sorbitan fatty acid ester, polyoxyethylene castor oil, polyethylene glycol fatty acid ester, polyoxyethylene polyoxypropylene glycol, sucrose fatty acid ester, sorbitan fatty acid ester, glycerin fatty acid ester and lecithin.

In some embodiments, the present disclosure provides for the emulsifier as at least one member selected from the group consisting of polyoxyethylene hydrogenated castor oil, polyoxyethylene sorbitan fatty acid ester, polyoxyethylene castor oil, sucrose fatty acid ester, sorbitan fatty acid ester and glycerin fatty acid ester.

In some embodiments, the present disclosure provides for the polyoxyethylene hydrogenated castor oil as at least one member selected from the group consisting of polyoxyethylene (20) hydrogenated castor oil, polyoxyethylene (40) hydrogenated castor oil, polyoxyethylene (50) hydrogenated castor oil, polyoxyethylene (60) hydrogenated castor oil, and polyoxyethylene (100) hydrogenated castor oil.

In some embodiments, the present disclosure provides for the polyoxyethylene sorbitan fatty acid ester as at least one member selected from the group consisting of polyoxyethylene sorbitan monooleate, polyoxyethylene sorbitan tristearate, polyoxyethylene sorbitan monostearate, polyoxyethylene sorbitan monopalmitate, and polyoxyethylene sorbitan monolaurate.

In some embodiments, the present disclosure provides for the polyoxyethylene castor oil as at least one member selected from the group consisting of compound prepared by adding polymerization of ethylene oxide to castor oil with an average ethylene oxide mole number of 3, 10, 20, 30, 40, 50 or more.

In some embodiments, the present disclosure provides for the sucrose fatty acid ester as at least one member selected from the group consisting of sucrose laurate, sucrose myristate, sucrose palmitate, sucrose stearate, and sucrose oleate.

In some embodiments, the present disclosure provides for the polyoxyethylene polyoxypropylene glycol as at least one member selected from the group consisting of polyoxyethylene (3) polyoxypropylene (17) glycol, polyoxyethylene (20) polyoxypropylene (20) glycol, polyoxyethylene (42) polyoxypropylene (67) glycol, polyoxyethylene (54) polyoxypropylene (39) glycol, polyoxyethylene (105) polyoxypropylene (5) glycol, polyoxyethylene (120) polyoxypropylene (40) glycol, polyoxyethylene (160) polyoxypropylene (30) glycol, polyoxyethylene (196) polyoxypropylene (67) glycol, and polyoxyethylene (200) polyoxypropylene (70) glycol.

In some embodiments, the present disclosure provides for the sorbitan fatty acid ester as at least one member selected from the group consisting of sorbitan monolaurate, sorbitan monostearate, sorbitan monooleate, sorbitan monopalmitate, sorbitan trioleate, and sorbitan sesquioleate.

In some embodiments, the present disclosure provides for the glycerin fatty acid ester as at least one member selected from the group consisting of glyceryl monooleate, glyceryl monostearate, decaglyceryl monooleate, decaglyceryl monolaurate decaglyceryl trioleate and tetraglyceryl monooleate.

In some embodiments, the present disclosure provides for the composition as containing a lecithin where lecithin is at least one member selected from the group consisting of soybean lecithin, enzymatically decomposed soybean lecithin, hydrogenated soybean lecithin, and egg yolk lecithin.

In some embodiments, the present disclosure provides the composition as containing a polyhydric alcohol, where it may further comprise propylene glycol or glycerin.

In some embodiments, the present disclosure provides the composition contains at least one member selected from the group consisting of eicosapentaenoic acid, docosahexaenoic acid, and their pharmaceutically acceptable amides, salts, esters and phospholipids.

In some embodiments, the present disclosure provides the composition contains less than 5% eicosapentaenoic acid, and their pharmaceutically acceptable amides, salts, esters and phospholipids.

In some embodiments, the present disclosure provides the composition contains less than 5% docosahexaenoic acid, and their pharmaceutically acceptable amides, salts, esters and phospholipids.

In some embodiments, the present disclosure provides the composition contains ethyl icosapentate and/or ethyl docosahexaenoate.

In some embodiments, the present disclosure provides the composition a total content of the emulsifier having an HLB of at least 10 is 10 to 100 parts by weight in relation to 100 parts by weight of the at least one compound selected from the group consisting of omega-3 polyunsaturated fatty acids and their pharmaceutically acceptable amides, salts, esters and phospholipids.

In some embodiments, the present disclosure provides the composition a total content of the emulsifier having an HLB of at least 10 is 10 to 50 parts by weight in relation to 100 parts by weight of the at least one compound selected from the group consisting of EPA-E and/or EPA and/or its pharmaceutically acceptable amides, salts, esters and phospholipids.

In some embodiments, the EPA-E and/or EPA present in the composition is at least 40% by weight in total of the fatty acids and derivatives.

In some embodiments, the pharmaceutical composition comprises Epadel® (Mochida Pharmaceutical Co., Ltd., Tokyo Japan), Lovaza ™(Glaxo SmithKline, FL USA), Omacor ™(Pronova Biopharma ASA, Oslo Norway), Lotriga™(Takeda Pharmaceutical Co., Ltd., Osaka Japan), Vascepa ™(Amarin Pharma Inc., NJ USA), Epanova™ (Astra Zeneca Pharmaceuticals LP, Wilmington, Germany) or Omtryg™ (Trygg Pharma Inc., VA USA).

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 shows data for changes in histologic features of steatosis score (STE) and NAS score by a NASH POC Test. The univariate associations predictor is the gender/age categories of: Men<50, Men≥50, Women<50, Women≥50. Each histologic feature is categorized by -1: improvement, 0: no change, 1: worsening.
Figure 2 shows data for multivariable associations for women with age younger than 50 by NASH POC Test. Cumulative odds ratio (COR) of changes in steatosis score (STE) and NAS score (NAS) change is presented.
Figure 3 shows data for multivariable associations for men with age younger than 50 by NASH POC Test. Cumulative odds ratio (COR) of changes in steatosis score (STE) and NAS score (NAS) change is presented.
Figure 4 depicts the chemical formulas of three naturally occurring estrogen types.
Figure 5 illustrated the reasons for patient exclusion from the trial.
Figure 6 shows the baseline demographic, clinical and laboratory data of those randomized patients.
Figure 7 presents histological outcomes of the patients (baseline versus end of study).
Figure 8 summarized the effects on women younger than 50 years old with EPA-E. There were no significant changes in AST, ALT or GGT from baseline to end of study in either the placebo or treatment arms.

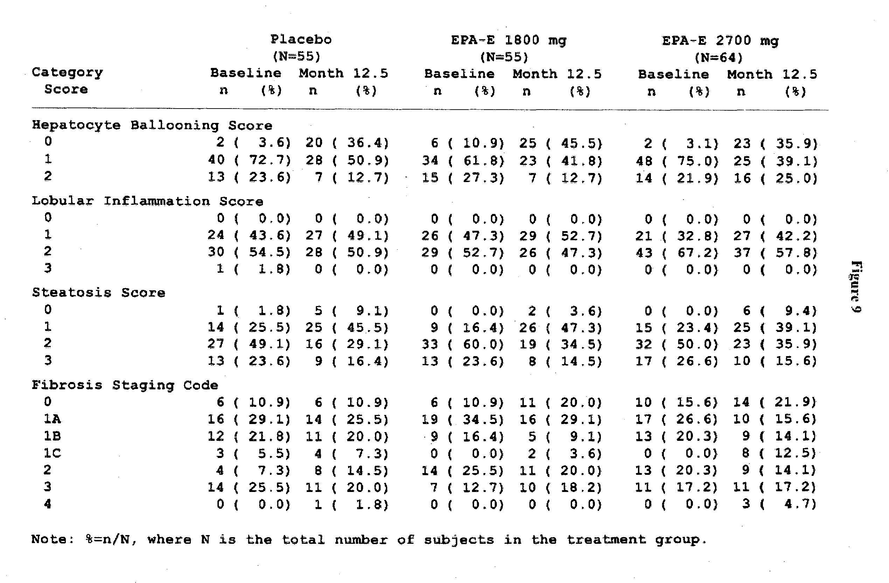

Figure 9 shows histological outcomes in subjects where baseline and end of treatment biopsies were available.

### DETAILED DESCRIPTION OF THE INVENTION

### I. Terminology

The terminology of the present disclosure is for the purpose of describing particular embodiments only and is not intended to be limiting of compositions and/or methods of this disclosure.

The terms "methods of treating" mean amelioration, prevention or relief from the symptoms and/or effects associated with a disorder or condition. The disorder or condition can be a NAFLD-associated disorder, including steatosis (simple fatty liver), NASH and advanced scarring of the liver (cirrhosis). As used herein, reference to "treatment" of a patient is intended to include prophylaxis.

As used herein, a "therapeutically effective amount" of a drug or pharmaceutical composition or formulation, or agent, described herein is an amount of a drug or agent that, when administered to a subject with a disease or condition, will have the intended therapeutic effect, e.g., alleviation, amelioration, palliation or elimination of one or more manifestations of the disease or condition in the subject. The full therapeutic effect does not necessarily occur by administration of one dose and may occur only after administration of a series of doses. Thus, a therapeutically effective amount may be administered in one or more administrations.

A "subject" or "patient" is a mammal, preferably a human, but can also be an animal in need of veterinary treatment, e.g., companion animals (e.g., dogs, cats, and the like), farm animals (e.g., cows, sheep, pigs, horses, and the like) and laboratory animals (e.g., rats, mice, guinea pigs, and the like).

The term "menopause" refers to the end of menstrual cycle and it signals the end of the fertile or potentially reproductive state of a woman. It can be defined as the permanent cessation of the primary functions of the ovaries comprising the production and release of hormones that cause the menses, the creation of the uterine lining and the subsequent shedding of the uterine lining. The hormones can include estrogen and progesteron. Menopause is normally not sudden or abrupt, but can happen in a period of time. Menopause can occur naturally as part of the biological aging process, or it can happen due to some functional disorder affecting the reproductive system (e.g., endometriosis, polycystic ovary syndrome, cancer of the reproductive organs, and the like). In some cases, menopause can also occur in those women who have a hysterectomy or women who have their ovaries removed. The date of menopause in human females can be medically defined as the time of the last menstrual period (or menstrual flow of any amount, however small). Factors that can affect the age that the menopause occurs including, without limitation, cigarette smoking, higher body mass index, racial and ethnic factors, illnesses, chemotherapy, radiation and the surgical removal of the ovaries, with or without the removal of the uterus. The term "pre-menopausal woman" refers to a woman whose ovaries still produce and release hormones that can cause menses. The "pre-menopausal woman" can refer to a woman that is fertile, potentially reproductive, and/or still has her menstrual cycles. The term "post-menopausal woman" refers to a woman that already has her menopause, stop of her menstrual cycles, and/or is not considered potentially reproductive. The "post-menopausal woman" can refer to a woman who has complete cessation of the primary functions of her ovaries.

The term "EPAs" refer to eicosapentaenoic acid (EPA) and/or any pharmaceutically acceptable amides, salts, esters and phospholipids of EPA or any other form which lead to metabolization of EPA, or the incorporation of EPA into body fluids, tissues or organs, including but not limited to inorganic salts such as sodium salts and potassium salts, organic salts such as benzylamine salts and diethylamine salts, salts with basic amino acids such as arginine salts and lysine salts, and exemplary esters include alkyl esters such as ethyl ester, and esters such as mono-, di- and TG, and exemplary phospholipids such as phosphatidylcholine, phosphatidylethanolamine, phosphatidylserine, phosphatidylglycerol and phosphatidylinositol. Preferable examples include ethyl ester and TG ester, and the more preferred is ethyl ester. More specifically, preferable examples include EPA-E, TG ester of EPA, and the like. The term "DHAs" refer to docosahexaenoic acid (DHA) and/or any pharmaceutically acceptable amides, salts, esters and phospholipids of DHA or any other form which lead to metabolization of DHA, or the incorporation of DHA into body fluids, tissues or organs, including but not limited to inorganic salts such as sodium salts and potassium salts, organic salts such as benzylamine salts and diethylamine salts, salts with basic amino acids such as arginine salts and lysine salts, and exemplary esters include alkyl esters such as ethyl ester, and esters such as mono-, di- and TG, and exemplary phospholipids such as phosphatidylcholine, phosphatidylethanolamine, phosphatidylserine, phosphatidylglycerol and phosphatidylinositol. Preferable examples include ethyl ester and TG ester, and the more preferred is ethyl ester. More specifically, preferable examples include ethyl docosahexaenoic acid (DHA-E), TG ester of DHA, and the like.

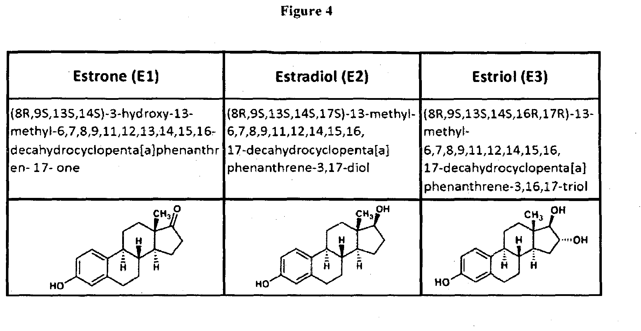

The term "estrogen" refers to any naturally occurring or synthetic primary female sex hormone or its analog, or compounds that possess estrogenic activities, (e.g., binding and activating estrogen receptors in cells). It is important in both menstrual and estrous reproductive cycles. Estrogens can be steroid hormones or non-steroidal. Estrogens can be synthesized or produced in vertebrates as well as some insects. Estrogen can be synthetic (xenoestrogens), produced by plants (phytoestrogens) or produced by fungi (mycoestrogens). Estrogens are used as part of some oral contraceptives, in estrogen replacement therapy for post-menopausal women, and in hormone replacement therapy for trans-women. Estrogen can be estrone, estradiol, estriol (Figure 4), their pharmaceutically acceptable analog or any combination thereof. The pharmaceutically acceptable analog of estrogen can be estradiol benzoate, estradiol valerate, estradiol cypionate, estradiol dipropionate, ethinylestradiol, mestranol, conjugated estrogen, estriol benzoate or estradiol dipropionate,

The "serum" level refers to a physiological level, and in some embodiments, "serum level" may refer to the level in body fluid including plasma level or bloodstream level and the like.

Generally, the term "concurrent administration", "co-administration", or "administration in conjunction with" in reference to two or more subjects of administration for administration to a subject body, such as components, agents, substances, materials, compositions, and/or the like, refers to administration performed using dose(s) and time interval(s) such that the subjects of administration are present together within the subject body, or at a site of action in the subject body, over a time interval in less than de minimus quantities. The time interval may be any suitable time interval, such as an appropriate interval of minutes, hours, days, or weeks, for example. The subjects of administration may be administered together, such as parts of a single composition, for example, or otherwise. The subjects of administration may be administered substantially simultaneously (such as within less than or equal to about 5 minutes, about 3 minutes, or about 1 minute, of one another, for example) or within a short time of one another (such as within less than or equal to about 1 hour, 30 minutes, or 10 minutes, or within more than about 5 minutes up to about 1 hour, of one another, for example). The subjects of administration so administered may be considered to have been administered at substantially the same time. One of ordinary skill in the art will be able to determine appropriate dose(s) and time interval(s) for administration of subjects of administration to a subject body so that same will be present at more than de minimus levels within the subject body and/or at effective concentrations within the subject body. When the subjects of administration are concurrently administered to a subject body, any such subject of administration may be in an effective amount that is less than an effective amount that might be used were it administered alone. Combinations according to the invention are generally envisioned and not specifically limited as long as the active ingredients are used in combination. Exemplary such embodiments of the drug administration include, for example, (1) administration of single preparation having both active ingredients incorporated therein; (2) administration of both active ingredients by preparing separate preparations each containing different active ingredients, and simultaneously administering these separate preparations from the same administration route with or without producing a kit of the combination of two preparations; (3) administration of both active ingredients by preparing separate preparations each containing different active ingredients, and administering these separate preparations at different timing with time lag from the same administration route with or without producing a kit of the combination of two preparations; (4) administration of both active ingredients by preparing separate preparations each containing different active ingredients, and simultaneously administering these separate preparations from different administration routes (of the same patient from different site) with or without producing a kit of the combination of two preparations; and (5) administration of both active ingredients by preparing separate preparations each containing different active ingredients, and administering these separate preparations at different timing with time lag from different administration routes (of the same patient from different site) with or without producing a kit of the combination of two preparations.

When the active ingredients are administered at different timing with time lag, the first and the second ingredients may be administered in this order, or in opposite order. When the active ingredients are administered simultaneously, these ingredients may be mixed immediately before the administration if the administration route is the same, while the active ingredients may be separately administered. The active ingredients may be used deliberately at different timing for various purposes. In an exemplary embodiment, one ingredient may be administered, and thereafter, the other ingredient may be administered while the effect of the first ingredient is about to occur or the effect of the first ingredient is still fully present.

In another embodiment, one drug, and in particular, the second ingredient may be administered once or twice a day by using an extended release formulation, and the other ingredient, and in particular, the first ingredient may be administered two or more times, for example, twice or three times a day, or alternatively, once or twice a day by using an extended release formulation. When both drugs are administered once or twice a day, and more preferably, when both drugs are administered once or twice a day simultaneously, or administered by incorporating in a composite formulation, the burden of the patients can be reduced to improve the drug compliance, and in turn, to improve the prophylactic/ameliorative or therapeutic effects and reduce the side effect. It is also possible that both drugs are administered and one drug is withdrawn while the effects of the ingredients are about to occur or the effects of the ingredients are still fully present.

The term "hepatocyte death" refers to any mode of cell death of liver cells. The term may be used to comprise cell death described as apoptosis, necrosis, necroptosis, autophagy or cornification, especially as apoptosis. Hepatocyte apoptosis may result from any form of liver injury or disease, including but not limited to cancer and fatty liver disorders which may further comprise non-alcoholic steatohepatitis (NASH), non-alcoholic associated fatty liver disease, secondary NAFLD, steatosis, progressive fibrosis, liver failure and cirrhosis. As used herein, secondary NAFLD may refer to NAFLD or similar symptoms that result from the use of one or more of the following medications: amiodarone, antiviral drugs such as nucleoside analogues, aspirin or NSAIDs, corticosteroids, methotrexate, nifedipine, perhexiline, tamozifen, tetracycline, and valproic acid. Hepatocytes apoptosis is a prominent pathologic feature of human NASH and the magnitude of apoptosis present correlates with the degree of liver damage and stage of fibrosis.

The term "non" or "early stage" as applied to hepatocyte apoptosis refers to the absence or beginning stages of cell death in the liver. Non and early stage hepatocyte apoptosis may be determined by any suitable means, including suitable immunoassays, biochemical assays, detection and measurement of abnormal or elevated levels of markers associated with hepatocyte apoptosis, such as sFas or M30, histological studies, microscopic studies or a NASH risk score calculated to be 3.0 or less. In some cases, non or early stages of hepatocyte apoptosis refers to a serum levels of sFas in a patient equal to or less than 10.0 ng/mL. In some cases non or early stages of hepatocyte apoptosis refers to a serum levels of M30 in a patient equal to or less than 1500 IU/L. In some cases non or early stages of hepatocyte apoptosis refers to a NASH risk score equal to or less than 3.0.

The term "sFas" refers to soluble Fas. Fas is a protein belonging to a death receptor of the tumor necrosis factor (TNF) family and involved in caspase activated pathways associated with cell death. Fas may also be known as Apo-1 or CD95. sFas is generated either by alternative mRNA splicing or proteolysis of membrane Fas. sFas may refer to any Fas or variant thereof capable of binding the Fas ligand. sFas refers to any related sFas that may be used as a biochemical marker, as measured in blood serum, to indicate the presence or absence of hepatocyte apoptosis in a subject.

The term "cytokeratin M30" or "M30" refers to cytokeratin-18 fragment M30 (M30). Cytokeratin-18 is a type I cytokeratin, or intermediate filament protein. M30 refers to a soluble fragment of the cytokeratin-18 protein that may be cleaved. M30 may be generated by any caspase enzymes during apoptosis or cell death, such as in hepatocytes. In some cases, M30 may be generated through cleavage of cytokeratin-18 by caspase-3. "M30", "M30 fragment" or CK-18 fragment may be used interchangeably as provided herein. M30 may also be known as ccK18, K19-Asp396, cytokeratin 18, ccCK18 or CK18-Asp396. M30 may further refer to any related soluble fragment of cytokeratin-18 that may be used as a biochemical marker, as measured in blood serum, to indicate the presence or absence of hepatocyte apoptosis in a subject.

In some cases, as determined by the method of Tamimi TI., et.al., J. Hepatol., 54, 1224-1229, 2011, incorporated by reference herein, NASH patients may be characterized by plasma sFas median (25^{th}, 75^{th} percentile) levels 11.8 (7.8, 12.5) ng/mL and M30 median (25^{th}, 75^{th} percentile) levels 598 (280, 846) IU/L. Non-NASH patients may be characterized by plasma sFas median (25^{th}, 75^{th} percentile) levels 5.9 (4.8, 8.3) ng/mL and M30 median (25^{th}, 75^{th} percentile) levels 176 (131, 224) IU/L.

The term "NASH risk score" or "risk score, refers to a composite score as calculated by the following equation: Risk Score=-6.4894+0.0078xM30(U/L)+0.4668xsFas(ng/mL) as cited by Tamimi TI., et.al., J. Hepatol., 54, 1224-1229, 2011. Serum or plasma levels sFas and M30 are measured in a subject and used to compute a risk score based on the regression equation as provided herein.

The term "liver injury" or "liver disease" or "liver dysfunction" may be used interchangeably and refer to any injury of the liver, including but not limited to hardening of the liver, scarring of the liver, decreased or abnormal biliary tract function, abnormal liver enzyme activity, hepatocyte apoptosis, cirrhosis of the liver, abnormal physiology as determined by common diagnostic methods include but not limited to ultrasound, or biopsy/histopathology, necrosis of the liver and the like.

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. The term "about" as used herein refers to a range that is 15% plus or minus from a stated numerical value within the context of the particular usage. For example, about 10 would include a range from 8.5 to 11.5. The term "about" also accounts for typical error or imprecision in measurement of values.

"Pharmaceutically acceptable carrier" or "pharmaceutically acceptable excipient" includes any and all solvents, dispersion media, coatings, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions of the invention is contemplated. Supplementary active ingredients can also be incorporated into the compositions.

### II. Subject

The methods and compositions described herein can be useful in both human therapeutics, pre-clinical, and veterinary applications. In some embodiments, the subject is a mammal. In some cases, the subject is a vertebrate animal. In some cases, the subject is human.

The methods and composition as described herein are useful for the treatment of subjects having or suspected in having a fatty liver disease or disorder. The methods and compositions comprise administration of a therapeutically effective amount of EPAs. The subject can be a pre-menopausal woman or has a serum level of estrogen that is similar to that of a pre-menopausal woman.

In some cases, the methods and compositions can be useful for the treatment of subject having or suspected in having a fatty liver disease or disorder. The methods and compositions can further comprise administration of an estrogen. The estrogen can be in an amount effective in elevating the serum level of estrogen to that of a pre-menopausal woman. The subjects can be post-menopausal woman, a pre-menarche woman, or a man, or a trans woman.

### A. Sex

The subject in need of a treatment comprising administration of EPAs can be a male subject or a female subject. In some cases, the subject can be a post-menopausal female. In some cases, the subject can be a pre-menopausal woman. Menopause of women can be linked to the risk of non-alcoholic fatty liver disease (NAFLD) or non-alcoholic steatohepatitis (NASH). Women after menopause may have higher risk of NASH or NAFLD as described in Nihon Naika Gakkai Zasshi 2009;98: 2101-2111, Fig.4. The deficiency state of estrogen associated with menopause can induce fatty liver and visceral fat increase, which are risk factors of NASH or NAFLD.

Methods and compositions as described herein can be useful in treating a subject that is a woman with serum level of estrogen that is similar to that of a pre-menopausal fertile woman. In some cases, methods and compositions as described herein can also be useful in treating a subject that is a male or woman with serum level of estrogen that is lower than that to a pre-menopausal fertile woman. The methods and compositions can further comprise co-administration of an estrogen. The co-administered estrogen can elevate the serum level of estrogen to that of a pre-menopausal fertile woman. In some cases, the subject can be a male, a trans woman, a post-menopausal woman or a subject with low serum level of estrogen.

### B. Age

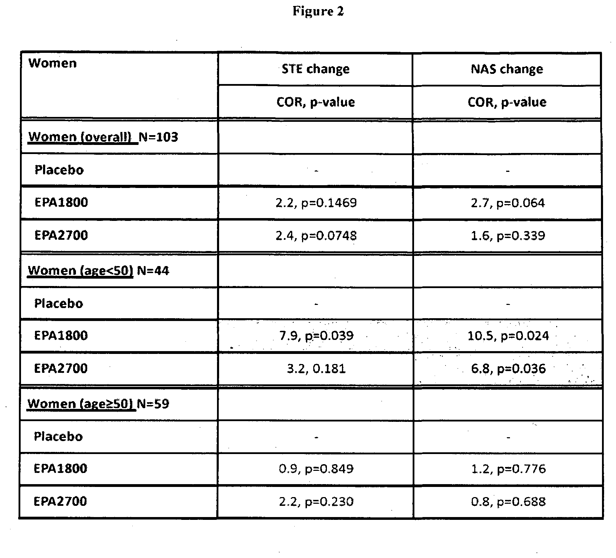

The subject can be at different ages. The methods and compositions as described herein can be particularly useful for treating subject having or suspected of having a fatty liver disease or disorder, wherein the subject can have a similar estrogen level or estrogenic activity as those of a pre-menopausal or fertile woman. In some cases, the subjects can be less than 50 years old. As shown in the Figures, unexpectedly beneficial effects were found by EPA-E administration to women that are younger than 50 years old. The age at menopause among women can be about 50 years old according to birth cohort, as described in Figure 2 and Table 3 of Am J Epidemiol 2006; 164: 1003-1011, hereby incorporated by reference in its entirety. In some cases, the subject can be less than 40 years old. In some cases, the subject can be less than 45 years old. In some cases, the subject can be less than 55 years old. In some cases, the subject can be less than 60 years old. In some cases, the subject can be less than 65 years old. In some cases, the subject can be less than 70 years old. For example, without limitation, the subject can be less than 20, 25, 30, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 years old.

The human subject can be a pubescent, or an adult (e.g., an early adult, a middle aged adult, a senior citizen). The human subject can be between about 10 years and 19 years old. The human subject can be between about 20 and about 39 year old; for example, about 20, 21, 22, 23, 24, 25, 26, 27, 28, 29, 30, 31, 32, 33, 34, 35, 36, 37, 38, or 39 years old. The human subject can be between about 40 to about 59 years old; for example, about 40, 41, 42, 43, 44, 45, 46, 47, 48, 49, 50, 51, 52, 53, 54, 55, 56, 57, 58, or 59 years old.

In some cases, methods and compositions as described herein can be useful in treatment for subjects that are not pre-menopausal or not fertile. The methods and compositions can further comprise co-administration of an estrogen. The subjects to be treated can be older than 50. In some cases, the subjects to be treated can be older than 40, 45, 50, 55, 60, 65, or 70. In some cases, the human subject can be about 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120 years old. The subject can be younger than 15, 10, or 5. In some cases, the subject can be a pre-menarche woman. The human subject can be a male subject or a female subject. The subject can be a transsexual woman.

### C. Hormone level

The methods and compositions as described herein can be useful to treat a subject that is a pre-menopausal woman, or has a physiological concentration of estrogen that is similar to that of a pre-menopausal woman. The physiological concentration or serum level of estrogen can change with menstrual cycles. For example without limitation, serum level of estrogen (i.e., estradiol) can be between 14-27 pg/ml from day 1-day 10; 14-54 pg/ml from day 11-day 20; and 19-40 pg/ml from day 21-day 30 of a menstrual cycle (see, e.g. Merck Manual Appendix II, incorporated herein by reference in its entirety). In some cases, serum level of estrogen of a pre-menopause woman can be about 10-55, 10-500, 15-450, 30-400, 15-350 or 19-160 pg/ml. The estrogen level can rapidly decrease post-menopause. The post-menopausal estrogen serum level can be 0 to about 35 pg/ml.

The serum level or estrogen can be measured by any methods known in the art. For example, without limitation, serum estradiol level can be measure by ELISA or direct immunoassays. Serum level of estradiol can also be measure by gas chromatography-tandem mass spectrometry (GC-MS/MS). Other methods to measure serum level of estrogen such as estradiol level can be found in Lee et al., J Clin Endocrinol Metab. 2006 Oct; 91(10): 3791-7. "Comparison of methods to measure low serum estradiol levels in postmenopausal women."

The methods and compositions comprising administration of EPAs as described herein can be useful in treating a subject that has varied serum level of estrogen. The serum level or physiological concentration of estrogen can be 15 pg/ml or above. The serum level or physiological concentration of estrogen can be 30 pg/ml or above. The serum level or physiological concentration of estrogen can be 35 pg/ml or above. The serum level or physiological concentration of estrogen can be 50 pg/ml or above. For example, the subject can have a physiological concentration of estrogen at about or more than about 10, 15, 20, 25, 30, 35, 40, 50, 60, 70, 80, 90, 100, 200, 300, 400 pg/ml in serum or plasma. The subject can have a physiological concentration of estrogen that is between about 10-55, 10-500, 15-50, 15-100, 15-150, 15-350, 15-450, 20-100, 20-150, 20-250, 30-400, 35-500, or 19-160 pg/mL.

In some cases, the methods and compositions comprising administration of EPAs can further comprise administration of an estrogen. The methods and compositions comprising administration of EPAs can be useful in treating a subject that is receiving hormone replace therapy. Hormone replacement therapy refers to any form of hormone therapy wherein the patient, in the course of medical treatment, receives hormones, either to supplement a lack of naturally occurring hormones, or to substitute other hormones for naturally occurring hormones. Examples of hormone replacement therapy can include: hormone replacement therapy for menopause and hormone replacement therapy for transgender people. Hormone replacement therapy for menopause may prevent discomfort caused by diminished circulating estrogen and progesterone hormones, or in the case of the surgically or prematurely menopausal, that it may prolong life and may reduce incidence of dementia. It can involve the use of one or more of a group of medications designed to artificially boost hormone levels. The types of hormones involved can be estrogens, progesterone or progestins, and sometimes testosterone. Hormone replacement therapy for transgender people introduces hormones associated with the gender that the patient identifies with (notably testosterone for trans men and estrogen for trans women). Some intersex people may also receive HRT. The subject to be treated by said methods and compositions can have a physiological concentration or serum level of endogenous estrogen that is lower than a level. The term "endogenous estrogen" refers to the estrogen that is naturally occurring in the body of the subject administered. The subject can have a physiological concentration of endogenous estrogen that is less than 100, 75, 60, 50, 45, 40, 35, 30, 25, 20, 15, 10 or 5 pg/mL in serum or plasma. In some cases, the subject can have a physiological concentration of endogenous estrogen that is less than 15 pg/mL. In some cases, the subject can have a physiological concentration of endogenous estrogen that is less than 35 pg/mL. In some cases, the subject can have a physiological concentration of endogenous estrogen that is less than 50 pg/mL.

### D. Diet

The subject can be on different types of diet. "Diet" refers to the types of food that a subject habitually consumes for a period of time. The period of time may be 3, 4, 5, 6, 7, 8, 9, 10, 11, 12, 13 or 14 days; 3, 4, 5, 6, 7 or 8 weeks; 3, 4, 5, 6, 7, 8, 9, 10, 1 1 or 12 months; 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100 years. In some embodiments, the subject on some types of diet may have or suspected to have a fatty liver disease or disorder. The methods and composition described herein comprising ornithine and/or aspartate may be administered to the subject. In some embodiments, the subject can be on a high-fat diet, high-cholesterol diet, high-saturated fat diet, high-sugar diet, high-protein diet, or low-fiber diet. In some cases, the subject can be on a diabetes diet. For each type of diet, "high" refers to a content level that is generally higher than the content in a normal diet that is well known in the art, as described by Food and Drug Administration ("Dietary Guidelines for Americans", 2010). The high-fat diet may have a fat content that is higher than 10%, 20%, 30%, 40%, 50%, 60%, 70%, 80%, 90%. The high-fat diet may contain 60% of fat. The subject may be on a diet that can cause elevation of one or more fatty liver disease or disorder risk factor levels. The subject can be on a diet that may cause one or more symptoms that are associated with a fatty liver disease or disorder. The subject may consume alcohol excessively. The subject may be on a diet that is high in red meat, including beef, pork, lamb, and the like. The subject may be on a diet that is high in processed food.

### E. BMI

The subject may have different body weights. The subject may be overweight or obese. Being overweight or obese means the body weight of the subject is above an ideal weight range. The ideal weight range is about 18-25 kg/m² in body mass index (BMI). The BMI is calculated by the number of weight of the subject in kilograms divided by the square of the number of the height of the subject in meters. The subject may have a BMI that is about 25-30 kg/m² or higher than 25 kg/m². For example, the subject may have a BMI that is about 25, 25.5, 26, 26.5, 27, 27.5, 28, 28.5, 29, 29.5, 29.9 or 30 kg/m². The subject may have a BMI that is higher than about 30 kg/m². For example, the subject may have a BMI that is about 30.5, 31, 32, 33, 34, 35, 36, 37, 38, 39 or 40 kg/m². The subject may have a BMI that is higher than about 40 kg/m². The subject may weight more than about 50, 75, 100, 125, 150, 160, 170, 180, 190, 200, 210, 220, 230, 240,250, 260, 270, 280, 290, 300, 310, 320, 330, 340, 350, 360, 370, 380, 390, or 400 lbs.

### F. Habits

The subject may have a habit or may be lack of a habit. "Habit" refers to an activity that is performed by a subject routinely or in a frequency that is higher than normal. In some embodiments, the habit can put the subject to have or suspected to have a fatty liver disease or disorder. In some embodiments, the habit can increase fatty liver disease or disorder risk factor levels in the bloodstream or serum in the subject. The subject may have a habit of smoking or inhaling smokes. The subject may have a habit of smoking tobacco products, including but are not limited to cigarettes, cigars, or pipes. The subject may be in an environment that contains smoke or second-hand smoke. In some cases, the subject may have or suspected to have a fatty liver disease or disorder because the subject does not have a habit that can lower the risk of a fatty liver disease or disorder. For example, the subject may not have a habit of exercising or performing physical activities. The subject may exercise or perform physical activities in a frequency that is lower than normal. The subject may exercise, on average, about once in a week, a month, 2, 3, 4, 5, 6, 7, 8, 9, 10, 11, 12 months, 2, 3, 4, 5, 6, 7, 8, 9, 10 years. The subject may exercise about less than once in a week, a month, 2, 3, 4, 5, 6, 7, 8, 9, 10, 11, 12 months, 2, 3, 4, 5, 6, 7, 8, 9, 10 years. The subject may be immobilized.

### G. Ethnic groups

The human subject can come from different ethnic groups, geographical regions, countries, continents and races. For example, the human subject can be an Asian (e.g., far-east Asian, middle-east Asian, south-east Asian, north-east Asian, or Asian Indian), a Caucasian (Canadian, American, European, or Mediterranean), an African, a pacific islander, or a Hispanic. The human subject can come from Khoid (Hottentot) race, Sanid (Bushmen) race, Central Congoid race (Geographic center and origin in the Congo river basin), Bambutid race (African Pygmies), Aethiopid race (Ethiopia, Somalia), Mediterranid race (from Mediterranean areas), Dinaric race (predominant in western Balkans [Dinaric Mountains] and northern Italy), Alpine race, Ladogan race (named after Lake Ladoga; indigenous to Russia; includes Lappish subrace of arctic Europe), Nordish or Northern European race, Armenid race (Armenia, Syria, Lebanon and northern Iraq), Turanid race (Kazakhstan, Hungary and Turkey), Irano-Afghan race (Iran and Afghanistan, Iraq, Turkey), Indic or Nordindid race (Pakistan and northern India), Dravidic race (India, Bangladesh and Sri Lanka), Veddoid race (remnant Australoid population in central and southern India) Melanesian race (New Guinea, Papua, Solomon Islands), Australian-Tasmanian race (Australian Aborigines), Northeast Asian or Northern Mogoloid race (China, Manchuria, Korea and Japan), Southeast Asian or Southern Mongoloid race (China, Indochina, Thailand, Myanmar [Burma], Malaysia, Indonesia and the Philippines), Micronesian-Polynesian race, Ainuid race (remnants of aboriginal population in northern Japan), Tungid race (Mongolia and Siberia, Eskimos), Amerindian race (American Indians). The human subject can come from Afghanistan, Albania, Algeria, Andorra, Angola, Antigua and Barbuda, Argentina, Armenia, Australia, Austria, Azerbaijan, Bahamas, Bahrain, Bangladesh, Barbados, Belarus, Belgium, Belize, Benin, Bhutan, Bolivia, Bosnia and Herzegovina, Botswana, Brazil, Brunei, Bulgaria, Burkina Faso, Burma, Burundi, Cambodia, Cameroon, Canada, Cape Verde, Central African Republic, Chad, Chile, China, Colombia, Comoros, Congo, Costa Rica, Cote d'lvoire, Croatia, Cuba, Cyprus, Czech Republic, Denmark, Djibouti, Dominican Republic, East Timor, Ecuador, Egypt, El Salvador, Equatorial Guinea, Eritrea, Estonia, Ethiopia, Fiji, Finland, France, Gabon, Gambia, Georgia, Germany, Ghana, Greece, Grenada, Guatemala, Guinea, Guinea-Bissau, Guyana, Haiti, Holy See, Honduras, Hong Kong, Hungary, Iceland, India, Indonesia, Iran, Iraq, Ireland, Israel, Italy, Jamaica, Japan, Jordan, Kazakhstan, Kenya, Kiribati, Korea, Kosovo, Kuwait, Kyrgyzstan, Laos, Latvia, Lebanon, Lesotho, Liberia, Libya, Liechtenstein, Lithuania, Luxembourg, Macau, Macedonia, Madagascar, Malawi, Malaysia, Maldives, Mali, Malta, Marshall Islands, Mauritania, Mauritius, Mexico, Micronesia, Moldova, Monaco, Mongolia, Montenegro, Morocco, Mozambique, Namibia, Nauru, Nepal, Netherlands, Netherlands Antilles, New Zealand, Nicaragua, Niger, Nigeria, North Korea, Norway, Oman, Pakistan, Palau, Palestinian Territories, Panama, Papua New Guinea, Paraguay, Peru, Philippines, Poland, Portugal, Qatar, Romania, Russia, Rwanda, Saint Kitts and Nevis, Saint Lucia, Saint Vincent and the Grenadines, Samoa, San Marino, Sao Tome and Principe, Saudi Arabia, Senegal, Serbia, Seychelles, Sierra Leone, Singapore, Slovakia, Slovenia, Solomon Islands, Somalia, South Africa, South Korea, South Sudan, Spain , Sri Lanka, Sudan, Suriname, Swaziland , Sweden, Switzerland, Syria, Taiwan, Tajikistan, Tanzania, Thailand, Timor-Leste, Togo, Tonga, Trinidad and Tobago, Tunisia, Turkey, Turkmenistan, Tuvalu, Uganda, Ukraine, United Arab Emirates, United Kingdom, Uruguay, Uzbekistan, Vanuatu, Venezuela, Vietnam, Yemen, Zambia, or Zimbabwe.

### III. Treatment indications

The methods and compositions of the present disclosure are useful for the treatment of subjects having fatty liver related disorders and are known to have or suspected of having non or early stage hepatocyte apoptosis. The methods and compositions comprise administration of EPAs. The methods and compositions can further comprise administration of an estrogen.

### A. Fatty liver disorders

This disclosure provides compositions and methods for treating fatty liver disorders which may include but are not limited to non-alcoholic steatohepatitis (NASH), non-alcoholic associated fatty liver disease, secondary NAFLD, steatosis, progressive fibrosis, liver failure and cirrhosis. As used herein, secondary NAFLD may refer to NAFLD or similar symptoms that result from the use of one or more of the following medications: amiodarone, antiviral drugs such as nucleoside analogues, aspirin or NSAIDs, corticosteroids, methotrexate, nifedipine, perhexiline, tamozifen, tetracycline, and valproic acid. The term fatty liver disorder, NASH, as described herein, may be referred to and used interchangeably as NASH herein.

### B. Hepatocyte Apoptosis

This disclosure also provides compositions and methods for treating NASH subjects who are known to have or suspected of having non or early stage hepatocyte death. As herein described, hepatocyte death generally relates to any modes of cell death of liver cells. Cell death may include mechanisms or biochemical pathways characterized by apoptosis, necrosis, necroptosis, autophagy or cornification, especially by apoptosis. Hepatocyte apoptosis may result from any form of liver injury or disease, including but not limited to cancer and fatty liver disorders which may further include non-alcoholic steatohepatitis (NASH), non-alcoholic associated fatty liver disease, secondary NAFLD, steatosis, progressive fibrosis, liver failure and cirrhosis.

### i. Caspase-dependent Apoptosis

In some cases, hepatocyte apoptosis may be induced by caspase-dependent or caspase-independent pathways. Biochemically, apoptosis may be commonly initiated and executed by activation of intracellular caspases (cysteine-dependent aspartate specific protease). Like other intracellular proteases, caspases are synthesized as zymogens that must undergo proteolytic cleavage to exert proteolytic activity. Caspases have a dominant specificity for protein substrates containing four- or five-amino acid sequences containing an aspartate residue in the P1 position (the amino acid on the NH₂-terminal side of the scissile bond), a unique feature of this class of proteases. Caspases themselves must be cleaved at aspartate residues for activation, and thus caspases are activated by other caspases. These proteases may be classified as initiator caspases, which contain a long prodomain for scaffolding with other proteins, and executioner (effector) caspases, containing a short prodomain. Initiator caspases include caspases 2, 8, 9, and 10. The initiator caspases 8 and 10 are involved in death receptor-mediated apoptosis, whereas caspase 9 initiates apoptosis following mitochondrial dysfunction. "Executioner caspases" such as caspases 3, 7, and 6 are activated by cleavage by initiator caspases at aspartate residues and can be activated by either death receptor or mitochondrial pathways of apoptosis. These caspases may activate caspase-activated DNase (CAD) by cleaving ICAD, an inhibitor of this enzyme. CAD activation results in DNA cleavage at internucleosomal linker regions of DNA resulting in the ladder pattern of DNA cleavage (multiples of the 180-bp nucleosomal regions) that may be characteristic of apoptosis.

### ii. sFas in hepatocyte apoptosis

Death receptors such as Fas are a subgroup of the TNF/nerve growth factor (NGF) receptor super family that may play a role in inducing apoptosis in hepatocytes. These cytokine receptors are type Itransmembrane proteins (single membrane-spanning proteins with an extracellular NH₂ terminus) with three distinct regions: an extracellular, NH₂-terminal ligand-interacting domain characterized by serial cysteine-rich repeat domains, a membrane-spanning region, and an intracellular COOH-terminal domain characterized by a stretch of ∼60-80 amino acids known as the "death domain" necessary for the initiation of cytotoxic signals when engaged by cognate ligands. Some death receptors, namely, Fas (CD95/APO1), TNF-R1 (p55/CD120a), TRAIL-R1 (death receptor 4-DR4), and TRAIL-R2 (death receptor 5-DR5/APO-2/KILLER), are ubiquitously expressed in the liver. These receptors bind to specific ligands, the majority of which are type II transmembrane proteins (single membrane-spanning proteins with an extracellular COOH terminus) belonging to the TNF family. Engagement of death receptors by their corresponding ligands such as Fas ligand (FasL) or soluble Fas ligand (sFasL) may trigger the so-called extrinsic pathways of apoptosis, a signaling cascade resulting in activation of effector caspases and cell death. The liver contains a high level of expression of death receptors and renders it more susceptible to excessive apoptosis by this pathway. Death receptors expressed in the liver have been implicated in various liver injuries in different pathological settings.

The Fas receptor is constitutively expressed by every cell type in the liver. Its ligand, FasL, may be expressed as a transmembrane protein on the cell surface of activated cytotoxic T lymphocytes (CTL) but is also found in the soluble non membrane bound form (sFasL). Both forms of FasL, including sFasL, may play a role in hepatocyte apoptosis. In certain pathological conditions, sFasL can also be expressed by hepatocytes and induce fratricide cell death of sFasL-expressing hepatocytes, amplifying the tissue damage. Kupffer cells also express FasL in response to engulfment ofapoptotic bodies, a process that may, under pathological conditions, exacerbate hepatocyte apoptosis and liver injury and promote liver inflammation and fibrosis such as in fatty liver related diseases such as NASH or NAFLD. sFas may be expressed as an alternative splice variant in response to a pathological condition such as NASH.

The FasL may bind to preoligomerized Fas receptor on the plasma membrane to initiate the signaling cascade. FasL binding induces a conformational change in the receptor intracellular domain which results in recruitment of the adaptor protein FADD (Fas-associated protein with death domain) and pro-caspase 8 and/or 10, after relocalization of the receptor to lipid rafts. Multiple receptors may then be recruited to the lipid rafts to form larger aggregates that are subsequently internalized via clathrin-mediated endocytosis and delivered to the early endosomal compartment, a step required for efficient complex formation and amplification of the death signal. This complex, comprising Fas, FasL, FADD, and caspase 8, is referred to as death-inducing signaling complex (DISC) which triggers death of the cell.

Dysregulation of Fas and sFas apoptosis has been associated with several liver diseases. Toxic bile acids accumulating within the hepatocyte in cholestatic disease are known to induce hepatocyte apoptosis in a sFas-mediated manner. Elevated intracellular concentrations of bile acids can induce Fas translocation from the cytosol and Golgi complex to the plasma membrane, where the increased surface density facilitates its oligomerization and initiation of the apoptotic signal.

Fas expression and hepatocyte apoptosis are elevated in the liver of NASH patients, and sensitivity to sFasL is increased in steatotic livers compared with normal livers, suggesting that Fas-mediated cell death may be an important feature of NASH and fatty liver diseases. sFas has been shown to be a suitable biomarker for the assessment of NASH disease.

### iii. Cytokeratin-M30 and sFas in Hepatocyte Apoptosis

Cytokeratin-18 fragment M30 (M30) is a type I cytokeratin, or intermediate filament protein that may be cleaved during hepatocyte apoptosis. M30 refers to a soluble fragment or product of this enzymatic cleavage. M30 may be generated by an enzyme, including caspase cleavage, such as with caspase-3, during activation of the caspase cascade pathway was described herein. As referenced in US7883904, M30 may be a suitable biomarker for hepatocyte apoptosis.

### C. Evaluation criteria for subjects with NASH

Generally, any suitable method or combination of methods may be used in evaluating NASH in a subject. In terms of physical symptoms, NASH is generally asymptomatic until severe liver impairment occurs. Patients may generally feel well in the early stages and only begin to have symptoms, such as fatigue, weight loss, and weakness once the disease is more advanced or cirrhosis develops. The progression of NASH may take years, or even decades. The process can stop and, in some cases, reverse on its own without specific therapy. In some cases, NASH may slowly worsen, causing scarring or "fibrosis" to appear and accumulate in the liver. As fibrosis worsens, cirrhosis develops; the liver becomes seriously scarred, hardened, and unable to function normally. Not every person with NASH develops cirrhosis, but once serious scarring or cirrhosis is present, few treatments can halt the progression. A person with cirrhosis experiences fluid retention, muscle wasting, bleeding from the intestines, and liver failure. These physical symptoms in the late stages of the disorder may be used to determine the presence or absence of NASH in a subject.

### i. Biopsy

Due to the asymptomatic nature of the disorder, particularly in early stages of the disease, one or more technique and methods may be used to assess the absence or presence of NASH in a subject. In some cases, a biopsy is performed on the liver, whereby a needle is inserted through the skin to remove a small piece of the liver. NASH is diagnosed when examination of the tissue with a microscope shows fat along with inflammation and damage to liver cells. If the tissue shows fat without inflammation and damage, simple fatty liver or NAFLD is diagnosed. In some cases, a biopsy may also indicate the presence or absence of scar tissue that has developed in the liver. Currently, no blood tests or scans can reliably provide this information.

### ii. Blood tests and biomarkers

While there are no single laboratory tests for NASH, various abnormal levels of liver enzymes, biomarkers and other biological blood components may be used in aiding diagnosis of the disorder. For example, in some cases, elevated serum aminotransferase may indicate NASH. In some cases measuring the level of a plurality of suitable biomarkers in a sample (e.g. serum or plasma) derived from the subject may be used. In some cases such as adipocytokine, apoptosis markers, and/or cell death markers, may be analyzed and compared to reference levels to aid in diagnosis of NASH.

In some cases subjects treated for NASH according to the present disclosure can also be evaluated for baseline scores of the following criteria before treatment, and evaluated after treatment for possible changes in those criteria.

### iii. NAS score

In some cases, patients or subjects treated for NASH according to the present disclosure can also be evaluated using a combination of standardized histologic scores, as known in the art. Due to significant inter-observer variance in the clinical diagnosis of NASH, and the severity of the disorder at given time, a composite score of histologic features using a standardized NASH scoring system has been implemented and can be used to provide a measurement of NASH in a subject. This combined measurement is known as the non-alcoholic fatty liver disease activity score (NAS).

In some cases the NAS score may be determined for a subject prior to initiation of treatment in order to provide a baseline level or score for the criteria as well as evaluation after the dosing regimen to evaluate any improvement in the criteria. In other cases, a NAS score may be determined for a subject undergoing treatment. In some cases, a NAS score and comparison of NAS scores over time may be used in assess disease progression of NASH.

The non-alcoholic fatty liver disease activity score (NAS) is defined as the unweighted sum of the values for steatosis (ranging from 0-3), lobular inflammation (ranging from 0-3) and ballooning (ranging from 0-2), thereby providing a range of NAS score of from 0 to 8. (See Kleinen et al., Design and Validation of a Histological Scoring System for Nonalcoholic Fatty Liver Disease, Hepatology, Vol. 41, No. 6, 2005, pp. 1313-1321) Patients treated for NASH according to the present disclosure can show a NAS score prior to treatment of ≧ 4, with a minimum score of 1 each for steatosis and lobular inflammation plus either ballooning or at least 1 a sinusoidal fibrosis and a finding of possible or definite steatohepatitis. After dosing/treatment, such as for one year, patients can show a composite NAS score of ≦ 3, ≦ 2 or ≦ 1, together with no worsening in fibrosis. Alternatively, patients can show an improvement in NAS by a value of ≧ 2 across at least two of the NAS components, together with no worsening in fibrosis. Alternatively, patients can show an improvement in NAS score by ≧ 3, 4, 5, 6, 7 or 8.

### iv. Steatosis

Steatosis is broadly understood to describe a process involving the abnormal accumulation of lipids within the liver, which inhibits the normal liver functions. Liver biopsy enables analysis and scoring of steatosis in a patient, with scores ranging from 0-3. Patients treated for NASH according to the present disclosure can have a steatosis score of 1, 2 or 3, such as between about 2 and about 3. After treatment, it is desired for patients to exhibit no worsening of steatosis, alternatively a reduction of at least 1 in the steatosis score, or a reduction of 2 or 3 in the steatosis score. Steatosis is traditionally graded with a score of 1 indicating the presence of fat droplets in less than 33% of hepatocytes, a score of 2 indicating fat droplets observed in 33-66% of hepatocytes, and a score of 3 indicating observation of fat droplets in greater than 66% of hepatocytes. (See Kleinen et al., Design and Validation of a Histological Scoring System for Nonalcoholic Fatty Liver Disease, Hepatology, Vol. 41, No. 6, 2005, pp. 1313-1321)

### v. Lobular inflammation

Lobular inflammation is also evaluated upon liver biopsy and scored with values of 0-3. (See Kleinen et al., Design and Validation of a Histological Scoring System for Nonalcoholic Fatty Liver Disease, Hepatology, Vol. 41, No. 6, 2005, pp. 1313-1321 Table 1) Patients to be treated for NASH can have lobular inflammation scores of 1, 2 or 3, alternatively ranging between 1 and 2 or 2 and 3. After treatment, patients can have a reduction in lobular inflammation score of at least 1, alternatively a reduction of 2 or 3 in lobular inflammation score, and at least no worsening of the lobular inflammation score.

### vi. Ballooning

Ballooning of hepatocytes is generally scored with values of 0-2, (See Kleinen et al., Design and Validation of a Histological Scoring System for Nonalcoholic Fatty Liver Disease, Hepatology, Vol. 41, No. 6, 2005, pp. 1313-1321 Table 1), and patients treated for NASH according to the present disclosure can have ballooning scores of 0-2, including specific values of 1 or 2, and alternatively a score ranging from 1 to 2. After treatment, patients can show at least no worsening of the ballooning score, alternatively a reduction of at least one value lower in the ballooning score, and alternatively a reduction of two in the value of the ballooning score.

### vii. Fibrosis stage

Fibrosis is also evaluated upon liver biopsy and scored with values of 0-4, the scores being defined as: 0 represents no fibrosis, 1 represents perisinusoidal or periportal fibrosis, 1a represents mild, zone 3, perisinusoidal fibrosis; 1b represents moderate zone 3, perisinusoidal fibrosis; 1c represents portal/periortal fibrosis; 2 represents perisinusoidal and portal/periportal fibrosis; 3 represents bridging fibrosis; and 4 represents cirrhosis. (See Kleinen et al., Design and Validation of a Histological Scoring System for Nonalcoholic Fatty Liver Disease, Hepatology, Vol. 41, No. 6, 2005, pp. 1313-1321) Patients treated according to the present disclosure can have a fibrosis stage score of 0-3, including 0, 1, 1a, 1 b, 1 c, 2 or 3, and can have a fibrosis stage score of at least 1a. After treatment, patients can have a fibrosis stage score that is at least no worse than the baseline score, and alternatively can have a reduction in the fibrosis stage score of at least one level, alternatively at least two or three levels.

### D. Evaluation criteria of hepatocyte apoptosis in NASH subjects

As described herein, the methods and compositions of the present disclosure are useful for the treatment of subjects having fatty liver related disorders and are known, or suspected to have non or early stage hepatocyte apoptosis, by administration of an effective amount of EPAs. The presence of absence non or early stage hepatocyte apoptosis may be determined in a subject using any suitable methods known in the art. Generally, preferred tests for non or early stage hepatocyte apoptosis in NASH patients may be characterized in two groups: histological based tests and biochemical based tests. Histological based tests may include but are not limited to a staining of tissue slice or visualization by fluorescent microscopy. Biochemical based tests may include but are not limited to serum level measurements of sFas and M30, liver function tests, liver enzyme tests or a composite NASH risk score as provided by Tamimi TI., et.al., J. Hepatol., 54, 1224-1229, 2011. In some cases, one or more tests may be used to characterize hepatocyte apoptosis. In some cases a combination of tests may be used to assess hepatocyte apoptosis in NASH subjects.

### i. Histological tests

Generally, the histological tests, examples of which are provided herein, provide a visualization of the liver, which may aid in the diagnosis of an hepatocyte apoptosis. For example, if apoptosis is present, histological samples may be taken to visualize tissue. In some cases visualization is achieved with differential staining such as with hetaoxylin and eosin-stains. Other stains, specific for dead or dying cells may also be used to visualize hepatocyte apoptosis. In some cases, fluorescent microscopy examination may be also used to visualize apoptotic cells. In some cases, visualization of other histological or cellular indicators such as an elevated presence of lysomes may also be used to assess hepatocyte apoptosis.

### ii. Measurement of sFas

In some cases, sFas may be used as a biochemical marker for the assessment of hepatocyte apoptosis. In some cases, sFas is measured as protein in serum or plasma of a subject having or suspected of having NASH. Blood test results for sFas suggest a level equal to or less than 10.0 ng/mL, preferably a level equal to or less than 9.5 ng/mL for a subject having non or early stage hepatocyte apoptosis.

In some cases, elevated serum or plasma sFas levels may be indicative of diseases of the liver and hepatocyte apoptosis. Elevated sFas may also been found to correlate with cardiovascular diseases in NASH subjects. sFas may circulate and may indicate specific pathologies such as metabolic syndrome, alcohol addiction and chronic liver disease. High body mass index (BMI) may be associated with type 2 diabetes subjects with high sFas. Tests for sFas levels may be used alone or in combination with other tests to assess hepatocyte apoptosis in NASH subjects.

Generally, any suitable means for assessing sFas levels may be used. In some cases, sFas may be determined using any suitable biochemical assay including but not limited to immunoassay, ELISA, western blot, PCR, activity assays, binding assays, chromatography, spectrometry, mass spectrometry and the like.

For example, sFas may be measured by any non commercial or commercial kits available. Commercial kits may include but are not limited to Quantikine Human soluble Fas Kit (R&D systems, Minneapolis, MN), sFas ELISA Kit (Orient Yeast, Osaka, Japan), Human Soluble Fas Ligand ELISA Kit (Kamiya Biomedical, Seattle, WA) or FAS Ligand (Soluble) Human ELISA Kit (Invitrogen, Camarillo, CA). The concentration of the sFas in the sample can be usually expressed in nanogram per milliliter (ng/mL) or picogram per milliliter (pg/mL).

The assayed level of sFas can be correlated with liver damage and/or liver disease progression by comparing the level of hepatocyte apoptosis with a predetermined value. In one aspect of the invention, the predetermined value can be based upon sFas in comparable samples obtained from the general population or from a select population of human subjects. For example, the select population may be comprised of apparently healthy subjects. In other words, such individuals, if examined by a medical professional, would be characterized as healthy and free of symptoms of liver disease.

### iii. Measurement of M30

In some cases, M30 may be used as a biochemical marker for the assessment of hepatocyte apoptosis. In some cases, M30 is measured as protein in serum or plasma of a subject having or suspected of having NASH. Blood test results for M30 suggest a level equal to or less than 1500 IU/L, preferably a level equal to or less than 900 IU/L, more preferably a level equal to or less than 500 IU/L for a subject having non or early stage hepatocyte apoptosis.

In some cases, elevated serum or plasma M30 levels may be indicative of diseases of the liver and hepatocyte apoptosis. Elevated M30 fragments may also been found to correlate with cardiovascular diseases in NASH subjects. M30 may circulate and may indicate specific pathologies such as metabolic syndrome, alcohol addiction and chronic liver disease. High body mass index (BMI) may be associated with type 2 diabetes subjects with high serum M30 levels. Tests for M30 levels may be used alone or in combination with other tests to assess hepatocyte apoptosis in NASH subjects.

Generally, any suitable means for assessing M30 levels may be used. In some cases, M30 may be determined using any suitable biochemical assay including but not limited to immunoassay, ELISA, western blot, PCR, activity assays, binding assays, chromatography, spectrometry, mass spectrometry and the like.

For example, M30 may be measured by any non commercial or commercial kits available. Commercial kits may include but are not limited to M30-Apoptosense ELISA (Peviva, Bromma, Sweden) or Human Cytokeratin 18-M30,CK 18-M30 ELISA kit (Cusabio, Newark, DE).

In some cases, M30 can be detected and/or quantified using an immunoassay, such as an enzyme-linked immunoabsorbent assay (ELISA). In an ELISA, antibodies specific to a particular antigen are used to detect the presence of, or measure the amount of, a particular molecule. By way of example, an M30-APOPTOSENSE (PEVIVA, Grünwald, Germany) ELISA may be used to detect the presence of, or measure the amount of or level of, caspase 3-generated CK-18 fragments. The APOPTOSENSE assay uses the M30 antibody, which recognizes a neo-epitope in the C-terminal domain of CK-18, which is exposed after cleavage of CK-18 by caspases (e.g., caspase 3) after aspartic acid residue 396. The M30 antibody used in the APOPTOSENSE assay is a mouse monoclonal antibody of the IgG2b subtype.

An ELISA typically comprises the steps of contacting a sample taken from a subject with one or more antibodies, and then assaying for the formation of a complex between the antibody and a protein or peptide in the sample. For ease of detection, the antibody can be attached to a substrate such as a column, plastic dish, matrix, or membrane, such as nitrocellulose. The sample may be untreated, subject to precipitation, fractionation, separation, or purification before combining with the antibody. The APOPTOSENSE assay, for example, is a solid-phase, two-site immunosorbent assay. In some cases bodily samples, such as blood serum, may be simultaneously reacted with the mouse monoclonal antibody M5 (directed against CK-18 and immobilized to a polystyrene well) and a horseradish peroxidase-conjugated M30 monoclonal antibody. Following the formation of the solid phase/antigen/labeled antibody sandwich, excess unbound conjugate may be removed by a washing step. It should be noted that the APOPTOSENSE assay may also be used in combination with the M65 ELISA (PEVIVA, Grünwald, Germany) which measures total CK-18. Combining the two assays allows the calculation of the relative fraction of CK-18 that is caspase-cleaved.

In an ELISA, interactions between the antibody or antibodies in the sample and the protein(s) or peptide fragment(s) are detected by radiometric, colorimetric, or fluorometric means, size-separation, or precipitation. In one example, detection of the antibody-protein or peptide complex is by addition of a secondary antibody coupled to a detectable tag, such as an enzyme, fluorophore, or chromophore. In the present invention, tetramethyl-benzidine substrate may be added to the assay and color develops in proportion to the bound analyte. The color development may then be stopped and color intensity measured in a microplate reader at 450 nm. By plotting a standard curve from known concentrations versus measured absorbance, the amount of caspase 3-generated CK-18 fragments in the bodily sample can be calculated. The concentration of the M30 fragment in the sample can be expressed in Units per Liter (U/L).

Additional assays can be used to detect and/or quantify M30 fragment in the bodily sample. These additional assays can include other immunoassays, such as immunoassays employing antibodies disclosed in U.S. Pat. Nos. 6,296,850, 6,716,968, 6,706,488 and 7883904 all of which are incorporated herein by reference in their entirety. Other immunoassays that use CK-18 detecting antibodies can be used to detect the CK-18 fragments. These assays can include radioimmunoassays, both solid and liquid phase, fluorescence-linked assays, and competitive immunoassays as well as other assays, such as mass spectrometry (MS)-based methods (e.g., liquid chromatography MS and electrospray ionization MS). MS based methods may be useful for detecting and/or quantifying the level of M30 fragments as, for example, the parent molecules, i.e., the non-cleaved M30 fragment molecules will have different masses than the M30 fragment. Additionally, methods such as HPLC may also be useful for detecting the presence of M30 fragment because distinct parent-daughter ion transitions can occur after cleavage of CK-18 by caspase 3. Thus, charge differences and changes in polarity between cleaved and non-cleaved CK-18 proteins have a high likelihood of showing distinct retention times on HPLC.

The assayed level of M30 can be correlated with liver damage and/or liver disease progression by comparing the level of hepatocyte apoptosis with a predetermined value. In one aspect of the invention, the predetermined value can be based upon the level of Ck-18 fragments (e.g., IU/L) in comparable samples obtained from the general population or from a select population of human subjects. For example, the select population may be comprised of apparently healthy subjects. In other words, such individuals, if examined by a medical professional, would be characterized as healthy and free of symptoms of liver disease.

### iv. Additional biochemical based tests

Generally, any suitable biochemical tests may be used in the assessment of hepatocyte apoptosis in NASH subjects. In some cases, tests, as part of a standard liver function panel, may be used to assess hepatocyte apoptosis. In some cases, the state of lysosomes may be assessed by determining the proportion of lysosomal enzymes (β-galactosidase, β-glucuronidase, cathepsin C and cathepsin B) present in a sample, such as liver tissue. Apoptosis may also be monitored by measuring caspase 3 activity (DEVDase) or the measurement of sulfite cytochrome c reductase, an enzyme located in the mitochondrial intermembrane space that may be released upon apoptotic pathways in liver cells. Generally, any protein, gene or biomarker known to be involved in cell death or apoptosis related pathways of liver cells may be used to assess hepatocyte apoptosis.

### iv. General thresholds for evaluating hepatocyte apoptosis

The tests recommended for screening are the same as those for making the diagnosis, with the result that a positive screen is equivalent to a diagnosis of non or early stage hepatocyte apoptosis. In this case, non or early stage hepatocyte apoptosis may also comprise a subject being at risk for non or early stage hepatocyte apoptosis. In some cases, non or early stage hepatocyte apoptosis is diagnosed by having one or both of the following: 1) sFas levels equal to or less than at least 10.0, 9.9, 9.8, 9.7, 9.6, 9.5, 9.4, 9.3, 9.2, 9.1, 9.0, 8.9, 8.8, 8.7, 8.6, 8.5, 8.4, 8.3, 8.2, 8.1, or 8.0 ng/mL or sFas levels equal to or less than at most 10.0, 9.9, 9.8, 9.7, 9.6, 9.5, 9.4, 9.3, 9.2, 9.1, 9.0, 8.9, 8.8, 8.7, 8.6, 8.5, 8.4, 8.3, 8.2, 8.1, or 8.0 ng/mL; 2) M30 levels equal to or less than at least 1500, 1400, 1300, 1200, 1100, 1000, 900, 800, 700, 600, or 500 IU/L or M30 levels equal to or less than at most 1500, 1400, 1300, 1200, 1100, 1000, 900, 800, 700, 600, or 500 IU/L; a NASH risk score equal to or less than at least 3.0, 2.9, 2.859, 2.8, 2.7, 2.6, 2.5, 2.4, 2.3, 2.2, 2.1 or 2.0 or a NASH risk score equal to or less than at most 3.0, 2.9, 2.859, 2.8, 2.7, 2.6, 2.5, 2.4, 2.3, 2.2, 2.1 or 2.0. In some cases, to get a diagnosis of non or early stage hepatocyte apoptosis, patients may also satisfy one of the following criteria: 1) symptoms of hepatocyte apoptosis (i.e. histological visualization, cirrhosis of the liver or other clinical symptoms related to liver damage) and levels of sFas or M30 corresponding to non or early stage hepatocyte apoptosis. Additional diagnostic criteria of one or more of these indicators may also indicate hepatocyte apoptosis. Subjects having early stages of hepatocyte apoptosis may have levels at or about threshold levels as described herein.

### IV. Pharmaceutical Compositions

### A. EPA-E composition

Eicosapentaenoic acid (EPA) is a known omega-3 polyunsaturated, long-chain fatty acid. Omega-3 fatty acids are known as components of oils, such as fish oil. A variety of commercial products are promoted as containing omega-3 fatty acids, or their salts, esters, phospholipids, derivatives, conjugates and the like. Eicosapentaenoic acid (EPA) is also known as its ethyl ester form, ethyl eicosapentanoate (EPA-E). According to the present disclosure, EPAs (including, but not limited to EPA or EPA-E) can be administered in a composition. Content of EPAs in the total fatty acid of the compositions of the present disclosure are not particularly limited as long as the composition contains EPAs as its effective component and intended effects of the present disclosure are attained, high purity EPAs is preferably used.

The present disclosure may be a self-emulsifying composition comprising 50 to 95% by weight in total of EPAs of preferably 60% by weight or more, more preferably 90% by weight or more, and still more preferably 96.5% by weight or more in total of the fatty acids and their derivatives. In some cases, the pharmaceutical composition may comprise at least about 40%, 46%, 50%, 55%, 60%, 65%, 70%, 75%, 80%, 85%, 90%, 95%, 96%, 96.5% or 98% EPAs in total of the fatty acids and their derivatives. In some cases, the pharmaceutical composition may comprise at most about 40%, 46%, 50%, 55%, 60%, 65%, 70%, 75%, 80%, 85%, 90%, 95%, 96%, 96.5% or 98% EPAs in total of the fatty acids and their derivatives. EPAs can be administered to patients in a highly purified form, including the product known as Epadel® (Mochida Pharmaceutical Co., Ltd., Tokyo Japan) and pharmaceutically acceptable amides, salts, esters and phospholipids thereof.

The EPAs used in the present disclosure may be a synthetic, semi-synthetic, natural EPAs, or a natural oil containing such EPAs. Examples of the natural EPAs include an extract from a natural oil containing an EPAs, a crudely purified natural oil containing an EPAs, and a highly purified natural oil containing an EPAs produced by a method known in the art. Exemplary semi-synthetic EPAs include EPAs produced by a microorganism or the like and the EPAs or the natural EPAs which have been subjected to a chemical treatment such as esterification or ester exchange.

### B. Estrogen composition

Estrogens or Oestrogens are a group of compounds named for their importance in both menstrual and estrous reproductive cycles. They are the primary female sex hormones. Natural estrogens can be steroid hormones, while some synthetic ones can be non-steroidal. Estrogens can be used as part of some oral contraceptives, in estrogen replacement therapy for postmenopausal women, and in hormone replacement therapy for trans women. Estrogens can readily diffuse across the cell membrane. Once inside the cell, they can bind to and activate estrogen receptors which in turn modulate the expression of many genes.

The methods and compositions in current invention can comprise estrogen. The estrogen can be any naturally or synthetic estrogen or its analogs, precursors or metabolites. There are three major naturally occurring estrogens in women: estrone (E1), estradiol (E2), and estriol (E3). Estradiol can be the predominant estrogen during reproductive years both in terms of absolute serum levels as well as in terms of estrogenic activity. During menopause, estrone is the predominant circulating estrogen and during pregnancy estriol is the predominant circulating estrogen in terms of serum levels. Though estriol is the most plentiful of the three estrogens it is also the weakest, whereas estradiol is the strongest with a potency of approximately 80 times that of estriol. Thus, estradiol can be the most important estrogen in non-pregnant females who are between the menarche and menopause stages of life. However, during pregnancy this role shifts to estriol, and in postmenopausal women estrone becomes the primary form of estrogen in the body. Another type of estrogen called estetrol (E4) is produced during pregnancy. All of the different forms of estrogen can be synthesized from androgens, specifically testosterone and androstenedione, by the enzyme aromatase. Premarin, a commonly prescribed estrogenic drug produced from the urine of pregnant mares, contains the steroidal estrogens equilin and equilenin. There are estradiol skin patches such as Estraderm (the original brand, introduced in the late 1980s) that offer a completely natural alternative. The composition can comprise any types of estrogen or any combination thereof. In some embodiments, the estrogen is estradiol.

In some cases, the pharmaceutical composition as described herein can comprise EPAs and estrogen. The estrogen can be estradiol, or any combination of estrogens and/or its analogs. The pharmaceutical composition may comprise an effective amount of estrogen. The effective amount can be an amount that is capable of elevating serum level of estrogen to a level that is described elsewhere in the current invention.

The composition can comprise EPAs and estrogen separately or mixed together. The estrogen can be packaged or formulated in any know methods in the art. The EPAs and estrogen can be separately packaged, and mixed prior to administration. The EPAs and estrogen can be separately packaged and separately administered. The EPAs and estrogen can be packaged together. The EPAs and estrogen can be co-administered. The estrogen composition can be administered orally, transdermally or intravenously. The EPAs and estrogen can be packaged in two different forms. For example, the EPAs can be packaged in a capsule and estrogen can be packaged in a tablet. The estrogen can have a purity that is at least 90%, 95%, 99%, 99.5%.

### C. Formulation

In some embodiments, EPAs are formulated as a self-emulsifying composition. The self-emulsifying composition of the present disclosure may preferably have at least one of the effects including excellent self-emulsifying property, excellent dispersibility in the composition, excellent emulsion stability, excellent storage stability, excellent absorption property, and in particular, excellent absorption property and rate under fasting, and excellent convenience or compliance for the patients so that the composition can exhibit pharmacological effect of the EPAs. In some cases, EPAs may be combined with 5 to 50% by weight of an emulsifier having an HLB of at least 10.

In some cases, the pharmaceutical composition may comprise at least about 5%, 10%, 15%, 20%, 25%, 30%, 35%, 40%, 45%, and 50% emulsifier. In some cases, the pharmaceutical composition may comprise at most about 5%, 10%, 15%, 20%, 25%, 30%, 35%, 40%, 45% and 50% emulsifier. The self-emulsifying composition is free from ethanol or the ethanol content is low. The present disclosure also provides a drug of such self-emulsifying composition, its production method, and the method of its use.

Examples of the pharmaceutically acceptable salts of the EPAs include inorganic salts such as sodium salts and potassium salts, organic salts such as benzylamine salts and diethylamine salts, salts with basic amino acids such as arginine salts and lysine salts, and exemplary esters include alkyl esters such as ethyl ester, and esters such as mono-, di- and TG. Preferable examples include ethyl ester and TG ester, and the more preferred is ethyl ester. More specifically, preferable examples include EPA-E, TG ester of EPAs, and the like.

The EPAs used for the starting material of the self-emulsifying composition of the present disclosure is not particularly limited for its purity. The purity is typically such that content of the EPAs in total of the fatty acids and their derivatives in the composition of the present disclosure could be preferably at least 40%, 45%, 46.5%, 50%, 51%, 52%, 53%, 54%, 55%. 56%, 57%, 58%, 59%, 60%, 65%, 70%, 75% by weight, more preferably at least 80% by weight, still more preferably at least 85% by weight, still more preferably at least 90% by weight, and most preferably at least 96.5% by weight. The EPAs containing EPAs and DHAs at a high purity, for example, the one with the content of (EPAs+DHAs) in relation to the EPAs of at least 50%, 55%, 60%, 65%, 70%, 75%, 80%, 85% by weight in total of the fatty acids and their derivatives is preferable, and the content is more preferably at least 90% by weight in total of the fatty acids and their derivatives, still more preferably at least 96.5% by weight in total of the fatty acids and their derivatives, and most preferably at least 98% by weight in total of the fatty acids and their derivatives. In other words, the composition of the present disclosure preferably has a high purity of EPAs in the total fatty acid.

For example, when EPA-E and DHA-E are used, compositional ratio of EPA-E/DHA-E and content of (EPA-E+DHA-E) in relation to total fatty acid are not particularly limited as long as the purity of EPAs in the composition of the present disclosure is in the range as described above. However, the compositional ratio of the EPA-E/DHA-E is preferably at least 0.5, 0.6, 0.7, 0.8, 0.9, 1.0, 1.1, 1.2, 1.5, more preferably at least 2.0, and most preferably at least 2.5.

The composition of the present disclosure may also contain an omega-3 polyunsaturated fatty acid other than the EPAs and DHAs such as DPA, hexadecatrienoic acid (HTA), alpha-linolenic acid (ALA), eicosatrienoic acid (ETE), stearidonic acid (SDA), eicosatetraenoic acid (ETA), heneicosapentaenoic acid (HPA), tetoracosapentaenoic acid (TPA) or tetracosahexaenoic acid (THA) or the pharmaceutically acceptable amide, salt or ester thereof. However, content of omega-3 polyunsaturated fatty acids other than the EPAs and DHAs preferably low, more preferably less than 60%, 55%, 50%, 45%, 40%, 35%, 30%, 25%, 20%, 15%, 10% , 5%, 4%, 3%, 2% by weight, still more preferably less than 1% by weight. The composition of the present disclosure may also contain an omega-6 polyunsaturated fatty acid other than the EPAs such as linoleic acid, γ linolenic acid, or dihomo-γ-linolenic acid or the pharmaceutically acceptable amide, salt or ester thereof. However, content of omega-6 polyunsaturated fatty acids preferably low, more preferably less than 5%, 4%, 3%, 2%, 1%, 0.5% by weight, still more preferably less than 0.2% by weight. In particular, the content of arachidonic acid or the pharmaceutically acceptable salts or esters in total of the fatty acids and their derivatives is preferably low, more preferably less than 2%, 1%, 0.5%, 0.2%, 0.1% by weight, still more preferably less than 0.05% by weight, and most preferably, the composition is substantially free from the arachidonic acid or the pharmaceutically acceptable salt or ester.

In some embodiments, the composition of the present invention comprise EPAs and DPA, alternatively EPAs, DPA and HPA, alternatively EPAs, DPA and TPA, alternatively EPAs, DPA, HPA and TPA. The preferable EPAs: DPA ratio is between 99:1 and 1:99, between 90:1 and 1:90, between 60:1 and 1:60, between 40:1 and 1:40, between 30:1 and 1:30, between 20:1 and 1:20, between 10:1 and 1:10, between 5:1 and 1:5 and between 2:1 and 1:2.

In some embodiments, the composition of the present invention comprise EPAs, DHAs and DPA, alternatively EPAs, DHAs, DPA and HPA, alternatively EPAs, DHAs, DPA and TPA, alternatively EPAs, DHAs, DPA, HPA and TPA. The preferable DHAs:DPA ratio is no more than 1:100, no more than 1:95, no more than 1/90, no more than 1:80, no more than 1/70, no more than 1:60, no more than 1:50, no more than 1:40, no more than 1:30, no more than 1:20, no more than 1:10, no more than 1:5, no more than 1:2, no more than 1:1, no more than 2:1, no more than5:1, no more than 10:1, no more than 20:1, no more than 50:1 and no more than 100:1.

Compared to the fish oil or the fish oil concentrate, the EPAs used in the composition or therapeutic agent of the present disclosure contains impurities such as saturated fatty acids and arachidonic acid which are unfavorable for cardiovascular events at a lower content, and this enables realization of the intended action without causing the problems of excessive nutrition or vitamin A intake. When the EPAs in the form of ester is used, a sufficiently stable composition can be obtained by adding a commonly used antioxidant since the ester form has higher oxidation stability than the fish oils which are mainly TG form.

Purified fish oils may also be used for the EPAs, and use of monoglyceride, diglyceride, and TG derivatives and combinations thereof of the EPAs are also preferable embodiments. Various products containing the EPAs are commercially available, for example, Incromega™ DHA27, Incromega™ DHA46, Incromega™ DHA700E, Incromega™ DHA700TG, Incromega™ DHA 500TG, Incromega™ E400200, Incromega™ E4030, Incromega™ E4520, Incromega™ E460180, Incromega™ E5020, Incromega™ E530200, Incromega™ E6814, Incromega™ E1050, Incromega™ E1070, Incromega™ E3322, Incromega™ E3525, Incromega™ E3826, Incromega™ E7010, Incromega™ EPA500EE, Incromega™ EPA500TG, Incromega™ EPA700E, Incromega™ TG0525, Incromega™ TG1040, Incromega™ TG1050, Incromega™ TG3322, Incromega™ TG3322SR, Incromega™ TG4030, Incromega™ TG6015, Incromega™ TG7010, Incromega™ TrioEE and Incromega™ Trio EE (Croda International PLC, Yorkshire, England), and EPAX6500EE, EPAX6015TG, EPAX6015EE, EPAX6000TG, EPAX6000EE, EPAX6000TGN, EPAX6000FA, EPAX5500EE, EPAX5000TG, EPAX4510TG, EPAX4020TG, EPAX4020EE, EPAX2050TG, EPAX7010EE, EPAX1050TG, EPAX1050TGN, K85TG, K85EE, and K80EE (FMC Corporation, Philadelphia, U.S.A.). These products may be purchased and used for the composition of the present disclosure.

In the present disclosure, the "polyoxyethylene hydrogenated castor oil" is a compound prepared by addition polymerization of ethylene oxide to the hydrogenated castor oil which is castor oil having hydrogen added thereto, Various compounds with different average degree of polymerization of ethylene oxide are commercially available, and examples include polyoxyethylene (20) hydrogenated castor oil (NIKKOL HCO-20, Nikko Chemicals Co., Ltd.), polyoxyethylene (40) hydrogenated castor oil (NIKKOL HCO-40, Nikko Chemicals Co., Ltd.), polyoxyethylene (50) hydrogenated castor oil (NIKKOL HCO-50, Nikko Chemicals Co., Ltd.), polyoxyethylene (60) hydrogenated castor oil (NIKKOL HCO-60, Nikko Chemicals Co., Ltd.), and polyoxyethylene (100) hydrogenated castor oil (NIKKOL HCO-100, Nikko Chemicals Co., Ltd.), and the preferred is polyoxyethylene (60) hydrogenated castor oil. These may be used alone or in combination of two or more. In the present disclosure, the "polyoxyethylene hydrogenated castor oil" includes all of such compounds unless otherwise noted.

In the present disclosure, the "polyoxyethylene sorbitan fatty acid ester" is polyoxyethylene ether of a fatty acid ester wherein a part of the hydroxy groups of anhydrous sorbitol have been esterified with a fatty acid. Various compounds with different esterified fatty acid are commercially available, and examples include polyoxyethylene (20) sorbitan monolaurate (NIKKOL TL-10, Nikko Chemicals Co., Ltd.), polyoxyethylene (20) sorbitan monopalmitate (NIKKOL TP-10V, Nikko Chemicals Co., Ltd.), polyoxyethylene (20) sorbitan monostearate (NIKKOL TS-10V, Nikko Chemicals Co., Ltd.), polyoxyethylene (20) sorbitan tristearate (NIKKOL TS-30V, Nikko Chemicals Co., Ltd.), polyoxyethylene (20) sorbitan monoisostearate (NIKKOL TI-10V, Nikko Chemicals Co., Ltd.), polyoxyethylene (20) sorbitan monooleate (NIKKOL TO-10V, Nikko Chemicals Co., Ltd.), and polyoxyethylene (20) sorbitan trioleate (NIKKOL TO-30V, Nikko Chemicals Co., Ltd.), and the preferred are polyoxyethylene (20) sorbitan monolaurate, polyoxyethylene (20) sorbitan monopalmitate, polyoxyethylene (20) sorbitan monostearate, and polyoxyethylene (20) sorbitan monooleate, and the more preferred are polyoxyethylene (20) sorbitan monooleate. These may be used alone or in combination of two or more. In the present disclosure, the "polyoxyethylene sorbitan fatty acid ester" includes all of such compounds unless otherwise noted.

In the present disclosure, the "polyoxyethylene castor oil" is a compound prepared by addition polymerization of ethylene oxide to castor oil. Various compounds having different average ethylene oxide mole number are commercially available, and examples include Kolliphor EL (BASF), Kolliphor EL-P (BASF), NIKKOL CO-3 with an average ethylene oxide mole number of 3 (Nikko Chemicals Co., Ltd.), NIKKOL CO-10 with an average ethylene oxide mole number of 10 (Nikko Chemicals Co., Ltd.), EMALEX C-20 with an average ethylene oxide mole number of 20 (Nippon Emulsion Co., Ltd.), EMALEX C-30 with an average ethylene oxide mole number of 30 (Nippon Emulsion Co., Ltd.), EMALEX C-40 with an average ethylene oxide mole number of 40 (Nippon Emulsion Co., Ltd.), and EMALEX C-50 with an average ethylene oxide mole number of 50 (Nippon Emulsion Co., Ltd.). These may be used alone or in combination of two or more. In the present disclosure, the "polyoxyethylene castor oil" includes all of such compounds unless otherwise noted.

In the present disclosure, the "polyethylene glycol fatty acid ester" is a fatty acid ester of a polyethylene glycol which is a fatty acid polymerized with ethylene oxide. Various compounds with different esterified fatty acid are commercially available, and examples include polyethylene glycol monolaurate (NIKKOL MYL-10, Nikko Chemicals Co., Ltd.), polyethylene glycol monostearate (NIKKOL MYS-10V, MYS-25V, MYS-40V, NYS-45V, and MYS-55V, Nikko Chemicals Co., Ltd.), polyethylene glycol monooleate (NIKKOL MYO-6 and MYO-10, Nikko Chemicals Co., Ltd.), polyethylene glycol distearate (NIKKOL CDS-6000P, Nikko Chemicals Co., Ltd.), and polyethylene glycol diisostearate (NIKKOL CDIS-400, Nikko Chemicals Co., Ltd.). These may be used alone or in combination of two or more. In the present disclosure, the "polyethylene glycol fatty acid ester" includes all of such compounds unless otherwise noted.

In the present disclosure, the "polyoxyethylene polyoxypropylene glycol" is a compound prepared by addition polymerization of ethylene oxide to the polypropylene glycol which is a polymerized propylene oxide. Various compounds having different average degree of polymerization of the propylene oxide and the ethylene oxide are commercially available, and examples include polyoxyethylene (3) polyoxypropylene (17) glycol (Adeka Pluronic L-31, ADEKA), polyoxyethylene (20) polyoxypropylene (20) glycol (Adeka Pluronic L-44, ADEKA), polyoxyethylene (42) polyoxypropylene (67) glycol (Adeka Pluronic P-123, ADEKA), polyoxyethylene (54) polyoxypropylene (39) glycol (Newdet PE-85, Sanyo Chemical Industries, Ltd.), polyoxyethylene (105) polyoxypropylene (5) glycol (PEP101, Sanyo Chemical Industries, Ltd.), polyoxyethylene (120) polyoxypropylene (40) glycol (Adeka Pluronic F-87, ADEKA), polyoxyethylene (160) polyoxypropylene (30) glycol (Adeka Pluronic F-68, ADEKA), polyoxyethylene (196) polyoxypropylene (67) glycol (Lutrol F127, BASF Japan), and polyoxyethylene (200) polyoxypropylene (70) glycol, and the preferred is polyoxyethylene (105) polyoxypropylene (5) glycol. These may be used alone or in combination of two or more. In the present disclosure, the "polyoxyethylene polyoxypropylene glycol" includes all of such compounds unless otherwise noted.

In the present disclosure, the "sucrose fatty acid ester" is an ester of sugar and a fatty acid. Various compounds with different types of the esterified fatty acids and degree of esterification are commercially available, and examples include Surfhope SE PHARMA J-1216 containing 95% of lauric acid in the fatty acid (Mitsubishi-Kagaku Foods Corporation), Surfhope SE PHARMA J-1416 containing 95% of myristic acid in the fatty acid (Mitsubishi-Kagaku Foods Corporation), Surfhope SE PHARMA J-1615 and J-1616 containing 80% of palmitic acid in the fatty acid, (Mitsubishi-Kagaku Foods Corporation), J-1811, J-1815, and J-1816 containing 70% of stearic acid in the fatty acid (Mitsubishi-Kagaku roods Corporation), and Surfhope SE PHARMA J-1715 containing 70% of oleic acid in the fatty acid, which may be used alone or in combination of two or more. The "sucrose fatty acid ester" used in the present disclosure include all of such compounds.

The emulsifier added to the self-emulsifying composition of the present disclosure may have an HLB of at least 10, preferably at least 11, and more preferably at least 12.

Total content of the emulsifier having an HLB of at least 10 in the self-emulsifying composition of the present disclosure is not particularly limited as long as it is at least 10 parts by weight in relation to 100 parts by weight of the EPAs. The content is typically 10 to 100 parts by weight, preferably 10 to 80 parts by weight, and more preferably 10 to 50 parts by weight in relation to 100 parts by weight of the EPAs.

In the present disclosure, the "sorbitan fatty acid ester" is an ester of anhydrous sorbitol and a fatty acid. Various compounds with different types of the esterified fatty acids and degree of esterification are commercially available, and examples include sorbitan monolaurate (NIKKOL SL-10, Nikko Chemicals Co., Ltd.), sorbitan monostearate (NIKKOL SS-10MV, Nikko Chemicals Co., Ltd.), sorbitan monooleate (NIKKOL SO-10V, Nikko Chemicals Co., Ltd.), sorbitan monopalmitate (NIKKOL SP-10V, Nikko Chemicals Co., Ltd.), sorbitan trioleate (NIKKOL SO-30, Nikko Chemicals Co., Ltd.), and sorbitan sesquioleate (NIKKOL SO-15MV, Nikko Chemicals Co., Ltd.).

In the present disclosure, the "glycerin fatty acid ester" is an ester of monoglycerin or polyglycerin and a fatty acid. Various compounds with different types of the esterified fatty acids and degree of esterification are commercially available, and examples include glyceryl monooleate (PECEOL), glyceryl monostearate (NIKKOL MGS-F50SEV, Nikko Chemicals Co., Ltd.), decaglyceryl monooleate (NIKKOL Decaglyn 1-OV, Nikko Chemicals Co., Ltd.), decaglyceryl monolaurate (NIKKOL Decaglyn 1-L, Nikko Chemicals Co., Ltd.), decaglyceryl trioleate (NIKKOL Decaglyn 3-OV, Nikko Chemicals Co., Ltd.), and tetraglyceryl monooleate (NIKKOL Tetraglyn 1-OV, Nikko Chemicals Co., Ltd.).

In the present disclosure, the "lecithin" is a type of glycerophospholipid, and examples include soybean lecithin, enzymatically decomposed soybean lecithin, hydrogenated soybean lecithin, egg yolk lecithin, hydrogenated phospholipid, phospholipid from milk, lysolecithin, phosphatidyl choline, and phosphatidyl serine. The preferred are soybean lecithin, enzymatically decomposed soybean lecithin, hydrogenated soybean lecithin, and egg yolk lecithin, and the more preferred are soybean lecithin. These may be used alone or in combination of two or more. In the present disclosure, the "lecithin" includes all of such compounds unless otherwise noted.

Commercially available products include purified soybean lecithin (Nisshin Oilio), purified egg yolk lecithin (Asahi Kasei Pharma Corporation), and egg yolk lecithin PL-100M (Kewpie Corporation), and use of such product is also possible.

In the present disclosure, the "polyhydric alcohol" is a polyol compound having the structure of a straight chain or cyclic aliphatic hydrocarbon wherein two or more carbon atoms are each substituted with one hydroxy group. Exemplary such polyhydric alcohols include divalent alcohols such as ethyleneglycol, propylene glycol, trimethylene glycol, 1,2-butylene glycol, tetramethylene glycol, 1,3-butylene glycol, 2,3-butylene glycol, and pentamethylene glycol; trivalent alcohols such as glycerin, trimethylolpropane, and 1,2,6-hexane triol, and polyhydric alcohol polymers such as diethylene glycol, dipropylene glycol, triethylene glycol, polyethylene glycol, polypropylene glycol, and polyglycerin, and the preferred are propylene glycol or glycerin. In the present disclosure, the "polyhydric alcohol" includes all of such compounds unless otherwise noted.

Total amount of the lecithin and/or the polyhydric alcohol added in the self-emulsifying composition of the present disclosure is not particularly limited. However, the total amount of the lecithin and/or the polyhydric alcohol is typically 0 to 50 parts by weight, preferably 3 to 40 parts by weight, and more preferably 5 to 30 parts by weight in relation to 100 parts by weight of the EPAs.

The ethanol in the self-emulsifying composition of the present disclosure is preferably used at an amount not causing quality change in the course of capsulation, distribution, or storage, at an amount not causing change in the content of the capsule, and at an amount not exceeding the established upper limit of the daily dose as a drug. The ethanol content is typically up to 10% by weight, preferably up to 4% by weight, more preferably up to 1% by weight, more preferably up to 0.5% by weight, more preferably up to 0.2% by weight, still more preferably up to 0.1% by weight, and most preferably 0% by weight (no ethanol addition).

Preferable ethanol concentration can be adequately determined in consideration of the EPAs concentration in the self-emulsifying composition and the daily dose. When the self-emulsifying composition of the present disclosure is orally administered at a daily dose in terms of the EPA-E of 1800 mg, and for example, the preparation contains 75% by weight of the EPA-E, the maximum daily dose of 3.26 mg described in "Dictionary of Drug Additives (in Japanese)" will not be exceeded when the ethanol content is not more than 0.135% by weight.

The preferable embodiment of the self-emulsifying composition of the present disclosure containing such EPAs and an emulsifier is a combination of EPAs and/or DHAs with at least one emulsifier selected from the group consisting of polyoxyethylene hydrogenated castor oil, polyoxyethylene sorbitan fatty acid ester, polyoxyethylene castor oil, sucrose fatty acid ester, sorbitan fatty acid ester, glycerin fatty acid ester and lecithin. When the self-emulsifying composition of the present disclosure is used as a food such as special purpose food, functional health food, and health food, the preferred is the combination of EPAs and/or DHAs with a sucrose fatty acid ester and/or a lecithin which has good results as a food additive. When a sucrose fatty acid ester is used, the preferable amount is 1% by weight to 20% by weight, more preferably 4% by weight to 20% by weight, and most preferably 4% by weight to 10% by weight in the self-emulsifying composition. The most preferable embodiments are a combination of EPAs and polyoxyethylene (50) hydrogenated castor oil or polyoxyethylene (60) hydrogenated castor oil; a combination of EPAs and polyoxyethylene (20) sorbitan monooleate; a combination of EPAs and polyoxyethylene castor oil; and a combination of EPAs and sucrose fatty acid ester J-1216 or J-1816.

Also preferred is the further combination with a lecithin such as soybean lecithin and/or a polyhydric alcohol such as propylene glycol.

When the emulsifier is at least one member selected from the group consisting of polyoxyethylene hydrogenated castor oil, polyoxyethylene sorbitan fatty acid ester, and polyoxyethylene castor oil, the polyhydric alcohol is preferably a dihydric alcohol, and use of propylene glycol is more preferable. When the emulsifier is a sucrose fatty acid ester, the polyhydric alcohol is preferably a trihydric alcohol, and use of glycerin is more preferable. Preferably, the composition and therapeutic agent of the present disclosure is substantially free from water. The "substantially free from water" means that the water content is up to 10% by weight. The water is preferably used at an amount of 0.5 to 6% by weight, more preferably at 0.5 to 4% by weight, more preferably at 0.5 to 3% by weight, and most preferably at 1 to 3% by weight when the total amount of the self-emulsifying composition is 100% by weight.

The self-emulsifying composition of the present disclosure may also contain additives such as an emulsion aid, stabilizer, antiseptic, surfactant, and antioxidant. Exemplary emulsion aids include fatty acids containing 12 to 22 carbon atoms such as stearic acid, oleic acid, linoleic acid, palmitic acid, linolenic acid, and myristic acid and their salts. Exemplary stabilizers include phosphatidic acid, ascorbic acid, glycerin, and cetanols, and exemplary antiseptics include ethyl paraoxybenzoate and propyl paraoxybenzoate. Exemplary surfactants include sucrose fatty acid esters, sorbitan fatty acid esters, glycerin fatty acid esters, polyglycerin fatty acid esters, polyoxyethylene sorbitan fatty acid esters, polyoxyethylene alkyl ethers, polyoxyethylene fatty acid esters, polyoxyethylene alkyl phenyl ethers, and polyoxyethylene polyoxypropylene alkyl ethers having an HLB of less than 10. Exemplary antioxidants include oil-soluble antioxidants such as butylated hydroxy toluene, butylated hydroxy anisole, propyl gallate, propyl gallate, pharmaceutically acceptable quinone, astaxanthin, and α-tocopherol.

In addition, an adequate carrier or mediator, a colorant, a flavor, and optionally, a vegetable oil or an additive such as non-toxic organic solvent or non-toxic solubilizing agent (for example glycerin), emulsifier, suspending agent (for example, Tween 80 and gum arabic solution), isotonic agent, pH adjusting agent, stabilizer, corrective, flavoring agent, preservative, antioxidant, or absorption promoter commonly used in the art may be adequately combined to prepare an appropriate pharmaceutical preparation.

More specifically, since the EPAs is highly unsaturated, effective amount of an oil-soluble antioxidant, for example, at least one member selected from butylated hydroxytoluene, butylated hydroxyanisole, propyl gallate, propyl gallate, pharmaceutically acceptable quinone, astaxanthin, and α-tocopherol is preferably incorporated in the composition. Storage temperature is preferably room temperature, and frozen storage is preferably avoided since the freezing may result in the loss of self-emulsifying property, dispersibility in the composition, or emulsion stability.

The self-emulsifying composition of the present disclosure can be produced by mixing the EPAs, the emulsifier having an HLB of at least 10, sorbitan fatty acid ester or glycerin fatty acid ester, and the optionally added components such as lecithin, polyhydric alcohol, water and antioxidant with optional heating to dissolve the components.

In some cases, the pharmaceutical composition may comprise additional elements which may include but are not limited to antihypertensives such as angiotensin II receptor blockers such as irbesartan, olmesartan medoxomil, candesartan cilexetil, telmisartan, valsartan, and losartan potassium; angiotensin-converting enzyme inhibitors such as alacepril, imidapril hydrochloride, enalapril maleate, captopril, quinapril hydrochloride, cilazapril hydrate, temocapril hydrochloride, delapril hydrochloride, trandolapril, benazepril hydrochloride, perindopril, and lisinopril hydrate; calcium antagonists such as azelnidipine, amlodipine besylate, aranidipine, efonidipine hydrochloride, cilnidipine, nicardipine hydrochloride, nifedipine, nimodipine, nitrendipine, nilvadipine, bamidipine hydrochloride, felodipine, benidipine, and manidipine; α receptor blocker such as tolazoline, and phentolamine; β receptor blockers such as atenolol, metoprolol, acebutolol, propranolol, pindolol, carvedilol, and labetalol hydrochloride; a receptors stimulant such as clonidine and methyldopa; and diuretics such as eplerenone, hydrochlorothiazide, and furosemide.

In some cases, the pharmaceutical composition may comprise additional elements which may include but are not limited to vitamins such as ascorbic acid (vitamin C), tocopherol (vitamin E), and tocopherol nicotinate, and N-acetylcysteine, probucol.

In some cases, the pharmaceutical composition may comprise additional elements which may include but are not limited to blood flow improving agents such as cilostazol, ticlopidine hydrochloride, alprostadil, limaprost, beraprost sodium, sarpogrelate hydrochloride, argatroban, naftidrofuryl, isoxsuprine hydrochloride, batroxobin, dihydroergotoxine mesilate, tolazoline hydrochloride, hepronicate, and shimotsu-to extract.

In some cases, the pharmaceutical composition may comprise additional elements which may include but are not limited to bile acid derivatives, which are optionally farnesoid X receptor (FXR) ligand, such as ursodeoxycholic acid, chenodeoxycholic acid, obeticholic acid, aramchol, GW4064, bile powder, deoxycholic acid, cholic acid, bile extract, bear bile, oriental bezoar, and dehydrocholic acid. Preferable examples also include biotin (vitamin B7), cyanocobalamin (vitamin B12), pantothenic acid (vitamin B5), folic acid (vitamin B9), thiamine (vitamin B1), vitamin A, vitamin D, vitamin K, tyrosine, pyridoxine (vitamin B6), branched chain amino acids such as leucine, isoleucine, and valine, calcium, iron, zinc, copper, and magnesium. Other examples include components used in designated health foods and functional nutritional foods such as soy protein, chitosan, low molecular weight sodium alginate, dietary fiber from psyllium seed coat, soy peptide with bound phospholipids, phytosterol ester, plant stanol ester, diacylglycerol, globin digest, and tea catechin.

### D. Administration and Dosage

Compositions comprising EPAs useful for the disclosure include commercially available compositions of EPAs, such as Epadel®, Lovaza™, Omacor™, Lotriga™, Vascepa™, Epanova™ or Omtryg™ noted above or developing composition such as SC401B or MAT9001. Compositions comprising EPAs and/or may be administered in tablet, capsule, powder or any other solid oral dosage form, as a liquid, as a soft gel capsule or other capsule form, or other appropriate and convenient dosage forms for administration to a patient in need thereof. Compositions can also include pharmaceutically acceptable excipients known to those of ordinary skill in the art including surfactants, oils, co-solvents or combinations of such excipients, together with stabilizers, emulsifiers, preservatives, solubilizers and/or other non-active pharmaceutical ingredients known to those of skill in the art relative to the preparation of pharmaceutical compositions.

The dose and dosage period of the EPAs and/or estrogen used in the self-emulsifying composition of the present disclosure is a dose and period sufficient for realizing the intended action, which may be adequately adjusted depending on the administration route, frequency of administration per day, seriousness of the symptoms, body weight, age, and other factors.

The self-emulsifying composition of the present disclosure may be administered to the patient orally, endorectally, or transvaginally. However, oral administration is preferable in the case of the patient who can take the drug orally, and the composition may be administered in the form of a jelly preparation in the case of patients undergoing dialysis or patients with aphagia by jelling the composition with gelatin or the like. The estrogen can be administered orally, transdermally, intravenously, endorectally, transvaginally, or parenterally.

Doses of the aforementioned compositions as the active ingredient can be suitably decided depending on the purpose of administration, i.e., therapeutic or preventive treatment, nature of a disease to be treated or prevented, conditions, body weight, age, sexuality and the like of a patient. The practically desirable method and sequence for administration varies depending on the purpose of administration, i. e., therapeutic or preventive treatment, nature of a disease to be treated or prevented, conditions, body weight, age, sexuality and the like of a patient. The optimum method and sequence for administration of the compounds described in detail herein under preset given conditions may be suitably selected by those skilled in the art with the aid of the routine technique and the information contained in the present specification and field of invention.

In the case of oral administration, the composition may be administered at a dose in terms of the EPAs of 0.05 to 10g/day, 0.1 to 5 g/day, 0.2 to 4 g/day, 0.3 to 3g/day, 0.4 to 2g/day, 0.5 to 1g/day, preferably 0.2 to 4 g/day,0.3 to 3.6g/day, 0.6 to 2.7g/day, 0.9 to 1.8g/day, more preferably 0.3 to 3.0 g/day, 0.5 to 2.5g/day, 1.0 to 2.0g/day and most preferably 0.6 to 2.7 g/day, 0.9 to 1.8g/day in 1 to 3 divided doses. In the case of oral administration, the composition may be administered at a dose in terms of the EPAs of 0.1g/day, 0.3g/day, 0.5g g/day, 0.6 g/day, 0.9 g/day, 1.0g/day, 1.2 g/day, 1.5 g/day, 1.8 g/day, 2.0 g/day, 2.1 g/day, 2.4 g/day, 2.5 g/day, 2.7 g/day, 3.0 g/day, 3.3 g/day, 3.5 g/day, 3.6 g/day, 3.9 g/day, 4.0 g/day, 4.2 g/day, 4.5 g/day, 4.8 g/day, 5.0 g/day, 5.1 g/day, 5.4 g/day, 5.5 g/day, 5.7 g/day, 6.0 g/day, 6.3 g/day, 6.5 g/day, 6.6 g/day, 6.9 g/day, 7.0 g/day, 7.2 g/day, 7.5 g/day, 7.8 g/day, 8.0 g/day, 8.1 g/day, 8.4 g/day, 8.5 g/day, 8.7 g/day, 9.0 g/day, 9.3 g/day, 9.5 g/day, 9.6 g/day, 9.9 g/day and 10.0 g/day in 1 to 3 divided doses. However, the entire dose may be administered at once or in several divided doses. In some cases, the EPAs can be administered for 2.7 g per day or 1.8 g per day. While meal affects absorption of the EPAs, and the administration of the EPAs is preferably conducted during the meal or after the meal, and more preferably immediately after the meal (within 30 minutes after the meal), the self-emulsifying composition of the present disclosure has excellent absorption under fasting, and therefore, it exhibits the intended effects even when administered at a timing other than during, after, or immediately after the meal, for example, before or immediately before the meal or before going to the bed; to patients with reduced absorption ability of the intestinal tract (for example, elderly, patients of intestinal disease, patients after intestinal surgery, terminal cancer patients, or patients taking a lipase inhibitor); or used at a reduced dose.

The compositions of EPAs are administered according to the disclosure to a subject or patient to provide the patient with a dosage of about 0.3-10 g per day of EPAs, alternatively 0.5-8g per day, alternatively 0.6-6 g per day, alternatively 1-4g per day, alternatively 0.9-3.6 g per day or specifically about 1800 mg per day or about 2700 mg per day of EPAs.

The composition of estrogen are administered according to the disclosure to a subject or patient to provide the patient with an amount that is capable of elevating the serum level of estrogen to a level that is similar of that of a pre-menopausal fertile woman. In some cases, the estrogen can be administered orally. The composition comprising estrogen can be administered in a dosage that is about 0.625 mg/day. In some cases, the composition comprising estrogen can be administered in a dosage that is about, less than about, or more than about 0.1, 0.2, 0.25, 0.3, 0.4, 0.5, 0.6, 0.625, 0.7, 0.75, 0.8, 0.9, 1, 1.1, 1.25, 1.5, 1.625, 1.75 or 2 mg/day. In some cases, the estrogen can be administered orally about 0.45 to 2 mg per day. The oral tablet can be Premarine™ tablet.

In some cases, the composition comprising estrogen or estradiol can be administered parenterally. The dosage of estrogen for parenteral administration can be between about 1 to 10 mg for every 3 to 4 weeks or 5 to 20 mg every 4 weeks. In some cases, the dosage of estrogen or estradiol can be about, more than about, less than about 1, 2, 3, 4, 5, 6, 7, 8, 9, 10, 11, 12, 13, 14, 15, 16, 17, 18, 19, or 20 mg every 1, 2, 3, or 4 weeks. The intravenous or intramuscular injection can be Premarine™ for injection.

In some cases, the composition comprising estrogen can be administered transvaginally. The estrogen can be comprised and delivered by a vaginal ring. The dosage of estrogen can be about 0.05, 0.06, 0.07, 0.08, 0.09, 0.1, 0.15 or 0.2 mg/day. The ring can remain in place for 3 months and then be replaced by a new ring. The vaginal cream comprising estrogen can be administered in a dosage that is about 0.625 mg/day. In some cases, the composition comprising estrogen can be administered in a dosage that is about, less than about, or more than about 0.1, 0.2, 0.25, 0.3, 0.4, 0.5, 0.6, 0.625, 0.7, 0.75, 0.8, 0.9, 1, 1.1, 1.25, 1.5, 1.625, 1.75 or 2 mg/day. The vaginal cream can be Premarine™ vaginal cream.

The composition comprising estrogen can be administered topically or transdermally. The composition can be formulated in a form of transdermal film or transdermal emulsion. The estrogen can be in a dosage of between about 0.025 to 1 mg/day applied topically 1 or 2 times a week. The estrogen can be in a dosage of about, more than about, less than about 0.005, 0.01, 0.02, 0.03, 0.04, 0.05, 0.06, 0.07, 0.08, 0.09, 0.1, 0.11, 0.12, 0.13, 0.14, 0.15, 0.16, 0.17, 0.18, 0.19, 0.2, 0.5, 0.75, or 1 mg/day. The application sites of the transdermal film can vary according to manufacturer formulation and can include the lower abdomen, upper thigh, buttocks, or upper arm. One or more films can be applied daily. The transdermal film can be Estraderm™.

The composition comprising estrogen can be administered topically via a transdermal gel. The concentration of estrogen in the transdermal gel can be about 0.05%, 0.1%, 0.2%, 0.25%, 0.5% or 1%. The estrogen can be administered in an unit dose of 0.05, 0.1, 0.25, 0.5, 0.75, or 1 mg applied topically once daily. The gel can be applied to the upper right or left thigh at the same time or different time daily. The composition comprising estrogen can be administered by a spray. The dosage of estrogen for each spray can be about 0.1, 0.5, 1, 1.25, 1.5, 1.75, 2, 2.5, 3, 3.5, 4 g of the 0.01%, 0.02%, 0.03%, 0.04%, 0.05%, 0.06%, 0.07%, 0.08%, 0.09%, or 0.1% gel applied topically to the arms daily. The transdermal gel can be EstroGel™, Evamist™ or Elestrin™.

The duration of hormone replacement therapy or administration of estrogen can be about, more than about or less than about 1 month, 3 months, 6 months, 9 months, 1 year, 1.5 years, 2 years, 3 years, 4 years or 5 years.

The composition to be administered can contain other fatty acids, especially any omega-3 unsaturated fatty acid, especially DHAs. The ratio of EPAs/DHAs in the composition, the content of EPAs and DHAs in the total fatty acids and administration amount of EPAs and DHAs are not limited but the ratio is preferably 0.8 or more, more preferably 1.0 or more, still more preferably 1.2 or more. The composition is preferably highly purified; for example, the proportion of EPAs+DHAs in the fatty acids and their derivatives is preferably 40%, 45%, 50%, 55%, 60%, 65%, 70%, 75%, 80%, 85% by weight or more, more preferably 90% by weight or more, and still more preferably 96.5% by weight or more. The daily amount in terms of EPAs+DHAs is typically 0.3 to 10.0 g/day, preferably 0.5 to 6.0 g/day, and still more preferably 1.0 to 4.0 g/day, alternatively 0.9-3.6 g per day or specifically about 1800 mg per day or about 2700 mg per day of EPAs+DHAs. The low content of other long chain saturated fatty acids is preferred, and among the long chain unsaturated fatty acids, the content of omega-6 fatty acids, and in particular, the content of arachidonic acid in total of the fatty acids and their derivatives is preferably as low as less than 2%, 1%, 0.5%, 0.2%, 0.1% by weight, and more preferably less than 0.05% by weight. For example, soft capsule (Lovaza™, Omacor™ and Lotriga™) containing about 46% by weight of EPA-E and about 38% by weight of DHA-E is commercially available in the U.S. and other countries as a therapeutic agent for hypertriglyceridemia and soft capsule (Vascepa™) containing at least 96% by weight of EPA-E is commercially available in the U.S as a therapeutic agent for hypertriglyceridemia and soft capsule (Epanova™) containing about 50-60% by weight of EPA and about 15-25% by weight of DHA and SC401B or MTA9001 containing EPAs and DHAs are developed in the U.S. as a therapeutic agent for hypertriglyceridemia.

Patients treated for NASH can be administered EPAs according to the disclosure for 3, 6 or 9 months, or for 1 year, 2years, 3 years, 4 years, 5yeras or more and can be administered EPAs in one, two or three dosage per day, or other multiple doses per day including 1 to about 10, 1 to 8, 1 to 6, 1 to 4 or 1 to 2 dosage units per day as appropriate for patient therapy. The term "dose unit" and "dosage unit" herein refer to a portion of a pharmaceutical composition that contains an amount of EPAs and/or estrogen for a single administration to a subject.

When orally administered at such dose, the administration period may be adequately determined depending on the target disease and degree of symptoms. For example, in the case of administration for NASH, the administration period is not particularly limited as long as improvements of biochemical markers related to NASH, improvement in the pathological conditions or therapeutic effects, and suppression of the progress in metabolic syndrome, cardio or cerebrovascular event, or ulcer and gangrene of extremities and peripheries are realized. However, administration period is determined to realize the improvements in the concentration of plasma lipid marker (total cholesterol (hereinafter abbreviated as Cho), TG, postprandial TG, low-density lipoprotein Cho, high-density lipoprotein Cho, very-low-density lipoprotein Cho, non-high-density lipoprotein Cho, intermediate-density lipoprotein Cho, very-high-density lipoprotein Cho, free fatty acid, phospholipid, chylomicron, ApoB, lipoprotein (a), remnant-like lipoprotein Cho, small dense low-density lipoprotein Cho, etc.), increase in the skin temperature of extremities and peripheries which can be measured by thermography or the like, increase in the walking distance, increase in the serum CPK or other test value, and improvement of various symptoms such as numbness, coldness, ache, pain at rest, itching, cyanosis, flare, chilblain, neck stiffness, anemia, poor complexion, itching, and crawling. The amelioration or therapeutic effects may be monitored by other biochemical, pathological, or symptomatic parameters related to NASH. The administration is preferably continued as long as abnormality is observed in biochemical index such as serum lipid concentration or pathology. In addition, the composition may be administered every alternate day or 2 or 3 days in a week, or as the case may be, a drug withdrawal period of about 1 day to 3 month, and more preferably about 1 week to 1 month may be included.

If indicated by the physician, oral administration may be started at a dose lower than the recommended daily EPAs dose at the first day, and then, the dose may be gradually increased to the maximum daily dose as the maintenance dose. The dose may be reduced depending on the conditions of the patient. Lower daily dose is preferable in view of reducing the side effects, and administration of once or twice a day is preferable in view of the drug compliance.

The method of the present invention may administer a therapeutically effective amount of a pharmaceutical composition comprising EPAs in combination with a second effective component. The second effective component may be adequately determined depending on the target disease and the seriousness of the symptom. However, the second effective component is preferably a component that does not adversely affect the effects of EPAs, and examples include therapeutic agent for hyperlipidemia, antihypertensives, antidiabetics, antioxidants, blood flow improving agents, farnesoid X receptor (FXR) ligands and bile acid derivatives. The more preferable second effective component are agent for hyperlipidemia, antihypertensives, antidiabetics and farnesoid X receptor (FXR) ligands.

Of the preferable examples of the second effective component, exemplary therapeutic agents for hyperlipiciemia include polyenephosphatidylcholine, unsaponifiable soybean oil (soy sterol), gamma-oryzanol, riboflavin butyrate, dextran sulfate sodium sulfur 18, pantethine, and elastase; statins such as lovastatin, pravastatin, simvastatin, atorvastatin, fluvastatin, pitavastatin, rosuvastatin, and cerivastatin; fibrates such as simfibrate, clofibrate, clinofibrate, bezafibrate, and fenofibrate; lipolytic enzyme inhibitors such as orlistat and cetilistat; resins such as colestyramine and colestimide; and ezetimibe. The preferable agents for hyperlipidemia are statins such as lovastatin, pravastatin, simvastatin, atorvastatin, fluvastatin, pitavastatin, rosuvastatin, and cerivastatin; fibrates such as simfibrate, clofibrate, clinofibrate, bezafibrate and ezetimibe. The more preferable agents for hyperlipidemia are statins such as pravastatin, simvastatin, atorvastatin, fluvastatin, pitavastatin, rosuvastatin, and cerivastatin.

Exemplary antihypertensives include angiotensin II receptor blockers such as irbesartan, olmesartan medoxomil, candesartan cilexetil, telmisartan, valsartan, and losartan potassium; angiotensin-converting enzyme inhibitors such as alacepril, imidapril hydrochloride, enalapril maleate, captopril, quinapril hydrochloride, cilazapril hydrate, temocapril hydrochloride, delapril hydrochloride, trandolapril, benazepril hydrochloride, perindopril, and lisinopril hydrate; calcium antagonists such as azelnidipine, amlodipine besylate, aranidipine, efonidipine hydrochloride, cilnidipine, nicardipine hydrochloride, nifedipine, nimodipine, nitrendipine, nilvadipine, barnidipine hydrochloride, felodipine, benidipine, and manidipine; [alpha] receptor blocker such as tolazoline, and phentolamine; [beta] receptor blockers such as atenolol, metoprolol, acebutolol, propranolol, pindolol, carvedilol, and labetalol hydrochloride; a receptors stimulant such as clonidine and methyldopa; and diuretics such as eplerenone, hydrochlorothiazide, and furosemide. The preferable antihypertensives are angiotensin II receptor blockers such as irbesartan, olmesartan medoxomil, candesartan cilexetil, telmisartan, valsartan, and losartan potassium, angiotensin-converting enzyme inhibitors such as alacepril, imidapril hydrochloride, enalapril maleate, captopril, quinapril hydrochloride, cilazapril hydrate, temocapril hydrochloride, delapril hydrochloride, trandolapril, benazepril hydrochloride, perindopril, and lisinopril hydrate and calcium antagonists such as azelnidipine, amlodipine besylate, cilnidipine, nicardipine hydrochloride, nifedipine and manidipine. The more preferable antihypertensives are angiotensin II receptor blockers such as irbesartan, olmesartan medoxomil, candesartan cilexetil, telmisartan, valsartan, and losartan potassium, angiotensin-converting enzyme inhibitors such as imidapril hydrochloride, enalapril maleate, captopril, and lisinopril hydrate and calcium antagonists such as azelnidipine, amlodipine besylate, and cilnidipine.

Exemplary antidiabetics include [alpha]-glucosidase inhibitors such as acarbose, voglibose, and miglitol; sulfonyl urea hypoglycemics such as gliclazide, glibenclamide, glimepiride, and tolbutamide; fast-acting insulin secretagogues such as nateglinide and mitiglinide; biguanide hypoglycemics such as metformin hydrochloride and buformin hydrochloride; dipeptidyl phosphatase 4 inhibitors such as sitagliptin, vildagliptin, alogliptin, linagliptin and saxagliptin; thiazolidine reagents such as pioglitazone hydrochloride and rosiglitazone maleate; and glucagon-like peptide 1 derivative reagents such as exenatide, lixisenatide and liraglutide. The preferable antidiabetics are [alpha]-glucosidase inhibitors such as acarbose and voglibose, sulfonyl urea hypoglycemics such as glibenclamide, glimepiride and tolbutamide, fast-acting insulin secretagogues such as nateglinide and mitiglinide, biguanide hypoglycemics such as metformin hydrochloride and buformin hydrochloride, dipeptidyl phosphatase 4 inhibitors such as sitagliptin, vildagliptin, alogliptin, linagliptin and saxagliptin, thiazolidine reagents such as pioglitazone hydrochloride and rosiglitazone maleate and glucagon-like peptide 1 derivative reagents such as exenatide, lixisenatide and liraglutide. The more preferable antidiabetics are sulfonyl urea hypoglycemics such as glibenclamide, glimepiride and tolbutamide, biguanide hypoglycemics such as metformin hydrochloride and buformin hydrochloride, dipeptidyl phosphatase 4 inhibitors such as sitagliptin, vildagliptin, alogliptin, linagliptin and saxagliptin, thiazolidine reagents such as pioglitazone hydrochloride and rosiglitazone maleate and glucagon-like peptide 1 derivative reagents such as exenatide, lixisenatide and liraglutide. The most preferable antidiabetics are dipeptidyl phosphatase 4 inhibitors such as sitagliptin, vildagliptin, alogliptin, linagliptin and saxagliptin and thiazolidine reagents such as pioglitazone hydrochloride and rosiglitazone maleate.

The dose of the second effective component is not particularly limited since such dose depends on the conditions and body type of the individual patient. However, exemplary such daily dose of the second effective component is equal or less than the recommended daily dose of mono-therapy. In the case of lovastatin is less than the recommended daily dose of 10 mg/day, preferably at least 0.2 mg and up to 8 mg, more preferably at least 0.4 mg and up to 6 mg, and still more preferably at least 1 mg and up to 4 mg; in the case of pravastatin sodium salt, less than the recommended daily dose of 40 mg/day, preferably at least 1 mg and up to 30 mg, more preferably at least 2 mg and up to 25 mg, and still more preferably at least 4 mg and up to 20 mg; in the case of simvastatin, less than the recommended daily dose of 1 day 5 mg, preferably at least 0.1 mg and up to 4 mg, more preferably at least 0.2 mg and up to 2 mg, and still more preferably at least 0.4 mg and up to 1 mg; in the case of atorvastatin calcium hydrate, less than the recommended daily dose of 20 mg/day, preferably at least 0.4 mg and up to 16 mg, more preferably at least 0.8 mg and up to 12 mg, and still more preferably at least 2 mg and up to 10 mg; in the case of fluvastatin sodium salt, less than the recommended daily dose of 20 mg/day, preferably at least 0.4 mg and up to 16 mg, more preferably at least 0.8 mg and up to 12 mg, and still more preferably at least 1.5 mg and up to 8 mg; in the case of pitavastatin calcium salt, less than the recommended daily dose of 1 day 1 mg, preferably at least 0.02 mg and up to 0.8 mg, more preferably at least 0.04 mg and up to 0.6 mg, and still more preferably at least 0.1 mg and up to 0.4 mg; in the case of rosuvastatin calcium salt, less than the recommended daily dose of 2.5 mg, preferably at least 0.05 mg and up to 2 mg, more preferably at least 0.1 mg and up to 1.5 mg, and still more preferably at least 0.2 mg and up to 1 mg and in the case of bezafibrate, less than the recommended daily dose of 800 mg, preferably at least 50 mg and up to 600 mg, more preferably at least 100 mg and up to 500 mg, and still more preferably at least 200 mg and up to 400 mg.

In the case of irbesartan, less than the recommended daily dose of 50 mg/day, preferably at least 1 mg and up to 40 mg, more preferably at least 2 mg and up to 30 mg, and still more preferably at least 5 mg and up to 20 mg; in the case of olmesartan medoxomil, less than the recommended daily dose of 10 mg/day, preferably at least 0.2 mg and up to 8 mg, more preferably at least 0.5 mg and up to 6 mg, and still more preferably at least 1 mg and up to 4 mg; in the case of candesartan cilexetil, less than the recommended daily dose of 4 mg/day, preferably at least 0.1 mg and up to 3 mg, more preferably at least 0.2 mg and up to 2 mg, and still more preferably at least 0.4 mg and up to 1 mg; in the case of telmisartan, less than the recommended daily dose of 20 mg/day, preferably at least 0.5 mg and up to 15 mg, more preferably at least 1 mg and up to 10 mg, and still more preferably at least 2 mg and up to 5 mg; in the case of valsartan, less than the recommended daily dose of 40 mg/day, preferably at least 1 mg and up to 30 mg, more preferably at least 2 mg and up to 20 mg, and still more preferably at least 4 mg and up to 10 mg; and in the case of losartan potassium salt is less than the recommended daily dose of 50 mg/day, preferably at least 1 mg and up to 40 mg, more preferably at least 2 mg and up to 30 mg, and still more preferably at least 4 mg and up to 20 mg.

In the case of pioglitazone hydrochloride, the daily dose is equal or less than the recommended daily dose of 60 mg/day, preferably at least 5 mg and up to 50 mg, more preferably at least 10 mg and up to 40 mg, and still more preferably at least 20 mg and up to 30 mg; in the case of rosiglitazone maleate, the daily dose is equal or less than the recommended daily dose of 16 mg/day, preferably at least 1 mg and up to 12 mg, more preferably at least 2 mg and up to 10 mg, and still more preferably at least 4 mg and up to 8 mg; in the case of nateglinide, less than the recommended daily dose of 500 mg/day, preferably at least 10 mg and up to 400 mg, more preferably at least 20 mg and up to 350 mg, and still more preferably at least 50 mg and up to 300 mg; in the case of metformin hydrochloride, the daily dose is equal or less than the recommended daily dose of 2000 mg/day, preferably at least 40 mg and up to 1500 mg, more preferably at least 80 mg and up to 1200 mg, and still more preferably at least 200 mg and up to 1000 mg; and in the case of buformin hydrochloride, less than the recommended daily dose of 400 mg/day, preferably at least 10 mg and up to 300 mg, more preferably at least 20 mg and up to 250 mg, and still more preferably at least 50 mg and up to 200 mg.

Exemplary antioxidants include vitamins such as ascorbic acid (vitamin C), tocopherol (vitamin E), and tocopherol nicotinate, and N-acetylcysteine, probucol.

Exemplary blood flow improving agents include cilostazol, ticlopidine hydrochloride, alprostadil, limaprost, beraprost sodium, sarpogrelate hydrochloride, argatroban, naftidrofuryl, isoxsuprine hydrochloride, batroxobin, dihydroergotoxine mesilate, tolazoline hydrochloride, hepronicate, and shimotsu-to extract.

Exemplary bile acid derivatives include ursodeoxycholic acid, chenodeoxycholic acid, bile powder, deoxycholic acid, cholic acid, bile extract, bear bile, oriental bezoar, and dehydrocholic acid. Preferable examples also include biotin (vitamin B7), cyanocobalamin (vitamin B12), pantothenic acid (vitamin B5), folic acid (vitamin B9), thiamine (vitamin B1), vitamin A, vitamin D, vitamin K, tyrosine, pyridoxine (vitamin B6), branched chain amino acids such as leucine, isoleucine, and valine, calcium, iron, zinc, copper, and magnesium. Other examples include components used in designated health foods and functional nutritional foods such as soy protein, chitosan, low molecular weight sodium alginate, dietary fiber from psyllium seed coat, soy peptide with bound phospholipids, phytosterol ester, plant stanol ester, diacylglycerol, globin digest, and tea catechin. Exemplary antioxidants include vitamins such as ascorbic acid (vitamin C), tocopherol (vitamin E), and tocopherol nicotinate, and N-acetylcysteine, probucol. The preferable antioxidant is tocopherol (vitamin E).

Exemplary blood flow improving agents include cilostazol, ticlopidine hydrochloride, clopidogrel, prasugrel, edoxaban tosilate hydrate, rivaroxaban, dabigatran etexilatealprostadil, limaprost, beraprost sodium, sarpogrelate hydrochloride, argatroban, naftidrofuryl, isoxsuprine hydrochloride, batroxobin, dihydroergotoxine mesilate, tolazoline hydrochloride, hepronicate, and shimotsu-to extract. The preferable blood flow improving agents are clopidogrel, prasugrel, edoxaban tosilate hydrate, rivaroxaban and dabigatran etexilate.

Exemplary bile acid derivatives, which are optionally FXR ligand, include ursodeoxycholic acid, chenodeoxycholic acid, obeticholic acid, aramchol, GW4064, bile powder, deoxycholic acid, cholic acid, bile extract, bear bile, oriental bezoar, and dehydrocholic acid. The preferable bile acid derivatives are ursodeoxycholic acid, chenodeoxycholic acid, obeticholic acid, aramchol, GW4064, bile powder, deoxycholic acid, and cholic acid. The more preferable bile acid derivatives are FXR ligand such as chenodeoxycholic acid, obeticholic acid, aramchol and GW4064. Preferable examples also include biotin (vitamin B7), cyanocobalamin (vitamin B12), pantothenic acid (vitamin B5), folic acid (vitamin B9), thiamine (vitamin B1), vitamin A, vitamin D, vitamin K, tyrosine, pyridoxine (vitamin B6), branched chain amino acids such as leucine, isoleucine, and valine, calcium, iron, zinc, copper, and magnesium. Other examples include components used in designated health foods and functional nutritional foods such as soy protein, chitosan, low molecular weight sodium alginate, dietary fiber from psyllium seed coat, soy peptide with bound phospholipids, phytosterol ester, plant stanol ester, diacylglycerol, globin digest, and tea catechin. In the case of tocopherol (vitamin E), the daily dose is equal or less than 1600 IU/day, preferably at least 100 IU and up to 1200 IU, more preferably at least 200 IU and up to 1000 IU, and still more preferably at least 400 IU and up to 800 IU or the daily dose is equal or less than 600 mg/day, preferably at least 50mg and up to 500mg, more preferably at least 100 mg and up to 400 mg, and still more preferably at least 200mg and up to 300 mg and in the case of ascorbic acid (vitamin C) or vitamin Bs , the daily dose is equal or less than 2000 mg/day, preferably at least 40 mg and up to 1500 mg, more preferably at least 80 mg and up to 1200 mg, and still more preferably at least 200 mg and up to 1000 mg . In the case of ursodeoxycholic acid, the daily dose is equal or less than 30 mg/day, preferably at least 1 mg and up to 25 mg, more preferably at least 2 mg and up to 20 mg, and still more preferably at least 5 mg and up to 15 mg, in the case of obeticholic acid , the daily dose is equal or less than 50 mg/day, preferably at least 1 mg and up to 40 mg, more preferably at least 2 mg and up to 30 mg, and still more preferably at least 5 mg and up to 25 mg, and in the case of aramchol , the daily dose is equal or less than 500 mg/day, preferably at least 10 mg and up to 400 mg, more preferably at least 20 mg and up to 300 mg, and still more preferably at least 50 mg and up to 250 mg.

The preferable combinations are comprising at least EPAs and lovastatin, EPAs and pravastatin, EPAs and simvastatin, EPAs and atorvastatin, EPAs and fluvastatin, EPAs and pitavastatin, EPAs and rosuvastatin, EPAs and cerivastatin, EPAs and simfibrate, EPAs and clofibrate, EPAs and clinofibrate, EPAs and bezafibrate, EPAs and ezetimibe, EPAs and irbesartan, EPAs and olmesartan medoxomil, EPAs and candesartan cilexetil, EPAs and telmisartan, EPAs and valsartan, EPAs and losartan potassium, EPAs and alacepril, EPAs and imidapril hydrochloride, EPAs and enalapril maleate, EPAs and captopril, EPAs and quinapril hydrochloride, EPAs and cilazapril hydrate, EPAs and temocapril hydrochloride, EPAs and delapril hydrochloride, EPAs and trandolapril, EPAs and benazepril hydrochloride, EPAs and perindopril, EPAs and lisinopril hydrate, EPAs and azelnidipine, EPAs and amlodipine besylate, EPAs and cilnidipine, EPAs and nicardipine hydrochloride, EPAs and nifedipine, EPAs and manidipine, EPAs and acarbose, EPAs and voglibose, EPAs and glibenclamide, EPAs and glimepiride, EPAs and tolbutamide, EPAs and nateglinide, EPAs and mitiglinide, EPAs and metformin hydrochloride, EPAs and buformin hydrochloride, EPAs and sitagliptin, EPAs and vildagliptin, EPAs and alogliptin, EPAs and linagliptin, EPAs and saxagliptin, EPAs and pioglitazone hydrochloride, EPAs and rosiglitazone maleate, EPAs and exenatide, EPAs and lixisenatide and EPAs and liraglutide, EPAs and tocopherol (vitamin E), EPAs and clopidogrel, EPAs and prasugrel, EPAs and edoxaban tosilate hydrate, EPAs and rivaroxaban, EPAs and dabigatran etexilate, EPAs and ursodeoxycholic acid, EPAs and chenodeoxycholic acid, EPAs and obeticholic acid, EPAs and aramchol, EPAs and GW4064, EPAs and bile powder, EPAs and deoxycholic acid, and EPAs and cholic acid. The more preferable combinations are comprising at least EPAs and pravastatin, EPAs and simvastatin, EPAs and atorvastatin, EPAs and fluvastatin, EPAs and pitavastatin, EPAs and rosuvastatin, EPAs and ezetimibe, EPAs and irbesartan, EPAs and olmesartan medoxomil, EPAs and candesartan cilexetil, EPAs and telmisartan, EPAs and valsartan, EPAs and losartan potassium, EPAs and sitagliptin, EPAs and vildagliptin, EPAs and alogliptin, EPAs and linagliptin, EPAs and saxagliptin, EPAs and pioglitazone hydrochloride, EPAs and rosiglitazone maleate, EPAs and exenatide, EPAs and lixisenatide and EPAs and liraglutide, EPAs and tocopherol (vitamin E), EPAs and chenodeoxycholic acid, EPAs and obeticholic acid, EPAs and aramchol, EPAs and GW4064. The most preferable combinations are comprising at least EPAs and atorvastatin, EPAs and fluvastatin, EPAs and pitavastatin, EPAs and rosuvastatin, EPAs and irbesartan, EPAs and olmesartan medoxomil, EPAs and candesartan cilexetil, EPAs and telmisartan, EPAs and valsartan, EPAs and losartan potassium, EPAs and sitagliptin, EPAs and vildagliptin, EPAs and alogliptin, EPAs and linagliptin, EPAs and saxagliptin, EPAs and pioglitazone hydrochloride, EPAs and rosiglitazone maleate, EPAs and tocopherol (vitamin E), EPAs and chenodeoxycholic acid, EPAs and obeticholic acid. The preferable combinations are EPAs, at least one member selected from the group consisting of above mentioned agents for hyperlipidemia and at least one member selected from the group consisting of above mentioned antihypertensives. The preferable combinations are EPAs, at least one member selected from the group consisting of above mentioned agents for hyperlipidemia and at least one member selected from the group consisting of above mentioned anti-diabetic agents. The preferable combinations are EPAs, at least one member selected from the group consisting of above mentioned agents for hyperlipidemia and tocopherol (vitamin E). The preferable combinations are EPAs, at least one member selected from the group consisting of above mentioned agents for hyperlipidemia and at least one member selected from the group consisting of blood flow improving agents. The preferable combinations are EPAs, at least one member selected from the group consisting of above mentioned agents for hyperlipidemia and at least one member selected from the group consisting of above mentioned bile acid derivatives (including FXR ligands).

The preferable combinations are EPAs, at least one member selected from the group consisting of above mentioned antihypertensives and at least one member selected from the group consisting of above mentioned anti-diabetic agents. The preferable combinations are EPAs, at least one member selected from the group consisting of above mentioned antihypertensives and tocopherol (vitamin E). The preferable combinations are EPAs, at least one member selected from the group consisting of above mentioned antihypertensives and at least one member selected from the group consisting of blood flow improving agents. The preferable combinations are EPAs, at least one member selected from the group consisting of above mentioned antihypertensives and at least one member selected from the group consisting of above mentioned bile acid derivatives (including FXR ligands).

The preferable combinations are EPAs, at least one member selected from the group consisting of above mentioned anti-diabetic agents and tocopherol (vitamin E). The preferable combinations are EPAs, at least one member selected from the group consisting of above mentioned anti-diabetic agents and at least one member selected from the group consisting of blood flow improving agents. The preferable combinations are EPAs, at least one member selected from the group consisting of above mentioned anti-diabetic agents and at least one member selected from the group consisting of above mentioned bile acid derivatives (including FXR ligands). The preferable combinations are EPAs, tocopherol (vitamin E) and at least one member selected from the group consisting of blood flow improving agents. The preferable combinations are EPAs, tocopherol (vitamin E) and at least one member selected from the group consisting of above mentioned bile acid derivatives (including FXR ligands). The preferable combinations are EPAs, at least one member selected from the group consisting of blood flow improving agents and at least one member selected from the group consisting of above mentioned bile acid derivatives (including FXR ligands).

The preferable combinations are EPAs, at least one member selected from the group consisting of above mentioned agents for hyperlipidemia, at least one member selected from the group consisting of above mentioned antihypertensives, at least one member selected from the group consisting of above mentioned anti-diabetic agents and tocopherol (vitamin E). The preferable combinations are EPAs, at least one member selected from the group consisting of above mentioned agents for hyperlipidemia, at least one member selected from the group consisting of above mentioned antihypertensives, at least one member selected from the group consisting of above mentioned anti-diabetic agents and at least one member selected from the group consisting of above mentioned bile acid derivatives (including FXR ligands). The preferable combinations are EPAs, at least one member selected from the group consisting of above mentioned agents for hyperlipidemia, at least one member selected from the group consisting of above mentioned antihypertensives, tocopherol (vitamin E) and at least one member selected from the group consisting of above mentioned bile acid derivatives (including FXR ligands). The preferable combinations are EPAs, at least one member selected from the group consisting of above mentioned agents for hyperlipidemia, at least one member selected from the group consisting of above mentioned anti-diabetic agents, tocopherol (vitamin E) and at least one member selected from the group consisting of above mentioned bile acid derivatives (including FXR ligands). The preferable combinations are EPAs, at least one member selected from the group consisting of above mentioned agents for hyperlipidemia, at least one member selected from the group consisting of above mentioned anti-diabetic agents, tocopherol (vitamin E) and at least one member selected from the group consisting of above mentioned bile acid derivatives (including FXR ligands). The preferable combinations are EPAs, at least one member selected from the group consisting of above mentioned antihypertensives, at least one member selected from the group consisting of above mentioned anti-diabetic agents, tocopherol (vitamin E) and at least one member selected from the group consisting of above mentioned bile acid derivatives (including FXR ligands).

The preferable combinations are EPAs, at least one member selected from the group consisting of above mentioned agents for hyperlipidemia, at least one member selected from the group consisting of above mentioned antihypertensives, at least one member selected from the group consisting of above mentioned anti-diabetic agents, tocopherol (vitamin E) and at least one member selected from the group consisting of above mentioned bile acid derivatives (including FXR ligands).

The combination use of EPAs (first ingredient) and the second effective component such as agent for hyperlipidemia, antihypertensives, antidiabetics and farnesoid X receptor (FXR) ligands realizes safety and remarkable effectiveness of the level which are not observed by single administration of the corresponding agents. The combination use of EPAs (first ingredient) and the second effective component is expected to exhibit synergetic prophylactic/ameliorative or therapeutic effects for the NASH. The combination use of EPAs (first ingredient) and the second effective component is capable of reducing dose of agents and side effects of the agents. So it can also be administered to the patients who could not receive the treatment or the patients who had to stop the treatment because of the side effects of the second effective components.

### E. Treatment Effects

The methods and self-emulsifying composition of the present disclosure can be used as a therapeutic agent for various diseases of animals, and in particular, mammals, for example, therapeutic agent for NASH and related disorders. The methods and self-emulsifying composition of the present disclosure is particularly expected to exhibit amelioration or therapeutic effects NASH subjects without hepatocyte apoptosis, or in the early stages of this indication. NASH subjects may improve or maintain a variety of symptoms, which may include but are not limited, increase in blood lipid, expression of insulin resistance, increase in blood pressure, abnormal liver function tests, abnormal liver enzyme activity, elevated glucose levels, liver dysfunction, hyperlipidemia, or any abnormal criteria. In some cases, the method and pharmaceutical composition may aid in maintaining a function, level or activity present in a subject.

In some cases, the method and composition as described herein method and may lower sFas, M30 or NASH risk score levels lower than the thresholds as provided by the disclosure. In some cases, the composition may lower sFas, M30 or NASH risk score levels as determined to be above normal, as would be found in NASH subjects having hepatocyte apoptosis or be at risk for the indication. In some cases, the method and composition may lower sFas, M30 or NASH risk score levels in subjects not considered to have hepatocyte apoptosis. In some cases, the methods and pharmaceutical composition may aid in maintaining a sFas, M30 or NASH risk score levels present in a subject. Further, in some cases a patient's EPA/AA ratio as compared to a baseline EPA/AA ratio may improve by equal to or greater than 0.1, 0.2, 0.3 or 0.4.

In some cases, the methods and compositions as described herein can improve the NAS score in the subject administered. The NAS score can be improved by at least about 30%. In some cases, the NAS score can be improved by at least about 10%, 15%, 20%, 25%, 30%, 35%, 40$, 45%, 50%, 55%, 60%, 65%, 70%, 75%, 80% or 85%. In some cases, the methods and compositions as described herein can improve the steatosis score in the subject administered. %. In some cases, the steatosis score can be improved by at least about 10%, 15%, 20%, 25%, 30%, 35%, 40$, 45%, 50%, 55%, 60%, 65%, 70%, 75%, 80% or 85%.

The method and self-emulsifying composition of the present disclosure can reduce burden of the patients by reducing the dose and daily frequency of the administration, and hence, by improving the drug compliance. This also results in the higher effects of amelioration or treatment.

### V. Examples

### Example 1: Pre-clinical Experience

In animal and *in vitro* model studies, EPA-E (ethyl all-*cis*-5,8,11,14,17-eicosapentaenoate) has been shown to lower lipids in rats, hamsters and rabbits; have anti-aggregation effects on platelets from rats, rabbits and humans; and to preserve the elasticity of arteries in rabbits. In other studies, polyunsaturated fatty acids (PUFAs) have been shown to ameliorate hepatic steatosis in *ob*/*ob* mice through down-regulation of hepatic nuclear sterol regulatory element binding protein-lc (SREBP-1c). In a similar manner, EPA-E following repeat oral administration at ≥0.1 mg/g suppressed fat accumulation in a mouse diet-induced hepatic steatosis model by suppressing hepatic SREBP-1c levels as well as monounsaturated fatty acid (MUFA) synthesis by stearoyl-Coenzyme A desaturase 1 (SCD1). In a galactosamine-induced steatohepatitis mouse model, EPA-E after oral administration at 1000 mg/kg retarded progression of steatohepatitis by suppressing triglyceride (TG) accumulation. EPA-E following repeat oral administration at 1000 mg/kg inhibited fibrosis by suppressing inflammation and oxidative stress in a methionine-choline deficient diet rat model of nonalcoholic steatohepatitis.

In safety pharmacology studies, at oral doses up to 3000 mg/kg, EPA-E had no effect on the central nervous, autonomic nervous, respiratory and cardiovascular systems except for a reduction in gastric fluid levels in pylorus-ligated rats after a 3000 mg/kg oral dose. The effects of the metabolites and impurities of EPA-E as well as oxidized EPA-E on the above systems were not marked and do not appear to significantly contribute to general pharmacological effects of EPA-E.

### Pharmacokinetics and Product Metabolism in Animals Summary

EPADEL® is a product containing ethyl all-*cis*-5,8,11,14,17-eicosapentaenoate (EPA-E), one of the n-3 essential fatty acids. The preclinical absorption, distribution, metabolism and excretion (ADME) characteristics of EPA-E have been determined primarily in rats.

Oral administration of a single dose of radioactive ¹⁴C-EPA-E at the dose of 30-1000 mg/kg in rats showed that EPA-E was well-absorbed, mainly through the lymphatic route. After administration of ¹⁴C-EPA-E into the ligated intestine of rats, the residual radioactivity in the intestine at 24 hours was only 4.6% of the administered dose. Radioactivity transferred to the plasma and lymph was mainly detected in the triglyceride (TG) fraction in the early phase after dosing, while distribution to the free fatty acid (FFA) fraction was slight at all timepoints. The radioactivity was widely distributed in the body tissues; relatively high levels were observed especially in the brown fat, adrenal, liver and pancreas. Within the tissues, the radioactivity was mainly distributed in the TG and/or phospholipid (PL) fractions. ¹⁴C-EPA-E was hydrolyzed rapidly in the small intestine homogenates, lymph, plasma and liver homogenates of the rat. Orally administered EPA-E was primarily incorporated in adrenal gland lipids as cholesterol esters of the fatty acids eicosapentaenoic acid (EPA), docosapentaenoic acid (DPA) and docosahexaenoic acid (DHA).

The uptake of ¹⁴C-EPA-E-derived radioactivity in rats described above is compatible with the absorption process of essential fatty acids in animals and humans described below. In addition, esterases are also distributed in most of the organs in humans). Therefore, the absorption and distribution profiles after oral dosing of EPA-E should be qualitatively similar in humans and dogs as observed in rats.

The digestion and absorption of essential fatty acids, mainly in the form of TG, are known to involve several processes.

The fatty acids are rapidly hydrolyzed from the TG to FFA by lipase in the intestine.

The FFA are taken up by the enterocytes where they are re-esterified into TG and enter the blood circulation, mainly through the lymphatic route as chylomicrons.

In the tissues, the TG of the chylomicrons is hydrolyzed again by lipoprotein lipase to FFA and taken up by the tissues.

Consequently, when EPA-E is administered orally to humans, it would appear that it is well absorbed, even though the unchanged ethyl ester form is not detected, and the free form (EPA) is only detected at a very low level, in the blood. Due to this absorption mechanism of essential fatty acids, the administered EPA-E exists as a blood-constituent fatty acid in the total lipids. In fact, according to the approval package document of Lovaza®, the free form of EPA is undetectable in the circulation (<1 µM) following an oral dose of 4 g of Omacor® (a mixture of the ethyl esters of EPA and DHA) Following oral administration of EPA-E, EPA, DPA and DHA were isolated and identified as metabolites in tissues and plasma. EPA, DPA and DHA were incorporated into TG and PL. Radioactivity was primarily excreted in expired air (44%) after single oral administration at 30 mg/kg EPA-E with minimal excretion in bile and urine (∼3%) and approximately 20% excreted in feces. Thus, the respiratory route (as ¹⁴CO₂) was considered to be the major elimination pathway of ¹⁴C-EPA-E. Excretion of radioactivity in dogs after single oral administration at 30 mg/kg was low with 1.0% excreted in the urine and 19.2% recovered in the feces after 1 week.

Renal excretion was the elimination route for several minor highly polar metabolites (<0.4%), but EPA-E, EPA, DPA or DHA were not detected in the urine. In feces, EPA-E and EPA, evidently derived from the unabsorbed drug, were detected; however, no DPA or DHA was detected in the feces.

In metabolism studies conducted *in vitro,* when ¹⁴C-EPA-CoA was incubated with the rat liver mitochondrial fraction, 17.1% of the radioactivity added to the incubation mixture was detected as ¹⁴CO2 and the formation of carbon chain-shortened products was observed. Incubation of ¹⁴CEPA-CoA with a rat peroxisome fraction also resulted in the formation of carbon chain-shortened products. These results show that EPA, after being taken up by the rat tissues, is finally almost entirely oxidized to CO₂ by mitochondrial and peroxisomal β-oxidation.

After oral administration of ¹⁴C-EPA-E to the rat, EPA, DPA and DHA were detected as metabolites in the total lipid fraction of the plasma and tissues (liver, fat, heart and brain). However, no unchanged EPA-E was found in the plasma or any of the tissues. In the total lipid fraction of the liver, the radioactivity originated mainly from EPA, DPA and DHA. Thus, it is considered that EPA, DPA and DHA are the predominant metabolites of ¹⁴C-EPA-E in tissues, as constituent fatty acids of the total lipid fraction. When ¹⁴C-EPA•K was incubated with the microsomal fraction, formation of DPA and DHA was detected. These results show that EPA taken up by the rat tissues is elongated to DPA and DHA in the microsomes.

Plasma protein binding in rats and dogs was >86% and >96%, respectively. Previous studies have shown that EPA is unlikely to inhibit CYP450 at free concentrations observed in humans.

### Example 2: Clinical Trial Data

### Study Design

This example provides the protocol used for an ongoing phase II clinical trial, double blind, placebo-controlled study to investigate the safety, efficacy, and pharmacokinetic profile of two doses of EPA-E in subjects with NASH. Up to 70 subjects were enrolled into each treatment arm, for a total of 210 subjects to be enrolled. Block randomization using an interactive voice response system (IVRS) was used to assign patients in a 1:1:1 ratio to two active doses and placebo. Patients were stratified at randomization by presence or absence of diabetes. Patients with diabetes comprised no more than 25% of the total number of patients enrolled. Subjects were treated with 600 mg EPA-E, 900 mg EPA-E or placebo three times a day for one year.
Study arm 1: 600 mg EPA-E (3 capsules), TID
Study arm 2: 900 mg EPA-E (3 capsules), TID
Study arm 3: placebo (3 capsules), TID

Subjects were required to have a liver biopsy with proven NASH in the 6 month period prior to screening. The Pharmacokinetic profile for EPA-E was evaluated in a subgroup of subjects in specified sites. Subjects were approached prior to providing informed consent to determine if they will participate in the PK group subset. Approximately 36 subjects participated in the PK subset evaluation (12 from each treatment arm, to include 6 males and 6 females).

Overall study duration plan was 2 years.

### The investigational drugs

In order to support the trial, EPA-E capsules and matching placebo capsules were prepared. EPA-E capsule is an oval soft gelatin capsule containing 300 mg of EPA-E as an active ingredient. Placebo capsule is an oval soft gelatin capsule containing olive oil as an inactive ingredient. These capsules are unidentifiable as EPA-E capsules or placebo capsules.

Subjects were administered orally 3 capsules 3 times daily, immediately after meals.

### Eligibility Criteria

Subjects with a histological diagnosis of NASH are eligible.

### Inclusion Criteria

Inclusion criteria were designed to ensure that subjects with biopsy proven NASH are included, and to avoid situations where potential harm may occur to subjects in conjunction with participation in the study.

Subjects were potentially included into the study if they met all the following criteria:
1. Diagnosis of definite NASH by the central reading pathologists:
   Liver biopsy slides will be submitted for evaluation by the central pathologists according to one of the following criteria:
   a. Previous liver biopsies must have been obtained within 6 months prior to informed consent and should be judged by the local pathologist as showing NAS ≥4 with a minimum score of 1 each for steatosis and lobular inflammation plus EITHER ballooning OR at least 1a sinusoidal fibrosis AND a finding of possible or definite steatohepatitis
   b. For liver biopsies performed after informed consent is obtained, all slides will be submitted for reading by the central pathologists in conformity with the independent pathology review charter (IPRC)
2. Patients of either gender greater than 18 years of age
3. Patients with diabetes that have been on stable doses of anti-diabetic agents since at least 6 months prior to liver biopsy may be enrolled
4. Females must be of non-child bearing potential (surgically sterilized or at least two years post-menopausal) or if of child-bearing potential, must have a negative pregnancy test at screening and agree to use an effective form of contraception during the study and for at least 30 days following the last dose of study medication
5. Normal ECG or clinically non-significant findings at the Screening and Baseline visits
6. No significant concomitant medical illness, without any clinically significant physical exam findings and without any clinically significant laboratory findings, as determined by the principal investigator
7. Signed an informed consent form indicating that they understand the purpose of and procedures required for the study and are willing to participate in the study and comply with the study procedures and restrictions

### Exclusion Criteria

Exclusion criteria have been designed to exclude subjects from the study if they will not be evaluable for the primary endpoint, if they have disease states that would interfere with analysis of study endpoints, or would put subjects at risk of serious adverse events associated with their participation.

Potential subjects will be excluded from participating in the study if they meet any of the following exclusion criteria:
1. Inability or unwillingness to have a liver biopsy
2. Diagnosis of cirrhosis by central pathology reviewers
3. Previous bariatric surgery or biliary diversion (i.e. gastric bypass), esophageal banding and gastric banding
4. Serum ALT > 300 U/L
5. Subject has used drugs associated with steatohepatitis within 6 months prior to screening (corticosteroids, high dose estrogens, methotrexate, amiodarone, anti-HIV drugs, tamoxifen, diltiazem)
6. Use of the following anti-NASH agents for more than a 2 week period in the 3 months prior to liver biopsy or the 3 months prior to screening:
   a. Vitamin E >60 IU per day
   b. Omega-3-acid ethyl esters or omega-3-PUFA-containing supplements >200 mg per day
   c. Thiazoledinediones (e.g. pioglitazone)
7. Patients on a non-stable dose of the following anti-NASH agents within 6 months of the liver biopsy or within 6 months of the screening visit: HMG-CoA reductase inhibitors (statins), fibrates, probucol, ezetimibe, ursodiol (UDCA), taurine, betaine, N-acetylcysteine, s-adenosylmethionine (SAM-e), milk thistle, anti-TNF therapies, or probiotics
8. Greater than a 10% decrease in weight within 8 weeks of baseline visit
9. Alcohol consumption > 30 g/day, currently or for more than 3 consecutive months within 5 years of screening
10. Blood alcohol level greater than 0.02% at screening and/or baseline
11. Evidence of active substance abuse, including prescription and recreational drugs
12. Other liver disease (hepatitis C, hepatitis B, Wilson's, autoimmune, α-1-antitrypsin and hemochromatosis) or known HIV infection
13. Pregnant or lactating at the screening visit
14. Renal insufficiency (creatinine >2 mg/dL), symptomatic coronary, peripheral or neurovascular disease, symptomatic heart failure (NYHA 2-4) or advanced respiratory disease requiring oxygen therapy
15. History of cerebral or retinal hemorrhage or other bleeding diathesis
16. QTc > 450 msec for males and >470 for females as corrected by the Fridericia formula
17. Inability to provide written informed consent
18. Received any investigational agent or participation in any clinical study of an investigational agent or investigational therapy within 3 months prior to the screening visit.
19. Any condition in the opinion of the Principal Investigator that would contraindicate the patient's participation

### Prohibited and Concomitant Medications

All prescription and over-the-counter medications taken by subjects during the 30 days before screening up to the start of treatment were recorded. The following medications were prohibited during participation in the study:

Omega-3-acid ethyl esters and omega-3-PUFA containing supplements >200 mg per day
Vitamin E >60 IU per day
Thiazolidinediones (e.g. pioglitazone)
The following medications were allowed during the study according to the specified restrictions:
Subjects could continue prescription or over-the-counter medications or herbal remedies (HMG-CoA reductase inhibitors [statins], fibrates, probucol, ezetimibe, ursodiol [UDCA], taurine, betaine, N-acetylcysteine, s-adenosylmethionine [SAM-e], milk thistle, anti-TNF therapies, or probiotics) ONLY if they have been on a stable dose for at least 6 months prior to screening
Subjects could continue the following anti-diabetic medications if they have been taking stable doses since at least 6 months prior to liver biopsy: biguanides (metformin), insulin, sulfonylureas, alpha-glucosidase inhibitors (acarbose), and phenylalanine derivatives (nateglinide)
Any subjects receiving anti-platelet therapy or anti-thrombotic agents (e.g. warfarin, ASA, and clopidogrel) after study commencement should be monitored closely during the study.

### Example 3: Self-emulsifying formulation

0.5 g of soybean lecithin, 1.0 g of polyoxyethylene (60) hydrogenated castor oil, 0.4 g of propylene glycol, and 3.1 g of EPA-E were weighed, and mixed while heating to a temperature of about 70° C. to prepare a self-emulsifying composition. After substituting with nitrogen, the self-emulsifying composition was hermetically sealed and stored at room temperature until the evaluation. Formulation of the self-emulsifying composition is shown below:

| Ingredients | Formulation (% by weight) |
|---|---|
| EPA-E | 62.0 |
| Soybean lecithin | 10.0 |
| Polyoxyethylene (60) hydrogenated castor oil | 20.0 |
| Propylene glycol | 8.0 |
| Total | 100.0 |

### Example 4: Self-emulsifying formulation

0.5 g of soybean lecithin, 1.0 g of polyoxyethylene (50) hydrogenated castor oil, 0.4 g of propylene glycol, and 3.1 g of EPA-E were weighed, and a self-emulsifying composition was prepared and stored by repeating the procedure of Example 3. Formulation of the self-emulsifying composition is shown below:

| Ingredients | Formulation (% by weight) |
|---|---|
| EPA-E | 62.0 |
| Soybean lecithin | 10.0 |
| Polyoxyethylene (60) hydrogenated castor oil | 20.0 |
| Propylene glycol | 8.0 |
| Total | 100.0 |

### Example 5: Self-emulsifying formulation

0.5 g of soybean lecithin. 0.9 g of polyoxyethylene castor oil, 0.6 g of propylene glycol, and 3.0 g of EPA-E were weighed, and a self-emulsifying composition was prepared and stored by repeating the procedure of Example 1. Formulation of the self-emulsifying composition is shown below:

| Ingredients | Formulation (% by weight) |
|---|---|
| EPA-E | 60.0 |
| Soybean lecithin | 10.0 |
| Polyoxyethylene castor oil | 18.0 |
| Propylene glycol | 12.0 |
| Total | 100.0 |

### Example 6: Self-emulsifying formulation

0.6 g of soybean lecithin, 0.6 g of polyoxyethylene (60) hydrogenated castor oil, 0.5 g of propylene glycol, and 3.3 g of EPA-E were weighed, and a self-emulsifying composition was prepared and stored by repeating the procedure of Example 1. Formulation of the self-emulsifying composition is shown below:

| Ingredients | Formulation (% by weight) |
|---|---|
| EPA-E | 66.0 |
| Soybean lecithin | 12.0 |
| Polyoxyethylene (60) hydrogenated castor oil | 12.0 |
| Propylene glycol | 10.0 |
| Total | 100.0 |

### Example 7: Self-emulsifying formulation

0.5 g of soybean lecithin, 0.5 g of polyoxyethylene (50) hydrogenated castor oil, 0.5 g of propylene glycol, and 3.5 g of EPA-E were weighed, and a self-emulsifying composition was prepared and stored by repeating the procedure of Example 3. Formulation of the self-emulsifying composition is shown below:

| Ingredients | Formulation (% by weight) |
|---|---|
| EPA-E | 70.0 |
| Soybean lecithin | 10.0 |
| Polyoxyethylene (50) hydrogenated castor oil | 10.0 |
| Propylene glycol | 10.0 |
| Total | 100.0 |

### Example 8: Self-emulsifying formulation

0.3 g of soybean lecithin, 0.3 g of polyoxyethylene (20) sorbitan monooleate, 0.9 g of polyoxyethylene (60) hydrogenated castor oil, 0.4 g of propylene glycol, and 3.1 g of EPA-E were weighed, and a self-emulsifying composition was prepared and stored by repeating the procedure of Example 3. Formulation of the self-emulsifying composition is shown below:

| Ingredients | Formulation (% by weight) |
|---|---|
| EPA-E | 62.0 |
| Soybean lecithin | 6.0 |
| Polyoxyethylene (20) sorbitan monooleate | 6.0 |
| Polyoxyethylene (60) hydrogenated castor oil | 18.0 |
| Propylene glycol | 8.0 |
| Total | 100.0 |

### Example 9: Self-emulsifying formulation

0.22 g of soybean lecithin, 0.36 g of polyoxyethylene (20) sorbitan monooleate, 0.36 g of polyoxyethylene 35 castor oil,1.2g of purified water and 4.0 g of EPA-E were weighed, and a self-emulsifying composition was prepared and stored by repeating the procedure of Example 3. Formulation of the self-emulsifying composition is shown below:

| Ingredients | Formulation (% by weight) |
|---|---|
| EPA-E | 80.0 |
| Soybean lecithin | 4.4 |
| Polyoxyethylene (20) sorbitan monooleate | 7.2 |
| Polyoxyethylene 35 castor oil | 7.2 |
| Purified water | 1.2 |
| Total | 100.0 |

### Example10: Effects of EPA-E on Women younger than 50

We conducted studies and analysis to investigate whether therapeutic effects of EPA are modulated by gender and/or reproductive state (e.g., menopause).

Adult subjects with biopsy-confirmed NASH within six months prior to randomization or those with suspected NASH were screened for the study. Those who did not have a liver biopsy within the last six months underwent a biopsy to confirm the presence of steatohepatitis. The histology was assessed using the NASH CRN criteria. The histological entry criteria included (1) the presence of possible or definite steatohepatitis, and (2) a NAFLD activity score (NAS) ≥ 4 with a minimum score of 1 each for steatosis and inflammation plus either ballooning or at least stage la sinusoidal fibrosis. The nonalcoholic nature of the disease was defined by a history of consumption of less than 3 drinks daily (approximately 10 gm alcohol/drink) for the previous 5 years. Those who exceeded these limits at the time of randomization or for more than 3 consecutive months within the five year time frame were excluded.

There were several exclusionary criteria in order to perform the study safely and have evaluable data at the end of the trial. The latter included refusal to provide informed consent, the presence of cirrhosis on the qualifying liver biopsy, decompensated liver disease with ascites, encephalopathy or variceal hemorrhage, serum ALT > 300 IU/l, pregnancy or lactation at the time of screening, serum creatinine > 2 mg/dl, symptomatic coronary, peripheral or neurovascular disease, symptomatic heart failure of New York Heart Association class 2 or higher, an EKG with a QTc > 450 msec for males and > 470 msec for females based on correction using the Frederica and Bazett formula, respiratory disease requiring oxygen therapy and a history of cerebral or retinal hemorrhage or known bleeding diatheses.

Several conditions that would confound analysis of the data were also considered to be exclusionary criteria. Subjects who had previously undergone bariatric surgery were excluded. Also, those with a greater than 10% change in weight in the two months prior to entry or with a blood alcohol over 0.02% at entry were excluded. Those with possible drug-induced steatohepatitis e.g. amiodarone or tamoxifen steatohepatitis were also excluded. Conversely, those who had received therapy with a non-stable dose of agents that potentially could benefit NASH within the previous six months prior to baseline liver biopsy e.g. taurine, betaine, s adenosyl methionine, milk thistle, anti-TNF agents, N-acetylcysteine, statins, fibrates, probucol, ezetemibe, ursodiol or probiotics were excluded. Specifically, those who consumed vitamin E > 60 IU/day, thiazolidinediones and n3 PUFA > 200 mg/day for more than two weeks within the three months prior to the qualifying liver biopsy were excluded. Subjects who had experienced a 10% or greater weight loss within 8 weeks prior to randomization were also excluded. The presence of other concomitant chronic liver diseases e.g. hepatitis C, HBsAg positive hepatitis B, Wilson disease, α1 antitrypsin deficiency and autoimmune hepatitis was also considered to be an exclusion criteria. Finally, subjects with poorly controlled type 2 diabetes (hemoglobin A1C > 9%) and those who had participated in an intervention trial within 3 months prior to entry in to this study were excluded. Subjects with type 2 diabetes or impaired glucose tolerance were considered eligible for the study if they had been on a stable dose of insulin, metformin, sulfonylurea, alpha-glucosidase inhibitor (acarbose), dipeptidyl-peptidase-4 inhibitors or phenylalanine derivatives for the previous six months prior to the qualifying liver biopsy. Subjects receiving anti-platelet therapy or anti-thrombotic agents such as warfarin after study commencement were monitored closely for safety and bleeding complications.

Efficacy data set (N=174) of same variable set for the main analysis was used in this study. The age 50 was used to divide the subject groups, resulting age groups of 0: age<50; 1: age>=50. The age of 50 is reported to be the average age at menopause among women in the US (Am J Epidemiol 2006;164: 1003-1011(Ref.1)). Three ethnic/races were analyzed: Non-Hispanic White vs. Hispanic vs. others.

The liver biopsies from subjects being considered for the trial were sent to a central pathology laboratory where they were read and scored. A total of 4 pathologists with experience in liver histopathology reviewed the biopsies. All the pathologists underwent a training session conducted by the lead pathologist (OWC) prior to the study. After slide quality assessment, the cases were randomly assigned to 2 reviewers who scored fatty change, lobular inflammation, hepatocyte ballooning and fibrosis as previously described. If there was a discrepancy between any of these evaluations, a third randomly assigned reviewer agreed with one of the choices thereby producing a single concordant NAS and fibrosis score for the biopsy.

Following confirmation of eligibility based on both inclusion and exclusion criteria, subjects were randomized to receive placebo or one of two doses of EPA-E (1800 mg/day or 2700 mg/day) in three divided doses daily. Block randomization using an interactive voice response system was used to assign subjects in a 1:1:1 ratio between the three arms. Subjects were stratified by the presence of diabetes, impaired glucose tolerance or metabolic syndrome on stable doses of medication. The total fraction of such individuals was capped at 40% of the study cohort.

All subjects were followed according to a predefined clinic schedule. Data from unscheduled visits were collected in separate forms. Following twelve months of treatment, all subjects underwent a protocol-driven liver biopsy. Study drug was then discontinued.

In order to incorporate baseline histologic grades/stages in the models, the baseline grades/stages were combined based on their distribution (median values (med) and frequencies in each grades/stages). This was done to avoid data separation in running the models due to limited sample size and low frequencies in some grades/stages.
NAS: med=5, 0: NAS<5; 1:NAS=5; 2: NAS>5
Lobular inflammation(LOB): med=2, 0: LOB<2; 1: LOB>=2 (mild vs. moderate to severe)
Hepatocyte ballooning(HB): med=1, 0: HB<2; 1: HB=2 (none to mild vs. severe)
Steatosis(STE): med=2, 0: STE<2; 1: STE=2; 2: STE=3
Fibrosis(FIB): med=1, 0: FIB=0; 1: FIB=1; 2:FIB>=2 (significant fibrosis)
Advanced FIB 0: FIB<3; 1: FIB=3
Baseline HOMA-IR: med=4.9, 0: HOMA-IR<4.9; 1: HOMA-IR>=4.9

The primary one-year efficacy endpoint was a composite of either a NAS ≤ 3 without worsening of fibrosis or a drop in NAS by 2 or more with contribution from more than one parameter and no worsening of fibrosis. The proportions of subjects meeting this endpoint after 12 months of treatment in those receiving EPA-E were compared to those receiving placebo using the Cochrane-Armitage trend test. A short-term efficacy endpoint was changes from baseline in alanine aminotransferase at 3 and 6 months. This was assessed by analysis of covariance. Secondary endpoints included changes in the individual histological parameters related to NAFLD, overall NAS, liver enzymes and function, hemoglobin A1C, acute phase reactants, insulin sensitivity as measured by the homeostatic model (HOMA-IR), levels of cytokeratin 18 (CK18), hyaluronic acid, type IV collagen (7S domain), procollagen III peptide and key cytokines. Proportions of subjects meeting categorical endpoints were compared using the Cochrane-Armitage test for trend. Numerical scores were compared using Wilcoxon 2-sample tests. Safety-related endpoints included adverse events including hematological-, biochemical-, renal- and EKG-related abnormalities. These were enumerated.

The sample size was estimated based on the assumption that the placebo response rate would be 20% while that for EPA-E (1800 mg/day) would be 30% and for EPA-E (2700 mg/day) would be 40%. It also accounted for a 10% drop out rate. Using these criteria, it was estimated that a sample size of 210 subjects (70 in each arm) would provide a power of 80% with a one-sided α set at 5%.

As a secondary analysis, univariate associations were analyzed for checking potential interaction by gender and the age group (Men<50, Men ≥50, Women<50, and Women≥50). More specifically, associations between the treatment groups and the changes in histologic features were separately assessed in a different gender/age category, using chi-square tests. The predictor was the treatment groups. The outcomes were changes in histologic features. Each histologic feature was categorized as -1: improvement; 0: no change; 1: worsening in Steatosis/Lobular inflammation/Hepatocyte ballooning/Fibrosis/ NAS.

Multivariable associations were then analyzed using multiple ordinal logistic regression models, adjusting for baselines histology, baseline BMI, and changes in BMI. Baseline histologic scores were combined as described above in order to avoid data separation in the model. All the analyses were performed using JMP statistical software version 9.0 (SAS institute Inc., NC).

### Results

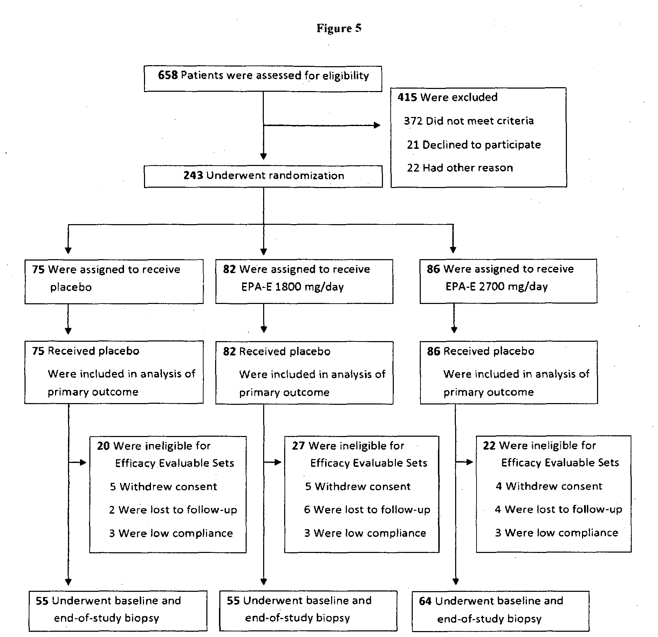

A total of 658 subjects were screened for the study at 37 centers within the United States. Of these, 415 were not considered to be eligible and were excluded. The reasons for exclusion from the trial are summarized in Figure 5. Of the remaining subjects, a total of 243 subjects met eligibility criteria and were randomized to placebo (n=75), EPA-E (1800 mg/day) (n=82) or EPA-E (2700 mg/day) (n=86). The subjects who received study medication were included in the safety evaluable dataset. A total of 17 subjects (22.7%) in the placebo arm, 27 (32.9%) subjects in EPA-E 1800 mg/day arm and 18 subjects (20.9%) in EPA-E 2700 mg/day arm discontinued the study prior to its conclusion. The principal reasons for these included withdrawal of consent (n=15), adverse events (n=10), use of prohibited medications (n=6) and loss to follow up (n=14). The distribution of these reasons was not significantly different across study arms. Thus, a total of 181 subjects completed the study and 174 subjects met criteria for a per protocol efficacy evaluable data set.. During the study, the averaged ratio of serum EPA to arachidonic acid was significantly different among the treatment groups (p<0.0001), and showed a dose-dependent increase from placebo to high-dose EPA-E: 0.087±0.029 vs. 0.395±0.029 vs. 0.568±0.027. This indicated that compliance was acceptable.

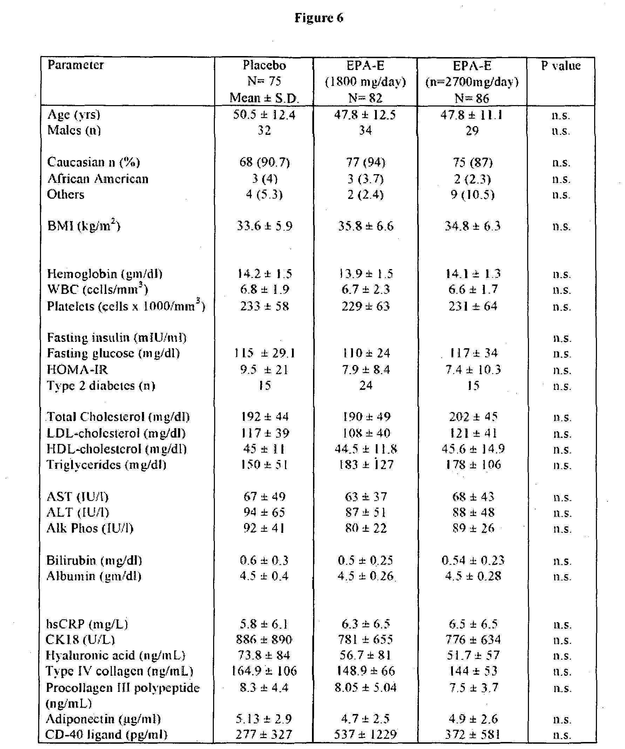

The baseline demographic, clinical and laboratory data of those randomized are shown in Figure 6. The mean age ranged was 48.6 years and 60.9% of the subjects were female. The cohort was also predominantly Caucasian (n=220). The placebo, low dose- and high dose EPA-E groups were comparable with respect to AST, ALT, Alkaline phosphatase and hepatic synthetic functions. Fifty four subjects had type 2 diabetes (placebo= 15, low dose EPA-E= 24, high dose EPA-E= 15). The groups were comparable with respect to hemoglobin A1C. The levels of acute phase reactants, cytokines, CK18 and circulating markers of fibrosis e.g. hyaluronic acid were all similar across the study arms. The total cholesterol, LDL-cholesterol, HDL-cholesterol and triglyceride levels were also similar across study arms.

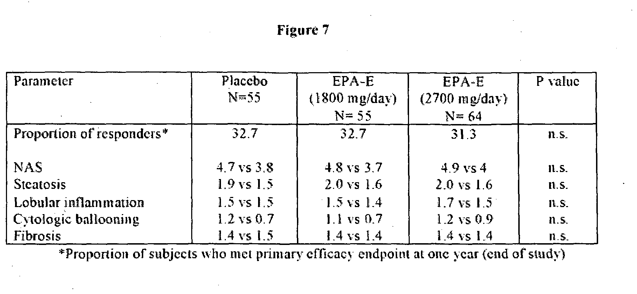

The primary one year and short-term efficacy analysis were completed using intention to treat as well as efficacy evaluable datasets. A total of 20 subjects in the placebo arm met the primary endpoint compared to 19 in the low dose EPA-E and 22 in the high dose EPA-E arms (p= ns) (Figure 7). The mean decrease in NAS was 0.9 in the placebo arm whereas it was 1 in the low dose EPA-E and 0.8 in the high dose EPA-E (p= n.s.). Sixteen subjects in the placebo arm had a decrease in NAS by more than 2 points while 16 and 18 subjects in the low dose- and high dose-EPA-E arms had a similar drop in NAS (p= n.s.). The results were similar when per protocol analysis was performed. There were also no differences noted on subset analysis based on the presence or absence of diabetes. The results were not qualitatively different in analysis of ITT population versus efficacy evalualable datasets.

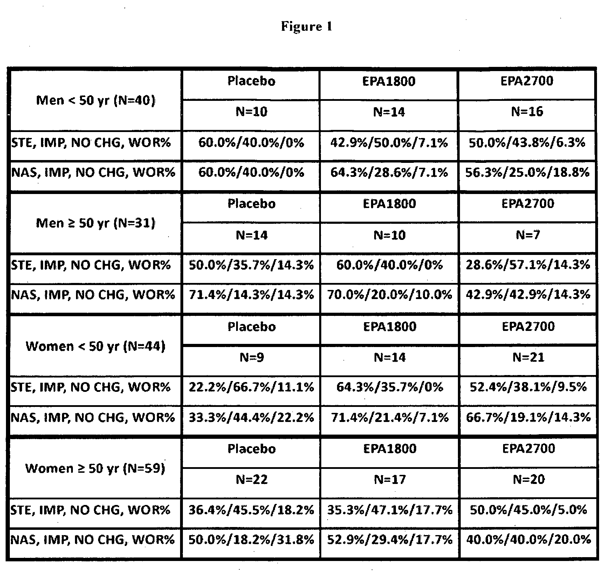

According to the univariate associations assessed separately in a different gender/age category, potential interaction was detected in changes or improvements in steatosis and NAS, which are shown below. The statistical outputs are as shown in Figure 1. Potential interaction was suggested for steatosis and NAS.

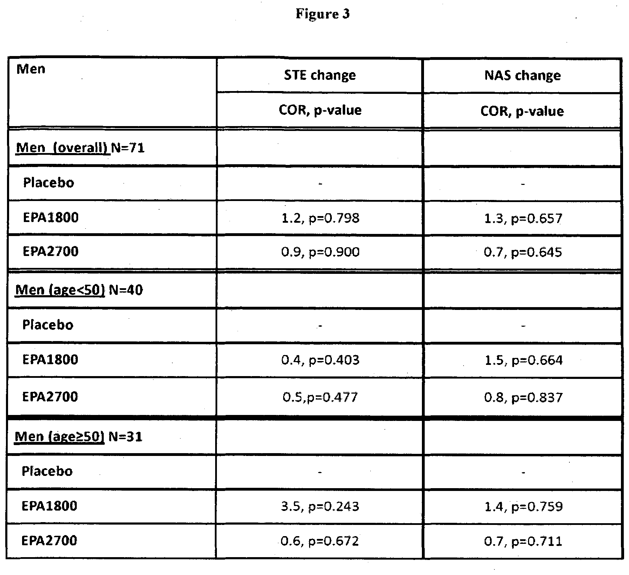

Multivariable models: Cumulative odds ratio (COR) was calculated in the multiple ordinal logistic regression models using the change in the histologic feature (steatosis or NAS) as an outcome and the treatment groups as a predictor, baseline histologic feature (steatosis or NAS), baseline BMI, and changes in BMI as covariates (Figures 2 and 3). Ordinal logistic regression adjusting for baseline histology, BMI and changes in BMI indicated a significant beneficial impact (more improvement, less worsening) in steatosis (Odds ratio: 7.9, p=0.03 for low dose EPA-E only) as well as overall NAS (Odds ratio: 10.5, p=0.02 for low dose EPA-E and 6.8. p=0.03) in women under 50 years of age receiving EPA-E. The results indicated a significant benefit in steatosis and NAS in women under 50 years of age receiving EPA-E.

These were analyzed using the efficacy evaluable dataset. At baseline, most subjects had grade 2 steatosis. Neither low dose- nor high dose-EPA-E affected the steatosis grade significantly for the cohort as a whole. Similarly, the distribution of cytological ballooning and lobular inflammation scores were comparable across the groups at baseline. These parameters were also not significantly altered by either low dose- versus high dose-EPA-E. The majority of subjects had either no fibrosis or stage 1 fibrosis. There were no significant changes in the distribution of fibrosis scores over the duration of the study in any of the treatment arms. There were also no across-group differences in fibrosis stage either at baseline or at the end of study across study arms.

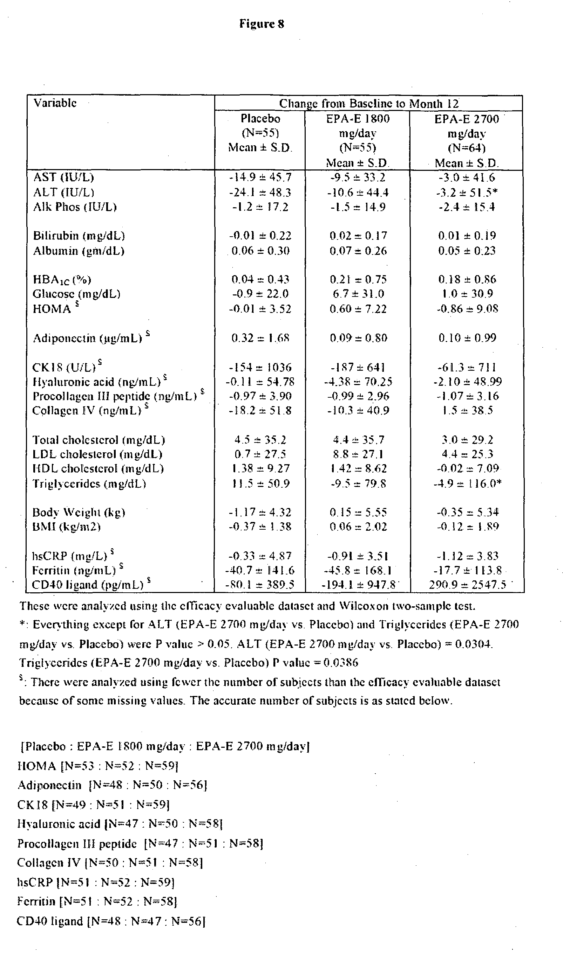

High dose EPA-E was associated with decreased triglyceride levels and less improvement in serum ALT when compared to the placebo (-4.9 ± 116.0 vs.11.5 ± 50.9, p<0.05 for serum triglycerides and -3.2 ± 51.5 vs. -24.1 ± 48.3, p<0.05 for ALT, Wilcoxon rank sum tests) (Figure 8). The hepatic synthetic functions also did not change over the course of the study and remained stable. Similarly, there were no significant changes in the HgbA1C or circulating fasting insulin concentrations in either active treatment arms compared to placebo. The body mass index also remained similar across groups.

Circulating CK18 levels remained similar across all three study arms from baseline to end of study. hsCRP levels also were similar across the study groups. Circulating hyaluronic acid, connective tissue growth factor, type IV collagen levels also did not change significantly over the course of the study in any study group or across study groups. Similarly, circulating TNFR1, TNFR2 and adiponectin levels remained relatively unchanged over the duration of the study.

Safety analysis was performed on the safety evaluable dataset. No deaths occurred over the course of the study. 94% of subjects on placebo reported adverse events compared 79% of subjects on low dose EPA-E and 86% of subjects on high dose EPA-E (p= n.s.). The number of severe adverse events was small and similarly distributed across the study arms. Seven, five and two subjects receiving placebo, low dose- or high dose-EPA-E respectively withdrew from the study due to these adverse events. The most common adverse events included nausea, diarrhea and abdominal discomfort followed by fatigue. Cardiovascular adverse events were reported in 6 subjects on placebo compared to 3 on low dose EPA-E and 2 subjects on high dose EPA-E. There were no significant differences in the rates of any specific or overall adverse events across the study arms.

In summary, univariate analysis in different sub-groups identified potential gender/age-interaction in the effects of EPA on hepatic steatosis and NAS; and potential beneficial effects were observed only in women <50. After adjusting for baseline histologic features, baseline BMI, and BMI changes, EPA1800 was associated with an increased likelihood (8-fold) of having beneficial impact (more improvement, less deterioration) on steatosis grade, when compared to placebo (p<0.04). In addition, EPA1800 was associated with an increased likelihood (10-fold) of having beneficial impact (more improvement, less deterioration) on overall NAS score, when compared to placebo (p<0.03). EPA2700 was associated with an increased likelihood (7-fold) of having beneficial impact (more improvement, less deterioration) on overall NAS score, when compared to placebo (p<0.04).

Age group did not significantly modify effects of EPA in men. Thus, the age-modification was only observed in women, which suggests that the modification may be associated with menopause as opposed to age itself. This is a significant and unexpected effects that post hoc analysis suggested a benefit for women less than 50 years of age.

### Example 11: Effects of EPA-E and estrogen on menopausal women

A Caucasian woman with NASH that has menopause is entered for the study. The woman is administered with 2700 mg/day of EPA-E for one month, along with co-administration of 0.625 mg of estrogen pills for her hormone replacement therapy. The administration takes place once daily and is maintained for 3 months. Both steatosis and NAS scores are monitored over time. The serum level of estrogen is measured with the estrogen pill.

## Claims

1. A pharmaceutical composition comprising ethyl eicosapentanoate (EPA-E), eicosapentaenoic acid (EPA) or pharmaceutically acceptable amides, salts, ester or phospholipids thereof for use in treating a fatty liver disease or disorder in a subject, wherein the subject receives a hormone replacement therapy, and wherein the fatty liver disease or disorder is Non-Alcoholic Steatohepatitis (NASH).

2. The composition for use according to claim 1, wherein the hormone in the hormone replacement therapy is estrogen.

3. A pharmaceutical composition comprising ethyl eicosapentanoate (EPA-E), eicosapentaenoic acid (EPA) or pharmaceutically acceptable amides, salts, ester or phospholipids thereof for use in treating a fatty liver disease or disorder in a subject, wherein the composition is used in combination with an estrogen, and wherein the fatty liver disease or disorder is Non-Alcoholic Steatohepatitis (NASH).

4. The composition for use according to claim 3, wherein the composition comprising at least one compound selected from the group consisting of ethyl eicosapentanoate (EPA-E), eicosapentaenoic acid (EPA) and its pharmaceutically acceptable amides, salts, esters and phospholipids and an effective amount of an estrogen.

5. A pharmaceutical composition comprising ethyl eicosapentanoate (EPA-E), eicosapentaenoic acid (EPA) or pharmaceutically acceptable amides, salts, ester or phospholipids thereof for use in treating a fatty liver disease or disorder in a subject, wherein the subject is a woman of an age of less than 50 years, and wherein the fatty liver disease or disorder is Non-Alcoholic Steatohepatitis (NASH)..

6. The composition for the use of any of claims 1-5, wherein the serum level of total estrogen in the subject is higher than 10 pg/ml.

7. The composition for use according to claims 2 to 4 or 6, wherein the estrogen is Estrone, Estradiol, Estriol or any combination thereof.

8. The composition for use according to claims 1 to 7, wherein fatty liver disease or disorder, which is Non-Alcoholic Steatohepatitis (NASH), is **characterized by** the baseline pretreatment level in the subject of at least one criteria selected from the group consisting of ALT in a range of 10 to 300 IU/L, AST in a range of 10 to 250 IU/L, HDL/C in a range of 25 to 55 mg/dl, LDL-C in a range of 100 to 200 mg/dl, triglycerides in a range of 100 to 1000 mg/dl, TC in a range of 170 to 300 mg/dl, High TG and low HDL-C, TG/HDL-C ratio in a range of 3.75 to 10, non-HDL-C in a range of 100 to 250 mg/dl, Free fatty acid in a range of 400 to 1000 µ Eq/L, HOMA-IR in a range of 1.5 to 5, HbAlc in a range of 5.7 to 10%, Fasting plasma glucose in a range of 100 to 200 mg/dl, impaired glucose tolerance and metabolic syndrome.

9. The composition for use according to claims 1 to 7, wherein fatty liver disease or disorder, which is Non-Alcoholic Steatohepatitis (NASH), is **characterized by** the baseline pretreatment level in the subject of at least one criteria selected from the group consisting of low level of EPA, DPA, DHA, EPA/AA, DHA/AA, DHA/DPA, AA/Homo- y - linoleic acid: and high level of AA, MUFA, Palmitoleic acid, Oleic acid, Oleic acid/Stearic acid, Palmitoleic acid/Palmitic acid, y - linoleic acid/Linoleic acid, Adrenic acid/AA compared to each average level in subjects with fatty liver disease or disorder.

10. The composition for use according to claims 1 to 9, wherein the pharmaceutical composition comprises a self emulsifying composition.

11. The composition for use according to claim 10, wherein the self emulsifying composition comprises at least one member selected from the group consisting of polyoxyethylene hydrogenated castor oil, polyoxyethylene sorbitan fatty acid ester, polyoxyethylene castor oil, polyethylene glycol fatty acid ester, polyoxyethylene polyoxypropylene glycol, sucrose fatty acid ester, sorbitan fatty acid ester, glycerin fatty acid ester and lecithin.

12. The composition for use according to claim 10 or 11, wherein the composition comprises at least one member selected from the group consisting of a combination of EPA-E, EPA, or pharmaceutically acceptable amides, salts, ester or phospholipids thereof and polyoxyethylene (50) hydrogenated castor oil or polyoxyethylene (60) hydrogenated castor oil; a combination of EPA-E, EPA, or pharmaceutically acceptable amides, salts, ester or phospholipids thereof and polyoxyethylene (20) sorbitan monooleate; a combination of EPA-E, EPA, or pharmaceutically acceptable amides, salts, ester or phospholipids thereof and polyoxyethylene castor oil; and a combination of EPA-E, EPA, or pharmaceutically acceptable amides, salts, ester or phospholipids thereof and sucrose fatty acid ester J- 1216 or J-1816.

13. The composition for use according to claims 10 to 12, wherein the composition comprises EPA-E, polyoxyethylene (20) sorbitan monooleate and polyoxyethylene 35 castor oil.

14. The composition a for use ccording to claims 1 to 13, wherein the composition comprises 50 to 95% by weight, EPA-E, EPA, or pharmaceutically acceptable amides, salts, ester or phospholipids thereof.

15. The composition for use according to claims 1 to 14, wherein EPA-E, EPA, or pharmaceutically acceptable amides, salts, ester or phospholipids thereof present in the composition is at least 40% by weight in total of the fatty acids and derivatives.

## Patentansprüche

1. Eine pharmazeutische Zusammensetzung, umfassend Ethyleicosapentanoat (EPA-E), Eicosapentaensäure (EPA) oder pharmazeutisch verträgliche Amide, Salze, Ester oder Phospholipide davon, zur Verwendung in der Behandlung einer Fettleberkrankheit oder - störung bei einem Patienten, wobei der Patient eine Hormonersatztherapie erhält und wobei die Fettleberkrankheit oder -störung Nichtalkoholische Steatohepatitis (NASH) ist.

2. Die Zusammensetzung zur Verwendung nach Anspruch 1, wobei das Hormon in der Hormonersatztherapie Östrogen ist.

3. Eine pharmazeutische Zusammensetzung, umfassend Ethyleicosapentanoat (EPA-E), Eicosapentaensäure (EPA) oder pharmazeutisch verträgliche Amide, Salze, Ester oder Phospholipide davon, zur Verwendung in der Behandlung einer Fettleberkrankheit oder - störung bei einem Patienten, wobei die Zusammensetzung in Kombination mit einem Östrogen verwendet wird und wobei die Fettleberkrankheit oder -störung Nichtalkoholische Steatohepatitis (NASH) ist.

4. Die Zusammensetzung zur Verwendung nach Anspruch 3, wobei die Zusammensetzung mindestens eine Verbindung ausgewählt aus der Gruppe bestehend aus Ethyleicosapentanoat (EPA-E), Eicosapentaensäure (EPA) und ihren pharmazeutisch verträglichen Amiden, Salzen, Estern und Phospholipiden und eine wirksame Menge eines Östrogens umfasst.

5. Eine pharmazeutische Zusammensetzung, umfassend Ethyleicosapentanoat (EPA-E), Eicosapentaensäure (EPA) oder pharmazeutisch verträgliche Amide, Salze, Ester oder Phospholipide davon, zur Verwendung in der Behandlung einer Fettleberkrankheit oder - störung bei einem Patienten, wobei der Patient eine Frau mit einem Alter von weniger als 50 Jahren ist und wobei die Fettleberkrankheit oder -störung Nichtalkoholische Steatohepatitis (NASH) ist.

6. Die pharmazeutische Zusammensetzung zur Verwendung nach einem der Ansprüche 1-5, wobei der Serumspiegel des Gesamtöstrogens in dem Patienten höher als 10 pg/ml ist.

7. Die Zusammensetzung zur Verwendung nach den Ansprüchen 2 bis 4 oder 6, wobei das Östrogen Estron, Estradiol, Estriol oder eine beliebige Kombination davon ist.

8. Die Zusammensetzung zur Verwendung nach den Ansprüchen 1 bis 7, wobei die Fettleberkrankheit oder -störung, die die Nichtalkoholische Steatohepatitis (NASH) ist, durch den Ausgangsniveau-Vorbehandlungsgrad des Subjekts nach mindestens einem Kriterium gekennzeichnet ist, das aus der Gruppe ausgewählt ist, bestehend aus ALT in einem Bereich von 10 bis 300 IU/L, AST in einem Bereich von 10 bis 250 IU/L, HDL/C in einem Bereich von 25 bis 55 mg/dl, LDL-C in einem Bereich von 100 bis 200 mg/dl, Triglyceride in einem Bereich von 100 bis 1000 mg/dl, TC in einem Bereich von 170 bis 300 mg/dl, hohes TG und niedriges HDL-C, TG/HDL-C-Verhältnis in einem Bereich von 3.75 bis 10, Nicht-HDL-C in einem Bereich von 100 bis 250 mg/dl, freie Fettsäure in einem Bereich von 400 bis 1000 µ Äq/L, HOMA-IR in einem Bereich von 1,5 bis 5, HbAlc in einem Bereich von 5,7 bis 10%, Nüchtern-Plasmaglukose in einem Bereich von 100 bis 200 mg/dl, beeinträchtigte Glukosetoleranz und metabolisches Syndrom.

9. Die Zusammensetzung zur Verwendung nach den Ansprüchen 1 bis 7, wobei die Fettleberkrankheit oder -störung, die eine nicht-alkoholische Steatohepatitis (NASH) ist, durch den Ausgangsniveau-Vorbehandlungsgrad des Subjekts nach mindestens einem Kriterium gekennzeichnet ist, das aus der Gruppe ausgewählt ist, bestehend aus einem niedrigen Niveau von EPA, DPA, DHA, EPA/AA, DHA/AA, DHA/DPA, AA/Homo- y - Linolsäure; und hohem Gehalt an AA, MUFA, Palmitoleinsäure, Ölsäure, Ölsäure/Stearinsäure, Palmitoleinsäure/Palmitinsäure, y -Linolsäure/Linolsäure, Adreninsäure/AA im Vergleich zu jeweils dem Durchschnittswert bei Personen mit Fettleberkrankheit oder -störung.

10. Die Zusammensetzung zur Verwendung nach den Ansprüchen 1 bis 9, wobei die pharmazeutische Zusammensetzung eine selbstemulgierende Zusammensetzung umfasst.

11. Die Zusammensetzung zur Verwendung nach Anspruch 10, wobei die selbstemulgierende Zusammensetzung mindestens ein Element ausgewählt aus der Gruppe bestehend aus Polyoxyethylen-hydriertem Rizinusöl, Polyoxyethylensorbitanfettsäureester, Polyoxyethylen-Rizinusöl, Polyethylenglykolfettsäureester, Polyoxyethylen-Polyoxypropylenglykol, Saccharosefettsäureester, Sorbitanfettsäureester, Glycerinfettsäureester und Lecithin umfasst.

12. Die Zusammensetzung zur Verwendung nach Anspruch 10 oder 11, wobei die Zusammensetzung mindestens ein Element umfasst, ausgewählt aus der Gruppe, bestehend aus einer Kombination von EPA-E, EPA oder pharmazeutisch verträglichen Amiden, Salzen, Estern oder Phospholipiden davon und Polyoxyethylen(50)- hydriertem Rizinusöl oder Polyoxyethylen(60)- hydriertem Rizinusöl; eine Kombination von EPA-E, EPA oder pharmazeutisch verträglichen Amiden, Salzen, Estern oder Phospholipiden davon und Polyoxyethylen(20)-Sorbitanmonooleat; eine Kombination von EPA-E, EPA oder pharmazeutisch verträglichen Amiden, Salzen, Estern oder Phospholipiden davon und Polyoxyethylen-Rizinusöl; und eine Kombination von EPA-E, EPA oder pharmazeutisch verträglichen Amiden, Salzen, Estern oder Phospholipiden davon und Saccharosefettsäureester J-1216 oder J-1816.

13. Die Zusammensetzung zur Verwendung nach den Ansprüchen 10 bis 12, wobei die Zusammensetzung EPA-E, Polyoxyethylen(20)sorbitanmonooleat und Polyoxyethylen-35-Rizinusöl umfasst.

14. Die Zusammensetzung zur Verwendung nach den Ansprüchen 1 bis 13, wobei die Zusammensetzung 50 bis 95 Gew.-% EPA-E, EPA oder pharmazeutisch verträgliche Amide, Salze, Ester oder Phospholipide davon umfasst.

15. Die Zusammensetzung zur Verwendung nach den Ansprüchen 1 bis 14, wobei EPA-E, EPA oder pharmazeutisch verträgliche Amide, Salze, Ester oder Phospholipide davon, die in der Zusammensetzung vorhanden sind, insgesamt mindestens 40 Gew.-% der Fettsäuren und Derivate ausmachen.

## Revendications

1. Composition pharmaceutique comprenant de l'éicosapentanoate d'éthyle (EPA-E), de l'acide éicosapentaénoïque (EPA) ou ses amides, sels, esters ou phospholipides pharmaceutiquement acceptables pour l'utilisation dans le traitement d'une maladie ou d'un trouble du foie gras chez un sujet, le sujet recevant un traitement hormonal substitutif, et la maladie ou le trouble du foie gras étant la stéatohépatite non alcoolique (NASH).

2. Composition pour l'utilisation selon la revendication 1, l'hormone dans le traitement hormonal substitutif étant l'œstrogène.

3. Composition pharmaceutique comprenant de l'éicosapentanoate d'éthyle (EPA-E), de l'acide éicosapentaénoïque (EPA) ou ses amides, sels, esters ou phospholipides pharmaceutiquement acceptables pour l'utilisation dans le traitement d'une maladie ou d'un trouble du foie gras chez un sujet, la composition étant utilisée en combinaison avec un œstrogène, et la maladie ou le trouble du foie gras étant la stéatohépatite non alcoolique (NASH).

4. Composition pour l'utilisation selon la revendication 3, la composition comprenant au moins un composé sélectionné dans le groupe constitué de l'éicosapentanoate d'éthyle (EPA-E), de l'acide éicosapentaénoïque (EPA) et ses amides, sels, esters et phospholipides pharmaceutiquement acceptables et une quantité efficace d'un œstrogène.

5. Composition pharmaceutique comprenant l'éicosapentanoate d'éthyle (EPA-E), l'acide éicosapentaénoïque (EPA) ou ses amides, sels, esters ou phospholipides pharmaceutiquement acceptables pour l'utilisation dans le traitement d'une maladie ou d'un trouble du foie gras chez un sujet, le sujet étant une femme d'un âge inférieur à 50 ans, et la maladie ou le trouble du foie gras étant la stéatohépatite non alcoolique (NASH).

6. Composition pour l'utilisation selon l'une quelconque des revendications 1 à 5, le niveau sérique d'œstrogène total chez le sujet étant supérieur à 10 pg/ml.

7. Composition pour l'utilisation selon les revendications 2 à 4 ou 6, l'œstrogène étant l'estrone, l'estradiol, l'estriol ou n'importe quelle combinaison de ceux-ci.

8. Composition pour l'utilisation selon les revendications 1 à 7, la maladie ou le trouble du foie gras, qui est la stéatohépatite non alcoolique (NASH), étant caractérisé·e par le niveau de prétraitement de ligne de base chez le sujet d'au moins un critère sélectionné dans le groupe constitué d'ALT dans une plage de 10 à 300 U.I./l, de l'AST dans une plage de 10 à 250 U.I./l, du HDL/C dans une plage de 25 à 55 mg/dl, du LDL-C dans une plage de 100 à 200 mg/dl, des triglycérides dans une plage de 100 à 1 000 mg/dl, du TC dans une plage de 170 à 300 mg/dl, du TG à teneur élevée et du HDL-C à faible teneur, le rapport TG/HDL-C situé dans une plage de 3,75 à 10, du non-HDL-C dans une plage de 100 à 250 mg/dl, de l'acide gras libre dans une plage de 400 à 1 000 µ d'éq./l, du HOMA-IR dans une plage de 1,5 à 5, de la HbAlc dans une plage de 5,7 à 10 %, le taux de glucose dans le plasma à jeun dans une plage de 100 à 200 mg/dl, la tolérance altérée au glucose et le syndrome métabolique.

9. Composition pour l'utilisation selon les revendications 1 à 7, la maladie ou le trouble du foie gras, qui est la stéatohépatite non alcoolique (NASH), étant caractérisé·e par le niveau de prétraitement de ligne de base chez le sujet d'au moins un critère sélectionné dans le groupe constitué du faible niveau d'EPA, DPA, DHA, EPA/AA, DHA/AA, DHA/DPA, AA/acide homo-γ-linoléique : et du niveau élevé de AA, MUFA, de l'acide palmitoléique, de l'acide oléique, de l'acide oléique/acide stéarique, de l'acide palmitoléique/acide palmitique, de l'acide γ-linoléique/acide linoléique, de l'acide adrénique/AA comparé à chaque niveau moyen chez les sujets ayant la maladie ou le trouble du foie gras.

10. Composition pour l'utilisation selon les revendications 1 à 9, la composition pharmaceutique comprenant une composition auto-émulsifiante.

11. Composition pour l'utilisation selon la revendication 10, la composition auto-émulsifiante comprenant au moins un élément sélectionné dans le groupe constitué de l'huile de ricin hydrogénée de polyoxyéthylène, de l'ester d'acide gras de polyoxyéthylène sorbitan, de l'huile de ricin de polyoxyéthylène, de l'ester d'acide gras de polyéthylène glycol, du polyoxyéthylène polyoxypropylène glycol, de l'ester d'acide gras de saccharose, de l'ester d'acide gras de sorbitan, de l'ester d'acide gras de glycérine et de la lécithine.

12. Composition pour l'utilisation selon la revendication 10 ou 11, la composition comprenant au moins un élément sélectionné dans le groupe constitué d'une combinaison d'EPA-E, EPA, ou de ses amides, sels, ester ou phospholipides pharmaceutiquement acceptables et d'huile de ricin hydrogénée de polyoxyéthylène (50) ou d'huile de ricin hydrogénée de polyoxyéthylène (60) ; d'une combinaison d'EPA-E, EPA, ou de ses amides, sels, esters ou phospholipides pharmaceutiquement acceptables et de monooléate de polyoxyéthylène (20) sorbitan ; d'une combinaison d'EPA-E, EPA, ou de ses amides, sels, esters ou phospholipides pharmaceutiquement acceptables et d'huile de ricin de polyoxyéthylène ; et d'une combinaison d'EPA-E, EPA, ou de ses amides, sels, esters ou phospholipides pharmaceutiquement acceptables et de l'ester d'acide gras de saccharose J-1216 ou J-1816.

13. Composition pour l'utilisation selon les revendications 10 à 12, la composition comprenant de l'EPA-E, du monooléate de polyoxyéthylène (20) sorbitan et de l'huile de ricin de polyoxyéthylène 35.

14. Composition pour l'utilisation selon les revendications 1 à 13, la composition comprenant de 50 à 95 % en poids d'EPA-E, EPA, ou ses amides, sels, esters ou phospholipides pharmaceutiquement acceptables.

15. Composition, pour l'utilisation selon les revendications 1 à 14, l'EPA-E, l'EPA, ou ses amides, sels, esters ou phospholipides pharmaceutiquement acceptables présents dans la composition est d'au moins 40 % en poids au total des acides gras et dérivés.
